# EUROPEAN PATENT APPLICATION

(11) **EP 4 253 959 A2**
(43) Date of publication of application: **04.10.2023**
(21) Application number: 23174022.6
(22) Date of filing: 14.02.2019
(51) Int. Cl.: G01N 33/68

(54) **ANTI-IDIOTYPIC ANTIBODIES DIRECTED TO THE ANTIGEN-BINDING PORTION OF AN BCMA-BINDING MOLECULE**

(30) Priority: 14.02.2018 US 201862630777 P
(62) Divisional of application: 19709820.5
(71) Applicant: Kite Pharma, Inc., Santa Monica, CA 90404 (US)
(72) Inventor: SIEVERS, Stuart A., Santa Monica, 90404 (US); WILTZIUS, Jed J. W., Winchester, 01890 (US)
(74) Representative: Thomann, William John

(57) **Abstract**

Isolated antigen binding molecules that specifically bind to a BCMA binding molecule are provided. The antigen binding molecules may be used in the methods provided herein. Specifically, monoclonal anti-idiotypic antibodies are provided which bind to the antigen-binding portion of a BCMA-binding molecule, in particular of anti-BCMA CARs.

## Description

### CROSS-REFERENCE TO RELATED APPLICATIONS

This application claims priority to U.S. Provisional Application No. 62/630,777, filed February 14, 2018, which is incorporated by reference herein in its entirety.

### TECHNICAL FIELD

This disclosure relates to antigen binding molecules, such as antibodies, which specifically bind to a BCMA binding molecule, as well as molecules comprising these sequences and cells presenting such molecules, polynucleotides encoding such antigen binding molecules, as well as humanized forms of the antigen binding molecules; methods of using the antigen binding molecules are also disclosed.

### BACKGROUND

Antigen binding molecules, including antibodies, and fragments such as Fabs, (Fab)₂, scFvs, etc, are used in immunotherapy and solid phase-based applications such as biosensors, affinity chromatography, and immunoassays. These antibodies and other antigen binding molecules gain their utility by virtue of their ability to specifically bind their targets.

Anti-idiotypic antibodies are a subset of antibodies, and are antibodies raised against immunizing antibodies. These anti-idiotypic antibodies demonstrated specific binding against the idiotopes (unique antigenic determinants on the surface of the antibodies) of the immunizing antibodies. Anti-idiotypic antibodies may be generally classified into three distinct groups: (1) antibodies are those that recognize idiotopes distinct from the antigen-binding site (ABS) on immunizing antibodies; (2) antibodies that recognize epitopes within the ABS and mimic the structure, and forming the so-called "internal image," of the nominal antigen; and (3) antibodies that recognize epitopes within the ABS without the structural resemblance of the nominal antigen (*see, e.g.,* Pan et al., (1995) FASEB J9:43-49).

### SUMMARY

Disclosed herein are rabbit antibodies that specifically bind to a BCMA binding molecule as well as molecules comprising these sequences and cells presenting such molecules.

In one aspect, an isolated antigen binding molecule that specifically binds a molecule comprising a BCMA binding molecule is provided. In some embodiments, the antigen binding molecule specifically binds a molecule comprising one or more peptides (e.g., complementarity determining regions (CDRs)) selected from the group consisting of SEQ ID NOs: 32-64. In some embodiments, the antigen binding molecule is selected from the group consisting of an antibody, an scFv, a Fab, a Fab', a Fv, a F(ab')₂, a dAb, a human antibody, a humanized antibody, a chimeric antibody, a monoclonal antibody, a polyclonal antibody, a recombinant antibody, an IgE antibody, an IgD antibody, an IgM antibody, an IgG1 antibody, an IgG1 antibody having at least one mutation in the hinge region, an IgG2 antibody an IgG2 antibody having at least one mutation in the hinge region, an IgG3 antibody, an IgG3 antibody having at least one mutation in the hinge region, an IgG4 antibody, an IgG4 antibody having at least one mutation in the hinge region, an antibody comprising at least one non-naturally occurring amino acid, and any combination thereof.

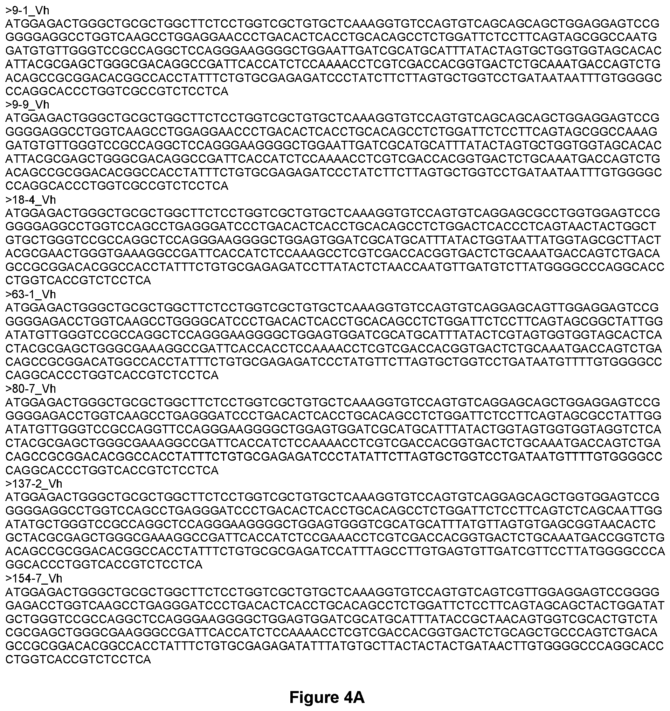

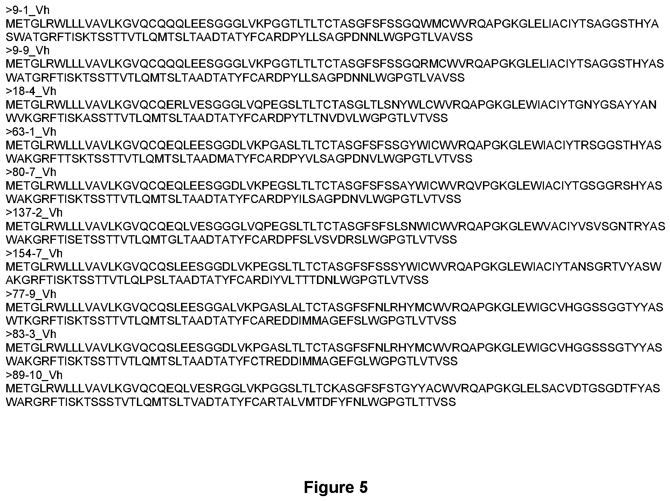

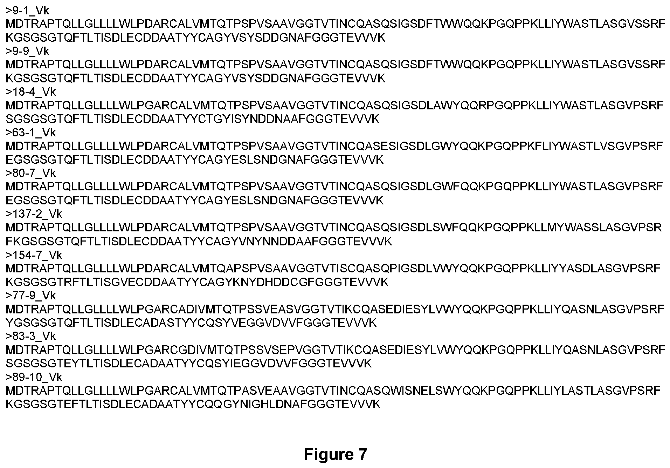

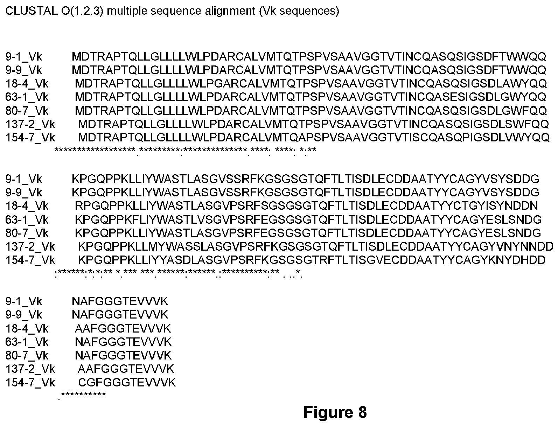

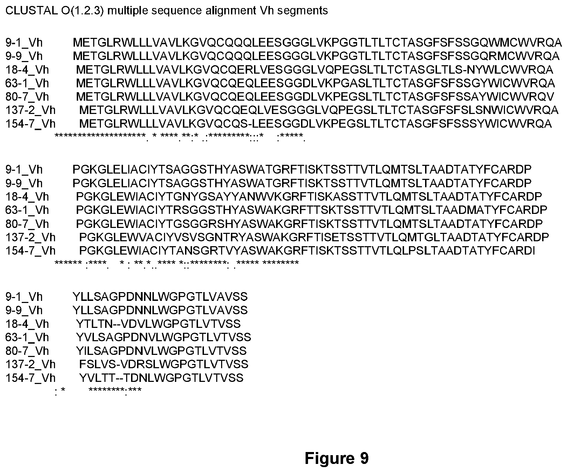

In some embodiments, the antigen binding molecule comprises a heavy chain (HC) and in further embodiments the HC comprises a heavy chain variable region (VH) sequence selected from the group consisting of SEQ ID NOs: 11-17, 89- 91. In still other embodiments, the variable region (VH) of the antigen binding molecule comprises one or more of (a) a CDR1, (b) a CDR2, and (c) a CDR3. In some embodiments, the antigen binding molecule comprises a heavy chain CDR1 selected from the group consisting of SEQ ID NOs: 38-43 65-67, 105, 106, 120, and 121. In some embodiments, the antigen binding molecule comprises a heavy chain CDR2 selected from the group consisting of SEQ ID NOs: 48-53, 68-73, 107-109, and 122-124. In some embodiments, the antigen binding molecule comprises a heavy chain CDR3 selected from the group consisting of SEQ ID NOs: 59-64, 110-112, and 125-127. In additional embodiments, the antigen binding molecule comprises a heavy chain comprising a heavy chain CDR1, a heavy chain CDR2, and a heavy chain CDR3, each CDR comprising an amino acid sequence shown in FIGURES 4A-11. In still further embodiments, an antigen binding molecule which comprises a VH amino acid sequence that is at least about 70%, at least about 75%, at least about 80%, at least about 85%, at least about 90%, at least about 95%, at least about 96%, at least about 97%, at least about 98%, at least about 99%, or about 100% identical to a VH of an antigen binding molecule provided herein.

In some embodiments, the antigen binding molecule comprises a light chain (LC) and in further embodiments the LC comprises a light chain variable region (VL) sequence selected from the group consisting of SEQ ID NOs: 25-31, and 95-97. In additional embodiments, the variable region (VL) and comprises one or more of (a) a CDR1, (b) a CDR2, and (c) a CDR3. In some embodiments, the antigen binding molecule comprises a light chain CDR1 selected from the group consisting of SEQ ID NOs: 32-37, 98, 99, 113, and 114. In some embodiments, the antigen binding molecule comprises a light chain CDR2 selected from the group consisting of SEQ ID NOs: 44-47, 100, 101, 115, and 116. In some embodiments, the antigen binding molecule comprises a light chain CDR3 selected from the group consisting of SEQ ID NOs: 54-58, 102-103, and 117-119. In some embodiments, the light chain comprises a light chain CDR1, a light chain CDR2, and a light chain CDR3, each CDR comprising an amino acid sequence shown in one of FIGURES 4A-11. In still further embodiments, an antigen binding molecule which comprises a VL amino acid sequence that is at least about 70%, at least about 75%, at least about 80%, at least about 85%, at least about 90%, at least about 95%, at least about 96%, at least about 97%, at least about 98%, at least about 99%, or about 100% identical to a VL of an antigen binding molecule provided herein.

In some embodiments, an antigen binding molecule comprises (a) a VH comprising the amino acid sequence of SEQ ID NO: 11; and (b) a VL comprising the amino acid sequence of SEQ ID NO: 25. In some embodiments, an antigen binding molecule comprises: (a) a VH CDR1 region comprising the amino acid sequence of SEQ ID NO: 38; (b) a VH CDR2 region comprising the amino acid sequence of SEQ ID NO: 48 or 68; (c) a VH CDR3 region comprising the amino acid sequence of SEQ ID NO: 59; (d) a VL CDR1 region comprising the amino acid sequence of SEQ ID NO: 32; (e) a VL CDR2 region comprising the amino acid sequence of SEQ ID NO: 44; and (f) a VL CDR3 region comprising the amino acid sequence of SEQ ID NO: 54.

In some embodiments, an antigen binding molecule comprises (a) a VH comprising the amino acid sequence of SEQ ID NO: 12; and (b) a VL comprising the amino acid sequence of SEQ ID NO: 26. In some embodiments, an antigen binding molecule comprises: (a) a VH CDR1 region comprising the amino acid sequence of SEQ ID NO: 38 or 39; (b) a VH CDR2 region comprising the amino acid sequence of SEQ ID NO: 48 or 68; (c) a VH CDR3 region comprising the amino acid sequence of SEQ ID NO: 59; (d) a VL CDR1 region comprising the amino acid sequence of SEQ ID NO: 32; (e) a VL CDR2 region comprising the amino acid sequence of SEQ ID NO: 44; and (f) a VL CDR3 region comprising the amino acid sequence of SEQ ID NO: 54.

In some embodiments, an antigen binding molecule comprises (a) a VH comprising the amino acid sequence of SEQ ID NO: 13; and (b) a VL comprising the amino acid sequence of SEQ ID NO: 27. In some embodiments, an antigen binding molecule comprises: (a) a VH CDR1 region comprising the amino acid sequence of SEQ ID NO: 40; (b) a VH CDR2 region comprising the amino acid sequence of SEQ ID NO: 49 or 69; (c) a VH CDR3 region comprising the amino acid sequence of SEQ ID NO: 60; (d) a VL CDR1 region comprising the amino acid sequence of SEQ ID NO: 33; (e) a VL CDR2 region comprising the amino acid sequence of SEQ ID NO: 44; and (f) a VL CDR3 region comprising the amino acid sequence of SEQ ID NO: 55.

In some embodiments, an antigen binding molecule comprises (a) a VH comprising the amino acid sequence of SEQ ID NO: 14; and (b) a VL comprising the amino acid sequence of SEQ ID NO: 28. In some embodiments, an antigen binding molecule comprises: (a) a VH CDR1 region comprising the amino acid sequence of SEQ ID NO: 41 or 65; (b) a VH CDR2 region comprising the amino acid sequence of SEQ ID NO: 50 or 70; (c) a VH CDR3 region comprising the amino acid sequence of SEQ ID NO: 61; (d) a VL CDR1 region comprising the amino acid sequence of SEQ ID NO: 34; (e) a VL CDR2 region comprising the amino acid sequence of SEQ ID NO: 45; and (f) a VL CDR3 region comprising the amino acid sequence of SEQ ID NO: 56.

In some embodiments, an antigen binding molecule comprises (a) a VH comprising the amino acid sequence of SEQ ID NO: 15; and (b) a VL comprising the amino acid sequence of SEQ ID NO: 29. In some embodiments, an antigen binding molecule comprises: (a) a VH CDR1 region comprising the amino acid sequence of SEQ ID NO: 42 or 66; (b) a VH CDR2 region comprising the amino acid sequence of SEQ ID NO: 51 or 71; (c) a VH CDR3 region comprising the amino acid sequence of SEQ ID NO: 62; (d) a VL CDR1 region comprising the amino acid sequence of SEQ ID NO: 35; (e) a VL CDR2 region comprising the amino acid sequence of SEQ ID NO: 44; and (f) a VL CDR3 region comprising the amino acid sequence of SEQ ID NO: 56.

In some embodiments, an antigen binding molecule comprises (a) a VH comprising the amino acid sequence of SEQ ID NO: 16; and (b) a VL comprising the amino acid sequence of SEQ ID NO: 30. In some embodiments, an antigen binding molecule comprises: (a) a VH CDR1 region comprising the amino acid sequence of SEQ ID NO: 43 or 67; (b) a VH CDR2 region comprising the amino acid sequence of SEQ ID NO: 52 or 72; (c) a VH CDR3 region comprising the amino acid sequence of SEQ ID NO: 63; (d) a VL CDR1 region comprising the amino acid sequence of SEQ ID NO: 36; (e) a VL CDR2 region comprising the amino acid sequence of SEQ ID NO: 46; and (f) a VL CDR3 region comprising the amino acid sequence of SEQ ID NO: 57.

In some embodiments, an antigen binding molecule comprises (a) a VH comprising the amino acid sequence of SEQ ID NO: 17; and (b) a VL comprising the amino acid sequence of SEQ ID NO: 31. In some embodiments, an antigen binding molecule comprises: (a) a VH CDR1 region comprising the amino acid sequence of SEQ ID NO: 85; (b) a VH CDR2 region comprising the amino acid sequence of SEQ ID NO: 53 or 73; (c) a VH CDR3 region comprising the amino acid sequence of SEQ ID NO: 64; (d) a VL CDR1 region comprising the amino acid sequence of SEQ ID NO: 37; (e) a VL CDR2 region comprising the amino acid sequence of SEQ ID NO: 47; and (f) a VL CDR3 region comprising the amino acid sequence of SEQ ID NO: 58.

In some embodiments, an antigen binding molecule comprises (a) a VH comprising the amino acid sequence of SEQ ID NO: 89; and (b) a VL comprising the amino acid sequence of SEQ ID NO: 95. In some embodiments, an antigen binding molecule comprises: (a) a VH CDR1 region comprising the amino acid sequence of SEQ ID NO: 120; (b) a VH CDR2 region comprising the amino acid sequence of SEQ ID NO: 122; (c) a VH CDR3 region comprising the amino acid sequence of SEQ ID NO: 125; (d) a VL CDR1 region comprising the amino acid sequence of SEQ ID NO: 113; (e) a VL CDR2 region comprising the amino acid sequence of SEQ ID NO: 115; and (f) a VL CDR3 region comprising the amino acid sequence of SEQ ID NO: 117.

In some embodiments, an antigen binding molecule comprises: (a) a VH comprising the amino acid sequence of SEQ ID NO: 90; and (b) a VL comprising the amino acid sequence of SEQ ID NO: 96. In some embodiments, the antigen binding molecule comprises: a VH CDR1 region comprising the amino acid sequence of SEQ ID NO: 120 (b) a VH CDR2 region comprising the amino acid sequence of SEQ ID NO: 123; (c) a VH CDR3 region comprising the amino acid sequence of SEQ ID NO: 126; (d) a VL CDR1 region comprising the amino acid sequence of SEQ ID NO: 113; (e) a VL CDR2 region comprising the amino acid sequence of SEQ ID NO: 115; and (f) a VL CDR3 region comprising the amino acid sequence of SEQ ID NO: 118.

In some embodiments, the antigen binding molecule comprises: (a) a VH comprising the amino acid sequence of SEQ ID NO: 91; and (b) a VL comprising the amino acid sequence of SEQ ID NO: 97. In some embodiments, the antigen binding molecule comprises: (a) a VH CDR1 region comprising the amino acid sequence of SEQ ID NO: 121; (b)
a VH CDR2 region comprising the amino acid sequence of SEQ ID NO: 124; (c) a VH CDR3 region comprising the amino acid sequence of SEQ ID NO: 127; (d) a VL CDR1 region comprising the amino acid sequence of SEQ ID NO: 114; (e) a VL CDR2 region comprising the amino acid sequence of SEQ ID NO: 116; and (f) a VL CDR3 region comprising the amino acid sequence of SEQ ID NO: 119.

In various embodiments, an antigen binding molecule provided herein further comprises a detectable label, and may be selected from the group consisting of a fluorescent label, a photochromic compound, a proteinaceous fluorescent label, a magnetic label, a radiolabel, and a hapten. In various embodiments, a fluorescent label is selected from the group consisting of an Atto dye, an Alexafluor dye, quantum dots, Hydroxycoumarin, Aminocouramin, Methoxycourmarin, Cascade Blue, Pacific Blue, Pacific Orange, Lucifer Yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhocamine B, Texas Red, Allophycocyanin (APC), APC-Cy7 conjugates, Indo-1, Fluo-3, Fluo-4, DCFH, DHR, SNARF, GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midorishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP,Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoeryhring (BPE), mCherry, HcRed1, Katusha, P3, Peridinin Chlorophyll (PerCP), mKate (TagFP635), TurboFP635, mPlum, and mRaspberry.

In another aspect, a composition comprising an antigen binding molecule disclosed herein is provided. Also provided is a polynucleotide encoding the heavy chain of an antigen binding molecule disclosed herein, and a polynucleotide encoding the light chain of an antigen binding molecule disclosed herein. A vector comprising the polynucleotides is also disclosed. Further, a cell comprising one or more such vectors is disclosed. In various embodiments, the cell comprises a cell selected from the group consisting of a CHO cell, a Sp2/0 cell, a rabbit cell and an *E. coli* cell. A method of making an antigen binding molecule disclosed herein comprising incubating a cell disclosed herein under suitable conditions is provided.

In another aspect, a method of administering a dose of a medicament to a subject, the dose comprising a preselected number of cells presenting a therapeutic molecule comprising a BCMA binding molecule, is provided. In some embodiments the method comprises (a) providing a sample of known volume comprising a population comprising a known number of cells, which cells are known or suspected to be presenting a molecule comprising a BCMA binding molecule; (b) providing an aliquot of the sample comprising a population of cells presenting a therapeutic molecule comprising a BCMA binding molecule ; (c) providing an antigen binding molecule that specifically binds a BCMA binding molecule, the antigen binding molecule further comprising a detectable label; (d) contacting the aliquot of (b) with the antigen binding molecule of (c) under conditions that permit the formation of a binding complex comprising a cell present in the sample and the antigen binding molecule; (e) determining the fraction of cells present in a binding complex of (d) in the aliquot; (f) determining the concentration of cells presenting a molecule comprising a BCMA binding molecule in the sample, based on the fraction of cells determined in (e); (g) determining the volume of the sample that comprises the selected number of cells; and (h) administering the volume of the sample determined in (g) to the subject.

In some embodiments of the method, (a) the molecule comprising a BCMA binding molecule is a CAR; and (b) the cell is an immune cell selected from the group consisting of CD8+ T cells, CD4+ T cells, tumor infiltrating lymphocytes (TILs), NK cells, TCR-expressing cells, dendritic cells, and NK-T cells. In another embodiment, the CAR further comprises a molecule, or a fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof. In an additional embodiment, the detectable label is selected from the group consisting of a fluorescent label, a photochromic compound, a proteinaceous fluorescent label, a magnetic label, a radiolabel, and a hapten. In further embodiments, the fluorescent label is selected from the group consisting of an Atto dye, an Alexafluor dye, quantum dots, Hydroxycoumarin, Aminocouramin, Methoxycourmarin, Cascade Blue, Pacific Blue, Pacific Orange, Lucifer Yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhocamine B, Texas Red, Allophycocyanin (APC), APC-Cy7 conjugates, Indo-1, Fluo-3, Fluo-4, DCFH, DHR, SNARF, GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midorishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP,Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoeryhring (BPE), mCherry, HcRed1, Katusha, P3, Peridinin Chlorophyll (PerCP), mKate (TagFP635), TurboFP635, mPlum, and mRaspberry. In additional embodiments, the immune cell is a T cell and in still further embodiments the T cell is disposed *in vitro* or the T cell is disposed *in vivo.* In other embodiments, the T cell is in one of blood, extracted tissue, tissue grown *ex vivo,* and cell culture media. In some embodiments, the T cell is an autologous T cell, and in other embodiments the T cell is an allogenic T cell. In an embodiment, the dose is 1.0×10⁶ cells per kilogram of the subject. Additionally, in various embodiments of the disclosed method the antigen binding molecule comprises an antigen binding molecule disclosed herein, and humanized forms thereof.

In another aspect, a method of determining a number of cells presenting a molecule comprising a BCMA binding molecule in a sample is provided. In an embodiment, the method comprises (a) providing a sample comprising cells known or suspected to be presenting a molecule comprising a BCMA binding molecule; (b) contacting the sample of (a) with an antigen binding molecule that specifically binds the molecule comprising a BCMA binding molecule, the antigen binding molecule further comprising a detectable label, under conditions that permit the formation of a binding complex comprising a cell present in the sample and the antigen binding molecule; and (c) determining the number of cells present in a binding complex of (b) in the sample.

In some embodiments of the disclosed method, (a) the molecule comprising a BCMA binding molecule is a CAR; and (b) the cell is an immune cell selected from the group consisting of CD8+ T cells, CD4+ T cells, tumor infiltrating lymphocytes (TILs), NK cells, TCR-expressing cells, dendritic cells, and NK-T cells. In some embodiments, the CAR further comprises a molecule, or a fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof. In some embodiments, the detectable label is selected from the group consisting of a fluorescent label, a photochromic compound, a proteinaceous fluorescent label, a magnetic label, a radiolabel, and a hapten. In some embodiments, the fluorescent label is selected from the group consisting of an Atto dye, an Alexafluor dye, quantum dots, Hydroxycoumarin, Aminocouramin, Methoxycourmarin, Cascade Blue, Pacific Blue, Pacific Orange, Lucifer Yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhocamine B, Texas Red, Allophycocyanin (APC), APC-Cy7 conjugates, Indo-1, Fluo-3, Fluo-4, DCFH, DHR, SNARF, GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midorishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP,Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoeryhring (BPE), mCherry, HcRed1, Katusha, P3, Peridinin Chlorophyll (PerCP), mKate (TagFP635), TurboFP635, mPlum, and mRaspberry. In additional embodiments, the immune cell is a T cell and in still further embodiments the T cell is disposed *in vitro* or the T cell is disposed *in vivo.* In other embodiments, the T cell is in one of blood, extracted tissue, tissue grown *ex vivo,* and cell culture media. In some embodiments, the T cell is an autologous T cell, and in other embodiments the T cell is an allogenic T cell. Additionally, in various embodiments of the disclosed method, the antigen binding molecule comprises an antigen binding molecule disclosed herein, and humanized forms thereof.

In another aspect, a method of isolating a molecule comprising a BCMA binding molecule is provided. In an embodiment, the method comprises (a) providing a sample known or suspected to comprise a molecule comprising a BCMA binding molecule; (b) providing an antigen binding molecule that specifically binds a molecule comprising a BCMA binding molecule, optionally comprising a detectable label; (c) contacting the sample with the antigen binding molecule, under conditions that permit the formation of a binding complex comprising the molecule comprising a BCMA binding molecule and the antigen binding molecule; (d) separating any molecules not part of a binding complex from formed binding complexes; and (e) separating a formed binding complex into: (a) a molecule comprising a BCMA binding molecule, and (b) an antigen binding molecule.

In embodiments of the disclosed method, the molecule comprising a BCMA binding molecule is a CAR. In embodiments, the CAR further comprises a molecule, or a fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof. In some embodiments, the antigen binding molecule is disposed on a surface selected from the group consisting of an agarose bead, a magnetic bead, a plastic welled plate, a glass welled plate, a ceramic welled plate and a cell culture bag. some embodiments, the detectable label is selected from the group consisting of a fluorescent label, a photochromic compound, a proteinaceous fluorescent label, a magnetic label, a radiolabel, and a hapten. In some embodiments, the fluorescent label is selected from the group consisting of an Atto dye, an Alexafluor dye, quantum dots, Hydroxycoumarin, Aminocouramin, Methoxycourmarin, Cascade Blue, Pacific Blue, Pacific Orange, Lucifer Yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhocamine B, Texas Red, Allophycocyanin (APC), APC-Cy7 conjugates, Indo-1, Fluo-3, Fluo-4, DCFH, DHR, SNARF, GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midorishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP,Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoeryhring (BPE), mCherry, HcRed1, Katusha, P3, Peridinin Chlorophyll (PerCP), mKate (TagFP635), TurboFP635, mPlum, and mRaspberry. Additionally, in various embodiments of the disclosed method the antigen binding molecule comprises an antigen binding molecule disclosed herein, and humanized forms thereof.

In another aspect, a method of determining the presence or absence of a molecule comprising a BCMA binding molecule in a sample. In some embodiments, the method comprises (a) providing a sample known or suspected to comprise a molecule comprising a BCMA binding molecule; (b) providing an antigen binding molecule comprising a detectable label that specifically binds a molecule comprising a BCMA binding molecule; (c) contacting the sample with the antigen binding molecule under conditions that permit the formation of a binding complex; (d) separating any molecules not part of a binding complex from formed binding complexes; and (e) detecting the presence or absence of a binding complex.

In some embodiments, the molecule comprising a BCMA binding molecule is a CAR, and in further embodiments, the CAR further comprises a molecule, or a fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof. In additional embodiments, the antigen binding molecule is disposed on a surface selected from the group consisting of an agarose bead, a magnetic bead, a plastic welled plate, a glass welled plate, a ceramic welled plate and a cell culture bag. In further embodiments, the detectable label is selected from the group consisting of a fluorescent label, a photochromic compound, a proteinaceous fluorescent label, a magnetic label, a radiolabel, and a hapten. In still further embodiments, the fluorescent label is selected from the group consisting of an Atto dye, an Alexafluor dye, quantum dots, Hydroxycoumarin, Aminocouramin, Methoxycourmarin, Cascade Blue, Pacific Blue, Pacific Orange, Lucifer Yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhocamine B, Texas Red, Allophycocyanin (APC), APC-Cy7 conjugates, Indo-1, Fluo-3, Fluo-4, DCFH, DHR, SNARF, GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midorishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP,Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoeryhring (BPE), mCherry, HcRed1, Katusha, P3, Peridinin Chlorophyll (PerCP), mKate (TagFP635), TurboFP635, mPlum, and mRaspberry. Additionally, in various embodiments of the disclosed method the antigen binding molecule comprises an antigen binding molecule disclosed herein, and humanized forms thereof.

In one aspect, a method of increasing the concentration of cells presenting a molecule comprising a BCMA binding molecule is provided. In some embodiments, the method comprises (a) providing a sample comprising a cell known or suspected to present a molecule comprising a BCMA binding molecule; (b) providing an antigen binding molecule that specifically binds a molecule comprising a BCMA binding molecule, optionally comprising a detectable label; (c) contacting the sample with the antigen binding molecule under conditions that permit the formation of a binding complex comprising the molecule comprising a BCMA binding molecule and the antigen binding molecule; (d) removing any components not part of a binding complex; and (e) repeating steps (a)-(d) a desired number of times.

In an embodiment, (a) the molecule comprising a molecule BCMA binding molecule is a CAR; and (b) the cell is an immune cell selected from the group consisting of CD8+ T cells, CD4+ T cells, tumor infiltrating lymphocytes (TILs), NK cells, TCR-expressing cells, dendritic cells, and NK-T cells. In other embodiments, the CAR further comprises a molecule, or a fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof. In additional embodiments, the immune cell is a T cell and in still further embodiments the T cell is disposed *in vitro* or the T cell is disposed *in vivo.* In other embodiments, the T cell is in one of blood, extracted tissue, tissue grown *ex vivo,* and cell culture media. In some embodiments, the T cell is an autologous T cell, and in other embodiments the T cell is an allogenic T cell. Additionally, in various embodiments of the disclosed method the antigen binding molecule comprises an antigen binding molecule disclosed herein, and humanized forms thereof. In further embodiments, the detectable label is selected from the group consisting of a fluorescent label, a photochromic compound, a proteinaceous fluorescent label, a magnetic label, a radiolabel, and a hapten. In embodiments, the fluorescent label is selected from the group consisting of an Atto dye, an Alexafluor dye, quantum dots, Hydroxycoumarin, Aminocouramin, Methoxycourmarin, Cascade Blue, Pacific Blue, Pacific Orange, Lucifer Yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhocamine B, Texas Red, Allophycocyanin (APC), APC-Cy7 conjugates, Indo-1, Fluo-3, Fluo-4, DCFH, DHR, SNARF, GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midorishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP,Topaz, Venus, mCitrine, YPet, TurboYFP, ZsYellowl, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoeryhring (BPE), mCherry, HcRed1, Katusha, P3, Peridinin Chlorophyll (PerCP), mKate (TagFP635), TurboFP635, mPlum, and mRaspberry.

In another aspect, a method of depleting a population of immune cells presenting a molecule comprising a BCMA binding molecule is provided. In some embodiments, the method comprises (a) providing a population of immune cells to be depleted, wherein the immune cells are known or suspected to be presenting a molecule comprising a BCMA binding molecule; and (b) contacting the immune cells with an antigen binding molecule that specifically binds to (a) the molecule comprising a BCMA binding molecule, and (b) an activating molecule expressed on the surface of the an immune cell not presenting the molecule comprising a BCMA binding molecule, under conditions that permit the formation of a ternary binding complex comprising the molecule comprising a BCMA binding molecule, the activating molecule and the antigen binding molecule. In further embodiments, (a) the molecule comprising a BCMA binding molecule is a CAR; and (b) the immune cell selected from the group consisting of CD8+ T cells, CD4+ T cells, tumor infiltrating lymphocytes (TILs), NK cells, TCR-expressing cells, dendritic cells, and NK-T cells. In still further embodiments, the CAR further comprises a molecule, or a fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof. In additional embodiments, the immune cell is a T cell and in still further embodiments the T cell is disposed *in vitro* or the T cell is disposed *in vivo.* In other embodiments, the T cell is in one of blood, extracted tissue, tissue grown *ex vivo,* and cell culture media. In some embodiments, the T cell is an autologous T cell, and in other embodiments the T cell is an allogenic T cell. Additionally, in various embodiments of the disclosed method the antigen binding molecule comprises an antigen binding molecule disclosed herein, and humanized forms thereof.

### BRIEF DESCRIPTION OF THE FIGURES

Figure 1 histograms using CAR-expressing T cells show that KIP-9 clonal supernatants (Clones 9-1, 9-9, 18-4, 63-1, 80-7, 137-2, and 154-7) bind anti-BCMA CAR-expressing T cells (top row), but not two control CAR-expressing T cells; the plots demonstrate specific, selective binding of the antibodies to the expressed anti-BCMA CAR.
Figure 2 shows a series of histograms showing clone 9-1 antibody binding. The results of flow cytometry experiments performed using anti-BCMA CAR demonstrate clone 9-1 antibody has different affinity for various affinity-matured anti-BCMA CARs (BCMA-AM1 and BCMA-AM2). Clone 9-1 binds to the anti-BCMA CAR-expressing cells and to one affinity-matured variant (right), but not others (e.g. center).
Figure 3 shows the results of flow cytometry experiments demonstrating additional clones bind to different epitopes on anti-BCMA CAR scFvs. Clones 77, 83, and 89 bind to the anti-BCMA CAR (BCMA-AM1), but clone 89 does not bind to BCMA-AM2.
Figure 4A and 4B shows a series of sequences showing the coding sequences for the VH region of anti-idiotypic binding molecules. Clones 9-1, 9-9, 18-4, 63-1, 80-7, 137-2, and 154-7 (Fig. 4A). The VH region of Clones 77-9, 83-3, and 89-10 are shown in Fig. 4B.
Figure 5 shows a series of sequences showing the amino acid sequences for the VH region of Clones 9-1, 9-9, 18-4, 63-1, 80-7, 137-2, and 154-7.
Figure 6A and 6B shows a series of sequences showing the coding sequences for the VL region of anti-idiotypic binding molecules. Clones 9-1, 9-9, 18-4, 63-1, 80-7, 137-2, and 154-7. The VL region of Clones 77-9, 83-3, and 89-10 are shown in Fig. 6B.
Figure 7 shows a series of sequences showing the amino acid sequences for the VL region of Clones 9-1, 9-9, 18-4, 63-1, 80-7, 137-2, and 154-7.
Figure 8 shows an alignment showing the variable light chain sequences of the seven distinct antibodies identified. Alignments were generated using CLUSTAL O(1.2.3).
Figure 9 shows an alignment showing the variable heavy chain sequences of the seven distinct antibodies identified. Alignments were generated using CLUSTAL O(1.2.3).
Figure 10 shows the CDR1, CDR2 and CDR3 sequences of the heavy and light chains of the seven distinct antibodies identified, assigned based on the Kabat numbering scheme.
Figure 11 shows the CDR1, CDR2 and CDR3 sequences of the heavy and light chains of the seven distinct antibodies identified, assigned based on the Chothia numbering scheme.

### DETAILED DESCRIPTION

The present invention relates to anti-idiotypic antigen binding molecules, including antibodies, which specifically bind to antigen binding molecules that specifically bind BCMA. Polynucleotides encoding the antigen binding molecules, as well as vectors comprising the polynucleotides, and *in vitro* cells comprising the polynucleotides and vectors, are also disclosed.

Methods of using the disclosed antigen binding molecules are provided. The antigen binding molecules, polynucleotides, vectors, *in vitro* cells and methods described herein may be used in a range of applications, *e.g*., as reagents to detect the presence of moieties comprising the BCMA binding molecule, as well as molecules comprising this sequence and cells presenting such molecules, quantifying the amount of a moiety comprising BCMA binding molecule, as well as molecules comprising this sequence and cells presenting such molecules, screening for moieties comprising the BCMA binding molecule, as well as molecules comprising this sequence and cells presenting such molecules, purifying moieties comprising a BCMA binding molecule, as well as molecules comprising this sequence and cells presenting such molecules, and biomarker studies focused on moieties comprising a BCMA binding molecule, as well as molecules comprising this sequence and cells presenting such molecules. Therapeutic uses are also provided, for example applications in which the biological activity of a moiety comprising a BCMA binding molecule, as well as cells presenting such molecules, is modulated (enhanced or repressed), as well as dose ranging studies related to therapeutics comprising a BCMA binding molecule, as well as molecules comprising this sequence and cells presenting such molecules, and cells presenting such molecules.

The antigen binding molecules (antibodies) disclosed herein were generated from hybridomas generated using B-cells of rabbit origin, but may be readily humanized using standard methods known to those of skill in the art, as well as those described herein. Representative humanized forms of the disclosed antigen binding molecules may be generated as described herein.

### I. Definitions

In order that the present disclosure may be more readily understood, certain terms are first defined. As used in this application, except as otherwise expressly provided herein, each of the following terms shall have the meaning set forth below. Additional definitions are set forth throughout the application. The headings provided herein are not limitations of the various aspects of the disclosure, which aspects should be understood by reference to the specification as a whole.

It is understood that, wherever aspects are described herein with the language "comprising," otherwise analogous aspects described in terms of "consisting of" and/or "consisting essentially of" are also provided.

Units, prefixes, and symbols used herein are provided using their Système International de Unites (SI) accepted form. Numeric ranges are inclusive of the numbers defining the range.

Unless defined otherwise, all technical and scientific terms used herein have the same meaning as commonly understood by one of ordinary skill in the art to which this disclosure is related. For example, Juo, The Concise Dictionary of Biomedicine and Molecular Biology, 2nd ed., (2001), CRC Press; The Dictionary of Cell & Molecular Biology, 5th ed., (2013), Academic Press; and The Oxford Dictionary Of Biochemistry And Molecular Biology, Cammack et al. eds., 2nd ed, (2006), Oxford University Press, provide those of skill in the art with a general dictionary for many of the terms used in this disclosure.

As used herein, the twenty conventional (*e.g*., naturally occurring) amino acids and their abbreviations follow conventional usage. *See, e.g.,* Immunology - A Synthesis (2nd Edition), Golub and Green, eds., Sinauer Assoc., Sunderland, Mass. (1991), which is incorporated herein by reference for any purpose. Stereoisomers (*e.g*., D-amino acids) of the twenty conventional amino acids, unnatural amino acids such as alpha-, alpha-disubstituted amino acids, N-alkyl amino acids, lactic acid, and other unconventional amino acids may also be suitable components for polypeptides of the present invention. Examples of unconventional amino acids include: 4-hydroxyproline, gamma-carboxyglutamate, epsilon-N,N,N-trimethyllysine, e-N-acetyllysine, O-phosphoserine, N-acetylserine, N-formylmethionine, 3-methylhistidine, 5-hydroxylysine, sigma-N-methylarginine, and other similar amino acids and imino acids (*e.g*., 4-hydroxyproline). In the polypeptide notation used herein, the left-hand direction is the amino terminal direction and the right-hand direction is the carboxy-terminal direction, in accordance with standard usage and convention.

As used herein, the term the terms "a" and "an" are used per standard convention and mean one or more, unless context dictates otherwise.

As used herein, the term "about" refers to a value or composition that is within an acceptable error range for the particular value or composition as determined by one of ordinary skill in the art, which will depend in part on how the value or composition is measured or determined, *i.e.,* the limitations of the measurement system. For example, "about" or "comprising essentially of' may mean within one or more than one standard deviation per the practice in the art. Alternatively, "about" or "comprising essentially of' may mean a range of up to 10% (*i.e.,* ±10%). For example, about 5mg may include any number between 4.5 mg and 5.5 mg. Furthermore, particularly with respect to biological systems or processes, the terms may mean up to an order of magnitude or up to 5-fold of a value. When particular values or compositions are provided in the instant disclosure, unless otherwise stated, the meaning of "about" or "comprising essentially of" should be assumed to be within an acceptable error range for that particular value or composition.

As described herein, any concentration range, percentage range, ratio range or integer range is to be understood to be inclusive of the value of any integer within the recited range and, when appropriate, fractions thereof (such as one-tenth and one-hundredth of an integer), unless otherwise indicated.

As used herein, the term "and/or" is to be understood as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or," as used in a phrase such as `A, B, and/or C" is intended to encompass each of the following aspects: A, B, and C; A, B, or C; A or C; A or B; B or C; A and C; A and B; B and C; A (alone); B (alone); and C (alone).

As used herein, the term the use of the alternative (*e.g*., "or") should be understood to mean either one, both, or any combination thereof of the alternatives.

As used herein, the term "allogeneic" refers to any material derived from one individual which is then introduced to another individual of the same species, *e.g*., allogeneic T cell transplantation.

As used herein, the term "antibody" (Ab) includes, without limitation, a glycoprotein immunoglobulin which binds specifically to an antigen. In general, an antibody may comprise at least two heavy (HC) chains and two light (LC) chains interconnected by disulfide bonds, or an antigen binding molecule thereof. Each HC chain comprises a heavy chain variable region (abbreviated herein as VH) and a heavy chain constant region. The heavy chain constant region comprises three constant domains, CH1, CH2 and CH3. Each LC chain comprises a light chain variable region (abbreviated herein as VL) and a light chain constant region. The light chain constant region is comprises one constant domain, CL. The VH and VL regions may be further subdivided into regions of hypervariability, termed complementarity determining regions (CDRs), interspersed with regions that are more conserved, termed framework regions (FR). Each VH and VL comprises three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the Abs may mediate the binding of the immunoglobulin to host tissues or factors, including various cells of the immune system (*e.g*., effector cells) and the first component of the classical complement system (C1q). The term "antibody" also encompasses an intact immunoglobulin or an antigen binding portion thereof that competes with the intact antibody for specific binding, unless otherwise specified. Antigen binding portions may be produced by recombinant DNA techniques or by enzymatic or chemical cleavage of intact antibodies. Antigen binding portions include, inter alia, Fab, Fab', F(ab')2, Fv, domain antibodies (dAbs), fragments including complementarity determining regions (CDRs), single-chain antibodies (scFv), chimeric antibodies, diabodies, triabodies, tetrabodies, and polypeptides that contain at least a portion of an immunoglobulin that is sufficient to confer specific antigen binding to the polypeptide.

The term "antibody" includes, both naturally occurring and non-naturally occurring (recombinantly-produced) antibodies, human and non-human antibodies, monospecific antibodies, multispecific antibodies (including bispecific antibodies), immunoglobulins, synthetic antibodies, tetrameric antibodies comprising two heavy chain and two light chain molecules, an antibody light chain monomer, an antibody heavy chain monomer, an antibody light chain dimer, an antibody heavy chain dimer, an antibody light chain-antibody heavy chain pair, intrabodies (*see, e.g.,* Stocks, (2004) Drug Discovery Today 9(22):960-66), antibody fusions (which term encompasses antibody-drug conjugates) and which are sometimes referred to herein as "antibody conjugates"), heteroconjugate antibodies, single domain antibodies, monovalent antibodies, single chain antibodies or single-chain Fvs (scFv), camelized antibodies, affybodies, Fab fragments, F(ab')₂ fragments, disulfide-linked Fvs (sdFv), anti-idiotypic (anti-Id) antibodies (including, *e.g*., anti-anti-Id antibodies), minibodies, domain antibodies, synthetic antibodies (sometimes referred to herein as "antibody mimetics"), and antigen-binding fragments thereof. In certain embodiments, antibodies described herein refer to polyclonal antibody populations.

A non-human antibody may be humanized using recombinant methods to reduce its immunogenicity in humans, as disclosed herein, with respect to antibodies that specifically bind a BCMA binding molecule, as well as molecules comprising this sequence and cells presenting such molecules. Where not expressly stated, and unless the context indicates otherwise, the term "antibody" also includes an antigen-binding fragment of an antigen binding molecule of any of the aforementioned immunoglobulins, and includes a monovalent and a divalent fragment or portion, and a single chain antibody (*i.e.,* a scFv).

In various embodiments, an antibody specifically binds a BCMA binding molecule, as well as molecules comprising this sequence and cells presenting such molecules. In some embodiments, the antibody specifically binds to a CAR (or component thereof) comprising a BCMA binding molecule, as well as molecules comprising this sequence, and cells presenting such molecules; cells presenting a BCMA binding molecule can, but need not be, an immune cell, such as a T cell.

As used herein, the term "antigen" means any molecule that provokes an immune response or is capable of being bound by an antibody or other antigen binding molecule. The immune response may involve either antibody production, or the activation of specific immunologically-competent cells, or both. Those of skill in the art will readily understand that any macromolecule, including virtually all proteins or peptides (including a BCMA binding molecule, as well as molecules comprising this sequence and cells presenting such molecules), may serve as an antigen. Generally, an antigen may be endogenously expressed, *i.e.* expressed by genomic DNA, or it may be recombinantly expressed, or it may be chemically synthesized. In one particular embodiment, an antigen comprises all or a portion of a BCMA binding molecule a BCMA binding molecule, as well as molecules comprising this sequence, which is optionally conjugated to an adjuvant such as keyhole limpet hemocyanin (KLH), or to an Fc to facilitate screening.

As used herein, the term "antigen binding molecule" means a protein comprising a portion that binds to an antigen or target protein and, optionally, a scaffold or framework portion that allows the antigen binding portion to adopt a conformation that promotes binding of the antigen binding molecule to the antigen. Examples of the representative types of antigen binding molecules include a scFv, a human, mouse or rabbit antibody; a humanized antibody; a chimeric antibody; a recombinant antibody; a single chain antibody; a diabody; a triabody; a tetrabody; a Fab fragment; a F(ab')2 fragment; an IgD antibody; an IgE antibody; an IgM antibody; an IgGl antibody; an IgG2 anti-body; an IgG3 antibody; or an IgG4 antibody, and fragments thereof.

An antigen binding molecule may comprise, for example, an alternative protein scaffold or artificial scaffold with grafted complementarity determining regions (CDRs) or CDR derivatives. Such scaffolds include, but are not limited to, antibody-derived scaffolds comprising mutations introduced to, for example, stabilize the three-dimensional structure of the antigen binding molecule as well as wholly synthetic scaffolds comprising, for example, a biocompatible polymer. *See, e.g.,* Komdorfer et al., 2003, Proteins: Structure, Function, and Bioinformatics, 53(1): 121-129 (2003); Roque et al., Biotechnol. Prog. 20:639-654 (2004). In addition, peptide antibody mimetics ("PAMs") may be used, as well as scaffolds based on antibody mimetics utilizing various components (e.g., fibronectin) as a scaffold. An antigen binding molecule may have, for example, the structure of a naturally occurring immunoglobulin.

An antigen binding molecule may have one or more binding sites. If there is more than one binding site, the binding sites may be identical to one another or they may be different. For example, a naturally occurring human immunoglobulin typically has two identical binding sites, while a "bispecific" or "bifunctional" antibody has two different binding sites, and is capable of specifically binding two different antigens (*e.g*., a BCMA binding molecule and a cell surface activator molecule).

In various embodiments, an antigen binding molecule is an antibody or fragment thereof, including one or more of the complementarity determining regions (CDRs) disclosed herein and shown in FIGURES 4A-11, which specifically bind a BCMA binding molecule, as well as molecules comprising a BCMA binding molecule, and cells presenting such molecules. In further embodiments, the antigen binding molecule binds to a CAR comprising a BCMA binding molecule, as well as molecules comprising a BCMA binding molecule, and may be expressed on an immune cell, such as a T cell.

The term "autologous" refers to any material derived from the same individual to which it is later to be re-introduced. For example, the engineered autologous cell therapy (eACT^{™}) methods described herein involve collection of lymphocytes from a patient, which are then engineered to express a construct, *e.g*., a CAR construct, and then administered back to the same patient.

As used herein, the term "binding affinity" means the strength of the sum total of noncovalent interactions between a single binding site of a molecule (*e.g*., an antigen binding molecule such as an antibody) and its binding partner (*e.g*., an antigen). Unless indicated otherwise, as used herein, "binding affinity" refers to intrinsic binding affinity which reflects a 1:1 interaction between members of a binding pair (*e.g*., antibody and antigen). The affinity of a molecule X for its partner Y may generally be represented by the dissociation constant (K_{D}). Affinity may be measured and/or expressed in a number of ways known in the art, including, but not limited to, equilibrium dissociation constant (K_{D}), and equilibrium association constant (K_{A}). The K_{D} is calculated from the quotient of k_{off}/kₒₙ, whereas K_{A} is calculated from the quotient of kₒₙ/k_{off}. kₒₙ refers to the association rate constant of, *e.g*., an antibody to an antigen, and k_{off} refers to the dissociation of, *e.g*., an antibody to an antigen. The kₒₙ and k_{off} may be determined by standard techniques known to one of ordinary skill in the art, such as BIAcore^{®} or KinExA or surface plasmon resonance.

As used herein, the term "complementarity determining region" or "CDR" means an amino acid sequence that contributes to antigen binding specificity and affinity. Framework regions may aid in maintaining the proper confirmation of the CDRs to promote binding between the antigen binding molecule and an antigen. A number of definitions of the CDRs are commonly in use: Kabat numbering, Chothia numbering, AbM numbering, or contact numbering. The AbM definition is a compromise between the Kabat and Chothia systems, and is used by Oxford Molecular's AbM antibody modelling software. Table A defines CDRs using each numbering system. The contact definition is based on an analysis of the available complex crystal structures.

**Table A**

| **Loop** | **Kabat** | **AbM** | **Chothia** | **Contact** |
|---|---|---|---|---|
| L1 | L24--L34 | L24--L34 | L24--L34 | L30--L36 |
| L2 | L50--L56 | L50--L56 | L50--L56 | L46--L55 |
| L3 | L89--L97 | L89--L97 | L89--L97 | L89--L96 |
| H1 | H31--H35B | H26--H35B | H26--H32..34 | H30--H35B |
| H1 | H31--H35 | H26--H35 | H26--H32 | H30--H35 |
| H2 | H50--H65 | H50--H58 | H52--H56 | H47--H58 |
| H3 | H95--H102 | H95--H102 | H95--H102 | H93--H101 |

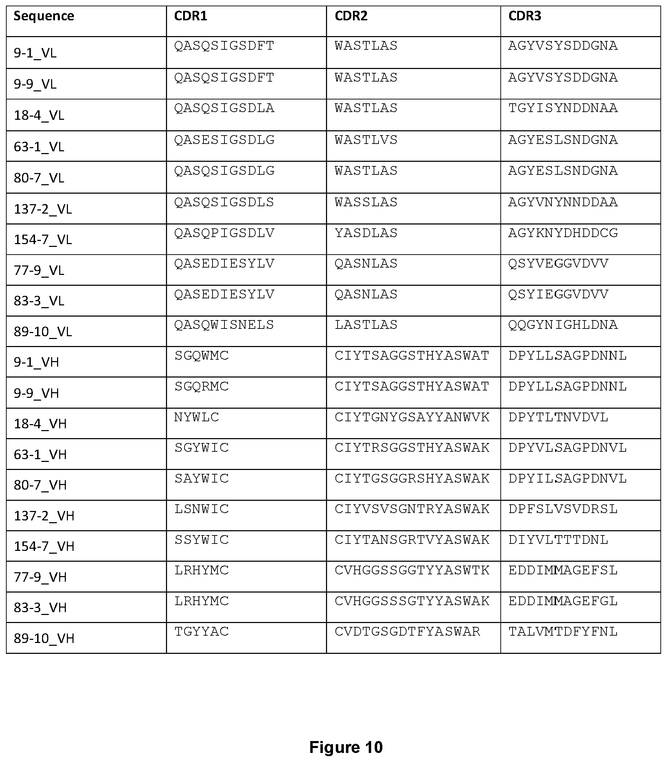

The term "Kabat numbering" and like terms are recognized in the art and refer to a system of numbering amino acid residues in the heavy and light chain variable regions of an antibody, or an antigen binding molecule thereof. In certain aspects, the CDRs of an antibody may be determined according to the Kabat numbering system (*see, e.g.,* Kabat et al. in Sequences of Proteins of Immunological Interest, 5th Ed., NIH Publication 91-3242, Bethesda MD 1991). Using the Kabat numbering system, CDRs within an antibody heavy chain molecule are typically present at amino acid positions 31 to 35, which optionally may include one or two additional amino acids, following 35 (referred to in the Kabat numbering scheme as 35A and 35B) (CDR1), amino acid positions 50 to 65 (CDR2), and amino acid positions 95 to 102 (CDR3). Using the Kabat numbering system, CDRs within an antibody light chain molecule are typically present at amino acid positions 24 to 34 (CDR1), amino acid positions 50 to 56 (CDR2), and amino acid positions 89 to 97 (CDR3). In some embodiments, the CDRs of the antibodies described herein may be described according to the Kabat numbering scheme, as shown in Figure 10 (although they may readily be construed in other numbering systems using Table A above).

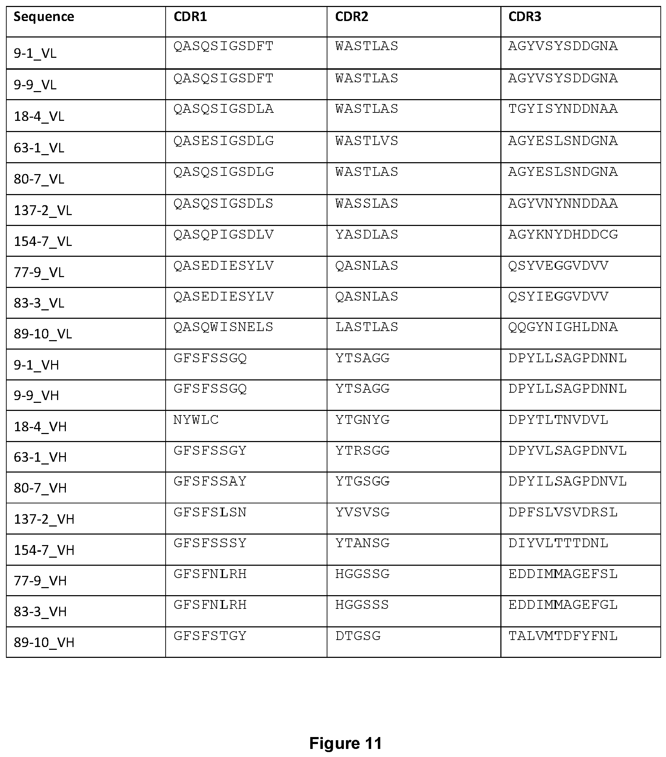

In certain aspects, the CDRs of an antibody may be determined according to the Chothia numbering scheme, which refers to the location of immunoglobulin structural loops (*see, e.g.,* Chothia C & Lesk AM, (1987), J Mol Biol 196: 901-917; Al-Lazikani B et al., (1997) J Mol Biol 273: 927-948; Chothia C et al., (1992) J Mol Biol 227: 799-817; Tramontane A et al., (1990) J Mol Biol 215(1): 175-82; and U.S. Patent No. 7,709,226). Typically, when using the Kabat numbering convention, the Chothia CDR-H1 loop is present at heavy chain amino acids 26 to 32, 33, or 34, the Chothia CDR-H2 loop is present at heavy chain amino acids 52 to 56, and the Chothia CDR-H3 loop is present at heavy chain amino acids 95 to 102, while the Chothia CDR-L1 loop is present at light chain amino acids 24 to 34, the Chothia CDR-L2 loop is present at light chain amino acids 50 to 56, and the Chothia CDR-L3 loop is present at light chain amino acids 89 to 97. The end of the Chothia CDR-HI loop when numbered using the Kabat numbering convention varies between H32 and H34 depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34). See Table A. In some embodiments, the CDRs of the antibodies described herein have been determined according to the Chothia numbering scheme, as shown in FIGURE 11.

As used herein, a "conservative amino acid substitution" is one in which the amino acid residue is replaced with an amino acid residue having a similar side chain. Families of amino acid residues having side chains have been defined in the art. These families include amino acids with basic side chains (*e.g.,* lysine, arginine, histidine), acidic side chains *(e.g.,* aspartic acid, glutamic acid), uncharged polar side chains (*e.g*., glycine, asparagine, glutamine, serine, threonine, tyrosine, cysteine, tryptophan), nonpolar side chains (*e.g*., alanine, valine, leucine, isoleucine, proline, phenylalanine, methionine), beta-branched side chains (*e.g*., threonine, valine, isoleucine) and aromatic side chains (*e.g*., tyrosine, phenylalanine, tryptophan, histidine). In certain embodiments, one or more amino acid residues within a CDR(s) or within a framework region(s) of an antibody or antigen binding molecule provided herein (or fragment thereof) may be replaced with an amino acid residue with a similar side chain.

Conservative amino acid substitutions, which are encompassed by the present disclosure, may encompass non-naturally occurring amino acid residues, which are typically incorporated by chemical peptide synthesis rather than by synthesis in biological systems. These include peptidomimetics and other reversed or inverted forms of amino acid moieties. Naturally occurring residues may be divided into classes based on common side chain properties:
hydrophobic: norleucine, Met, Ala, Val, Leu, Ile;
neutral hydrophilic: Cys, Ser, Thr, Asn, Gln;
acidic: Asp, Glu;
basic: His, Lys, Arg;
residues that influence chain orientation: Gly, Pro; and
aromatic: Trp, Tyr, Phe.

Non-conservative substitutions may involve the exchange of a member of one of these classes for a member from another class. Such substituted residues may be introduced, for example, into regions of a human antibody that are homologous with non-human antibodies, or into the non-homologous regions of the molecule. Exemplary conservative amino acid substitutions are set forth in Table B below.

**Table B**

| **Original Residues** | **Exemplary Substitutions** | **Preferred Substitutions** |
|---|---|---|
| Ala | Val, Leu, Ile | Val |
| Arg | Lys, Gln, Asn | Lys |
| Asn | Gln | Gln |
| Asp | Glu | Glu |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric acid, Gln, Asn | Arg |
| Met | Leu, Phe, Ile | Leu |
| Phe | Leu, Val, Ile, Ala,Tyr | Leu |
| Pro | Ala | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

As used herein, the terms "constant region" and "constant domain" are interchangeable and have a meaning common in the art. The constant region is an antibody portion, *e.g*., a carboxyl terminal portion of a light and/or heavy chain which is not directly involved in binding of an antibody to antigen but which may exhibit various effector functions, such as interaction with the Fc receptor. The constant region of an immunoglobulin molecule generally has a more conserved amino acid sequence relative to an immunoglobulin variable domain.

As used herein, the term "cross competes" means the situation in which the interaction between an antigen and a first antigen binding molecule or binding fragment thereof blocks, limits, inhibits, or otherwise reduces the ability of a reference antigen binding molecule or binding fragment thereof to interact with the antigen. Cross competition may be complete, *e.g*., binding of the binding molecule to the antigen completely blocks the ability of the reference binding molecule to bind the antigen, or it may be partial, *e.g*., binding of the binding molecule to the antigen reduces the ability of the reference binding molecule to bind the antigen. In certain embodiments, an antigen binding molecule that cross competes with a reference antigen binding molecule binds the same or an overlapping epitope as the reference antigen binding molecule. In other embodiments, the antigen binding molecule that cross competes with a reference antigen binding molecule binds a different epitope than the reference antigen binding molecule. Numerous types of competitive binding assays may be used to determine if one antigen binding molecule competes with another, for example: solid phase direct or indirect radioimmunoassay (RIA); solid phase direct or indirect enzyme immunoassay (EIA); sandwich competition assay (Stahli et al., (1983) Method Enzymol 9:242-53); solid phase direct biotin-avidin EIA (Kirkland et al., (1986) J Immunol 137:3614-19); solid phase direct labeled assay, solid phase direct labeled sandwich assay (Harlow and Lane, 1988, Antibodies, A Laboratory Manual, Cold Spring Harbor Press); solid phase direct label RIA using I¹²⁵ label (Morel et al., (1988) Molec Immunol 25:7-15); solid phase direct biotin-avidin EIA (Cheung et al., (1990) Virology 176:546-52); and direct labeled RIA (Moldenhauer et al., (1990) Scand J Immunol 32:77-82).

The term "derivative" refers to a molecule that includes a chemical modification other than an insertion, deletion, or substitution of amino acids (or nucleic acids). In certain embodiments, derivatives comprise covalent modifications, including, but not limited to, chemical bonding with polymers, lipids, or other organic or inorganic moieties. In certain embodiments, a chemically modified antigen binding molecule (a derivative) may have a greater circulating half-life than an antigen binding molecule that is not chemically modified. In some embodiments, a derivative antigen binding molecule is covalently modified to include one or more water soluble polymer attachments, including, but not limited to, polyethylene glycol, polyoxyethylene glycol, or polypropylene glycol.

As used herein, the term "diabody" or dAB means bivalent antibodies comprising two polypeptide chains, wherein each polypeptide chain comprises VH and VL domains joined by a linker that is too short to allow for pairing between two domains on the same chain, thus allowing each domain to pair with a complementary domain on another polypeptide chain (*see, e.g.,* Holliger et al., (1993) Proc Natl Acad Sci U.S.A. 90:6444-48, Poljak et al., (1994) Structure 2: 1121-23, and Perisic et al., (1994) Strucure 2(12): 1217-26). If the two polypeptide chains of a diabody are identical, then a diabody resulting from their pairing will have two identical antigen binding sites. Polypeptide chains having different sequences may be used to make a diabody with two different antigen binding sites. Similarly, tribodies and tetrabodies are antibodies comprising three and four polypeptide chains, respectively, and forming three and four antigen binding sites, respectively, which may be the same or different.

As used herein, an "epitope" is a term in the art and refers to a localized region of an antigen to which an antibody may specifically bind. An epitope may be, for example, contiguous amino acids of a polypeptide (linear or contiguous epitope) or an epitope can, for example, come together from two or more non-contiguous regions of a polypeptide or polypeptides (conformational, non-linear, discontinuous, or non-contiguous epitope). In certain embodiments, the epitope to which an antibody binds may be determined by, *e.g*., NMR spectroscopy, X-ray diffraction crystallography studies, ELISA assays, hydrogen/deuterium exchange coupled with mass spectrometry (*e.g*., liquid chromatography electrospray mass spectrometry), array-based oligo-peptide scanning assays, and/or mutagenesis mapping (*e.g*., site-directed mutagenesis mapping). For X-ray crystallography, crystallization may be accomplished using any of the known methods in the art (*e.g.,* Giege et al., (1994) Acta Crystallogr D Biol Crystallogr 50(Pt 4): 339-350; McPherson, (1990) Eur J Biochem 189: 1-23; Chayen, (1997) Structure 5: 1269-1274; McPherson, (1976) J Biol Chem 251: 6300-6303). Antibody:antigen crystals may be studied using well known X-ray diffraction techniques and may be refined using computer software such as X-PLOR (Yale University, 1992, distributed by Molecular Simulations, Inc.; *see, e.g.,* Meth Enzymol (1985) Vols 114 & 115, eds Wyckoff *et al.,*), and BUSTER (Bricogne, (1993) Acta Crystallogr D Biol Crystallogr 49(Pt 1): 37-60; Bricogne, (1997) Meth Enzymol 276A: 361-423, ed. Carter; Roversi et al., (2000) Acta Crystallogr D Biol Crystallogr 56(Pt 10): 1316-1323). Mutagenesis mapping studies may be accomplished using any method known to one of skill in the art. *See, e.g.,* Champe et al., (1995) J Biol Chem 270: 1388-94 and Cunningham & Wells, (1989) Science 244: 1081-85 for a description of mutagenesis techniques, including alanine and arginine scanning mutagenesis techniques.

As used herein, the term "Fab fragment" means is a monovalent fragment having the VL, VH, CL and CH domains; a "F(ab')2 fragment" is a bivalent fragment having two Fab fragments linked by a disulfide bridge at the hinge region; a "Fv fragment" has the VH and VL domains of a single arm of an antibody; and a "dAb fragment" has a VH domain, a VL domain, or an antigen-binding fragment of a VH or VL domain.

As used herein, the terms "immunospecifically binds," "immunospecifically recognizes," "specifically binds," and "specifically recognizes" are analogous terms and are used interchangeably in the context of antigen binding molecules, and means that a given molecule preferentially binds to an antigen (*e.g*., epitope or immune complex) as such binding is understood by one skilled in the art. For example, an antigen binding molecule that specifically binds to an antigen may bind to other peptides or polypeptides, but with a comparatively lower affinity as determined by, *e.g*., immunoassays, BIAcore^{®}, KinExA 3000 instrument (Sapidyne Instruments, Boise, ID), or other assays known in the art. In some embodiments, molecules that specifically bind to an antigen bind to the antigen with a K_{A} that is at least 2 logs, 2.5 logs, 3 logs, 4 logs or greater than the K_{A} when the molecules bind to another antigen.

In another embodiment, molecules that specifically bind to an antigen (*e.g.,* a BCMA binding molecule), as well as molecules comprising this sequence and cells presenting such molecules) bind with a dissociation constant (K_{d}) of about 1 × 10⁻⁷ M. In some embodiments, the antigen binding molecule specifically binds an antigen (*e.g.,* a BCMA binding molecule, as well as molecules comprising this sequence and cells presenting such molecules) with "high affinity" when the K_{d} is about 1 × 10⁻⁹ M to about 5 × 10⁻⁹ M. In some embodiments, the antigen binding molecule specifically binds an antigen (*e.g*., an anti-BCMA, as well as molecules comprising this sequence and cells presenting such molecules) with "very high affinity" when the K_{d} is 1 × 10⁻¹⁰ M to about 5 × 10⁻¹⁰ M.

In still another embodiment, molecules that specifically bind to an antigen (*e.g*., a BCMA binding molecule, as well as molecules comprising this sequence and cells presenting such molecules) do not cross react with other proteins under similar binding conditions. In some embodiments, molecules that specifically bind to an antigen (*e.g.,* a BCMA binding molecule, as well as molecules comprising this sequence and cells presenting such molecules) do not cross react with other proteins that do not comprise a BCMA binding molecule, molecules comprising this sequence and cells presenting such molecules. In some embodiments, provided herein is an antibody or fragment thereof that binds to a BCMA binding molecule, as well as molecules comprising this sequence and cells presenting such molecules, with higher affinity than to another unrelated antigen. In certain embodiments, provided herein is an antigen binding molecule (*e.g.,* an antibody) or fragment thereof that binds to a BCMA binding molecule, as well as molecules comprising this sequence and cells presenting such molecules, with a 20%, 25%, 30%, 35%, 40%, 45%, 50%, 55%, 60%, 65%, 70%, 75%, 80%, 85%, 90%, 95% or higher affinity than to another, unrelated antigen as measured by, *e.g.,* a radioimmunoassay, surface plasmon resonance, or kinetic exclusion assay. In some embodiments, the extent of binding of an antigen binding molecule, antibody or antigen binding fragment thereof that specifically binds a BCMA binding molecule, as well as molecules comprising this sequence and cells presenting such molecules, described herein compared to an unrelated protein which does not comprise a BCMA binding molecule, as well as molecules comprising this sequence and cells presenting such molecules, is less than 10%, 15%, or 20% of the binding of the antibody to linker fragment protein as measured by, *e.g.,* a radioimmunoassay.

As used herein, the term "heavy chain" when used in reference to an antibody may refer to any distinct type, *e.g.,* alpha (α), delta (δ), epsilon (ε), gamma (γ) and mu (µ), based on the amino acid sequence of the constant domain, which give rise to IgA, IgD, IgE, IgG and IgM classes of antibodies, respectively, including subclasses of IgG, *e.g.,* IgG₁, IgG₂, IgG₃ and IgG₄.

As used herein, the term "immunoglobulin" means an immune molecule from any of the commonly known isotypes, including but not limited to IgA, secretory IgA, IgG and IgM. IgG subclasses are also well known to those in the art and include but are not limited to human IgG1, IgG2, IgG3 and IgG4. Many of the molecules described herein are immunoglobulins. As used herein, "isotype" means the antibody class or subclass (*e.g*., IgM or IgG1) that is encoded by the heavy chain constant region genes.

An immunoglobulin is a tetrameric molecule, normally composed of two identical pairs of polypeptide chains, each pair having one "light" (about 25 kDa) and one "heavy" chain (about 50-70 kDa). The amino-terminal portion of each chain includes a variable region of about 100 to 130 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgG, IgA, or IgE, respectively. Within light and heavy chains, the variable and constant regions are joined by a "J" region of about 12 or more amino acids, with the heavy chain also including a "D" region of about 10 more amino acids. See generally, Berzofsky & Berkower, Ch. 7 in Fundamental Immunology (Paul, W., ed., Lippincott Williams & Wilkins (2012); which chapter and volume is incorporated by reference in its entirety for all purposes). The variable regions of each light/heavy chain pair form the antibody binding site such that an intact immunoglobulin has two primary binding sites.

Naturally occurring immunoglobulin chains exhibit the same general structure of relatively conserved framework regions (FR) joined by three hypervariable regions, also called complementarity determining regions or "CDRs." From N-terminus to C-terminus, both light and heavy chains comprise the domains FR1, CDR1, FR2, CDR2, FR3, CDR3 and FR4. The assignment of amino acids to each domain may be done in accordance with the definitions of Kabat (*see, e.g.,* Kabat et al. in Sequences of Proteins of Immunological Interest, 5th Ed., NIH Publication 91-3242, Bethesda MD (1991)) or Chothia (Chothia, used herein, (*see, e.g.,* Chothia & Lesk (1987), J. Mol. Biol. 196:901-917; Chothia et al., 1989, Nature 342:878-883 or Honegger & Pluckthun (2001), J Mol Biol 309:657-670). The Kabat, Chothia, IGMT and Abm (Oxford Molecular) numbering systems are described more fully herein.

As used herein, the term *"in vitro* cell" refers to any cell that is cultured *ex vivo.* An *in vitro* cell may include a human cell such as a T cell or dendritic cell, or it may include CHO, sP2/0, rabbit and other non-human cells.

As used herein, the term "light chain" when used in reference to an antibody may refer to any distinct type, *e.g*., kappa (κ) or lambda (λ) based on the amino acid sequence of the constant domains. Light chain amino acid sequences are known in the art. In specific embodiments, the light chain is a human light chain.

The term "neutralizing" refers to an antigen binding molecule, scFv, antibody, or a fragment thereof, that binds to a ligand (*e.g.,* a moiety comprising a BCMA binding molecule, as well as molecules comprising this sequence and cells presenting such molecules) and prevents or reduces the biological effect of that ligand. In some embodiments, the antigen binding molecule, scFv, antibody, or a fragment thereof, directly blocking a binding site on the ligand or otherwise alters the ligand's ability to bind through indirect means (such as structural or energetic alterations in the ligand). In some embodiments, the antigen binding molecule, scFv, antibody, or a fragment thereof prevents the protein to which it is bound from performing a biological function.

As used herein, the term "patient" means any human who is being treated for an abnormal physiological condition, such as cancer or has been formally diagnosed with a disorder, those without formally recognized disorders, those receiving medical attention, those at risk of developing the disorders, etc. The terms "subject" and "patient" are used interchangeably herein and include both human and non-human animal subjects.

As used herein, the terms "peptide," "polypeptide," and "protein" are used interchangeably herein, and mean a compound comprising amino acid residues covalently linked by peptide bonds. A protein or peptide must contain at least two amino acids, but no limitation is placed on the maximum number of amino acids that may comprise a protein's or peptide's sequence. The term polypeptide encompasses any peptide or protein comprising two or more amino acids joined to each other by peptide bonds. As used herein, the term refers to both short chains, which also commonly are referred to as peptides, oligopeptides and oligomers, and to longer chains, which generally are referred to as proteins. "Polypeptides" include, for example, biologically active fragments, substantially homologous polypeptides, oligopeptides, homodimers, heterodimers, variants of polypeptides, modified polypeptides, derivatives, analogs, fusion proteins, among others. The term "polypeptide" includes natural peptides, recombinant peptides, synthetic peptides, or a combination thereof.

In some aspects, the polypeptides and/or proteins have deletions from, additions to, and/or substitutions of one or more amino acids of antigen binding molecule. Useful polypeptide fragments may include immunologically functional fragments of antigen binding molecules, including not limited to one or more CDR regions, variable domains of a heavy and/or light chain, a portion of other portions of an antibody chain, and the like. Moieties that may be substituted for one or more amino acids of an antigen binding molecule include, *e.g.,* D or L forms of amino acids, an amino acid different from the amino acid normally found in the same position of an antigen binding molecule (relative to SEQ ID NOs: 2-73), deletions, non-naturally occurring amino acids, and chemical analogs of amino acids.

Peptide analogs are commonly used in the pharmaceutical industry as non-peptide drugs with properties analogous to those of the template peptide and form an aspect of the instant disclosure. These types of non-peptide compound are termed "peptide mimetics" or "peptidomimetics." *See, e.g.,* Fauchere, (1986) Adv. Drug Res. (Testa, ed.) 15:29-69; Veber & Freidinger, (1985) TINS, p.392; and Evans et al., (1987) J. Med. Chem, 30: 1229-39, which are incorporated herein by reference for any purpose.

Polypeptides, peptides, proteins and analogous molecules comprising a BCMA binding molecule, as well as molecules comprising this sequence and cells presenting such molecules, are specifically encompassed by the terms.

As used herein, the term "percent identity" means the percent of identical residues between the amino acids or nucleotides in the compared molecules. For these calculations, gaps in alignments (if any) must be addressed by a particular mathematical model or computer program (*i.e.,* an "algorithm"). Methods that may be used to calculate the identity of the aligned nucleic acids or polypeptides include those described in Computational Molecular Biology, (Lesk, ed.), (1988) New York: Oxford University Press; Biocomputing Informatics and Genome Projects, (Smith, ed.), 1993, New York: Academic Press; Computer Analysis of Sequence Data Part I, (Griffin and Griffin, eds.), 1994, New Jersey: Humana Press; von Heinje, (1987) Sequence Analysis in Molecular Biology, New York: Academic Press; Sequence Analysis Primer, (Gribskov and Devereux, eds.), 1991, New York: M. Stockton Press; and Carillo et al., (1988) J. Applied Math. 48:1073.

In calculating percent identity, the sequences being compared are aligned in a way that gives the largest match between the sequences. The computer program used to determine percent identity may be, *e.g.,* MOE (Chemical Computing Group) or DNASTAR (University of Wisconsin, Madison, WI). The computer algorithm GAP may be used to align the two polypeptides or polynucleotides for which the percent sequence identity is to be determined. The sequences are aligned for optimal matching of their respective amino acid or nucleotide (the "matched span," as determined by the algorithm). A gap opening penalty (which is calculated as 3x the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm. In certain embodiments, a standard comparison matrix (see, *e.g*., Dayhoff et al., (1978) Atlas of Protein Sequence and Structure 5:345-352 for the PAM 250 comparison matrix; Henikoff et al., (1992) Proc. Natl. Acad. Sci. U.S.A. 89: 10915-10919 for the BLOSUM 62 comparison matrix) is also used by the algorithm.

Certain alignment schemes for aligning two amino acid sequences may result in matching of only a short region of the two sequences, and this small aligned region may have very high sequence identity even though there is no significant relationship between the two full-length sequences. Accordingly, the selected alignment method (*e.g*., the GAP program) may be adjusted if desired to result in an alignment that spans at least 50 contiguous amino acids of the target polypeptide.

As used herein, the terms "single-chain antibody" and "single chain fragment variable (scFv)" are used interchangeably and mean an antigen binding molecule in which a VL and a VH region are joined via a linker to form a continuous protein chain wherein the linker is long enough to allow the protein chain to fold back on itself and form a monovalent antigen binding site (*see, e.g.,* Bird et al., (1988) Science 242:423-26 and Huston et al., (1988) Proc. Natl. Acad. Sci. U.S.A. 85:5879-83 (1988).

A "therapeutically effective amount," "effective dose," "effective amount," or "therapeutically effective dosage" of a therapeutic agent, (*e.g.,* a moiety comprising a BCMA binding molecule, as well as molecules comprising this sequence and cells presenting such molecules), is any amount that, when used alone or in combination with another therapeutic agent, protects a subject against the onset of a disease or promotes disease regression evidenced by a decrease in severity of disease symptoms, an increase in frequency and duration of disease symptom-free periods, or a prevention of impairment or disability due to the disease affliction. The ability of a therapeutic agent to promote disease regression may be evaluated using a variety of methods known to the skilled practitioner, such as in human subjects during clinical trials, in animal model systems predictive of efficacy in humans, or by assaying the activity of the agent in *in vitro* assays.

The terms "transduction" and "transduced" refer to the process whereby foreign DNA is introduced into a cell via viral vector (s*ee* Hartl and Jones (1997) "Genetics: Principles and Analvsis," 4th ed, Jones & Bartlett). In some embodiments, the vector is a retroviral vector, a DNA vector, a RNA vector, an adenoviral vector, a baculoviral vector, an Epstein Barr viral vector, a papovaviral vector, a vaccinia viral vector, a herpes simplex viral vector, an adenovirus associated vector, a lentiviral vector, or any combination thereof.

As used herein, the terms "variable region" or "variable domain" are used interchangeably and mean a portion of an antibody, generally, a portion of a light or heavy chain, typically about the amino-terminal end of the antibody and comprising about 100-130 amino acids in the heavy chain and about 90 to 115 amino acids in the light chain, which differ extensively in sequence among antibodies and are used in the binding and specificity of a particular antibody for its particular antigen. The variability in sequence is concentrated in those regions called complementarity determining regions (CDRs) while the more highly conserved regions in the variable domain are called framework regions (FR). The CDRs of the light and heavy chains are primarily responsible for the interaction and specificity of the antibody with antigen.

In certain embodiments, the variable region of an antigen binding molecule is a human variable region. In further embodiments, the variable region comprises rodent, human or murine CDRs and human framework regions (FRs). In further embodiments, the variable region is a primate (*e.g*., a non-human primate) variable region. In yet further embodiments, the variable region is a rabbit variable region. In other embodiments, the variable region comprises human CDRs and non-human (*e.g*., rabbit, murine, rat or non-human primate) framework regions (FRs). In other embodiments, the variable region comprises non-human (*e.g*., rabbit, murine, rat or non-human primate) CDRs and human framework regions (FRs).

The terms "VH," "VH domain" and "VH chain" are used interchangeably and mean the heavy chain variable region of an antigen binding molecule, antibody or an antigen binding fragment thereof.

The terms "VL," "VL domain" and "VL chain" are used interchangeably and mean the light chain variable region of an antigen binding molecule, antibody or an antigen binding fragment thereof.

Various aspects of the invention are described in further detail in the following subsections.

### II. Antigen Binding Molecules and Polynucleotides Encoding the Same

The present disclosure is directed to antigen binding molecules, including antibodies, that specifically bind a BCMA binding molecule, as well as molecules comprising this sequence and cells presenting such molecules, and/or those which cross compete with one or more antigen binding molecules described herein (*i.e*., one or more of those described in FIGURES 4A-11 and/or disclosed in the appended Sequence Listing). Polynucleotides encoding the antigen binding molecules are also provided, and form an aspect of the instant disclosure.

An antibody or antigen binding molecule encoded of the present invention may be single chained or double chained. In some embodiments, the antibody or antigen binding molecule is single chained. In certain embodiments, the antigen binding molecule is selected from the group consisting of an scFv, a Fab, a Fab', a Fv, a F(ab')₂, a dAb, and any combination thereof. In one particular embodiment, the antibody or antigen binding molecule comprises an scFv.

In certain embodiments, an antigen binding molecule such as an antibody comprises a single chain, wherein the heavy chain variable region and the light chain variable region are connected by a linker (*e.g.,* an scFv). In some embodiments, the VH is located at the N terminus of the linker and the VL is located at the C terminus of the linker. In other embodiments, the VL is located at the N terminus of the linker and the VH is located at the C terminus of the linker. In some embodiments, the linker comprises at least about 5, at least about 8, at least about 10, at least about 13, at least about 15, at least about 18, at least about 20, at least about 25, at least about 30, at least about 35, at least about 40, at least about 45, at least about 50, at least about 60, at least about 70, at least about 80, at least about 90, or at least about 100 amino acids. In some embodiments, the linker comprises between about 8 amino acids and about 18 amino acids (*e.g*., 10 amino acids).

In some embodiments, the antigen binding molecules of the present invention specifically bind to a BCMA binding molecule, as well as molecules comprising this sequence and cells presenting such molecules. In certain embodiments, an antigen binding molecule of the present disclosure specifically binds a BCMA binding molecule, as well as molecules comprising this sequence and cells presenting such molecules with a K_{D} of less than 1 × 10⁻⁶ M, less than 1 × 10⁻⁷ M, less than 1 × 10⁻⁸ M, or less than 1 × 10⁻⁹ M. In one particular embodiment, an antigen binding molecule specifically binds to a BCMA binding molecule, as well as molecules comprising this sequence and cells presenting such molecules, with a K_{D} of less than 1 × 10⁻⁷ M. In another embodiment, an antigen binding molecule specifically binds a BCMA binding molecule, as well as molecules comprising this sequence and cells presenting such molecules, with a K_{D} of less than 1 × 10⁻⁸ M. In some embodiments, an antigen binding molecule binds a BCMA binding molecule, as well as molecules comprising this sequence and cells presenting such molecules, with a K_{D} of about 1 × 10⁻⁷ M, about 2 × 10⁻⁷ M, about 3 × 10⁻⁷ M, about 4 × 10⁻⁷ M, about 5 × 10⁻⁷ M, about 6 × 10⁻⁷ M, about 7 × 10⁻⁷ M, about 8 × 10⁻⁷ M, about 9 × 10⁻⁷ M, about 1 × 10⁻⁸ M, about 2 × 10⁻⁸ M, about 3 × 10⁻⁸ M, about 4 × 10⁻⁸ M, about 5 × 10⁻⁸ M, about 6 × 10⁻⁸ M, about 7 × 10⁻⁸ M, about 8 × 10⁻⁸ M, about 9 × 10⁻⁸ M, about 1 × 10⁻⁹ M, about 2 × 10⁻⁹ M, about 3 × 10⁻⁹ M, about 4 × 10⁻⁹ M, about 5 × 10⁻⁹ M, about 6 × 10⁻⁹ M, about 7 × 10⁻⁹ M, about 8 × 10⁻⁹ M, about 9 × 10⁻⁹ M, about 1 × 10⁻¹⁰ M, or about 5 × 10⁻¹⁰ M. K_{D} may be calculated using standard methodologies, as described herein.

In specific embodiments, an antigen binding molecule of the instant disclosure is an antibody identified herein as Clones 9-1, 9-9, 18-4, 63-1, 80-7, 137-2, and 154-7, and each comprises the heavy and light chain amino acid, coding, variable, and CDR sequences, as provided and labeled herein.

In some embodiments, the antigen binding molecules of the present disclosure are antibodies and antigen binding fragments thereof. In some embodiments, the antibodies of the present disclosure comprise at least one CDR set forth in Figures 10 and 11. In another aspect, the present disclosure provides hybridomas capable of producing the antibodies disclosed herein, and also methods of producing antibodies from hybridomas, as described herein and as known in the art.

Humanized antibodies are described herein and may be prepared by known techniques. In some embodiments, a humanized monoclonal antibody comprises the variable domain of a murine or rabbit antibody (or all or part of the antigen binding site thereof) and a constant domain derived from a human antibody. Alternatively, a humanized antibody fragment may comprise an antigen binding site of a murine or rabbit monoclonal antibody and a variable domain fragment (lacking the antigen binding site) derived from a human antibody. Procedures for the production of engineered monoclonal antibodies include those described in Riechmann et al., (1988) Nature 332:323, Liu et al., (1987) Proc. Nat. Acad. Sci. USA 84:3439, Larrick et al., (1989) Bio/Technology 7:934, and Winter et al., (1993) TIPS 14:139. In some embodiments, the chimeric antibody is a CDR grafted antibody. Techniques for humanizing antibodies are discussed in, *e.g.,* U.S. Pat. Nos. 5,869,619; 5,225,539; 5,821,337; 5,859,205; 6,881,557; Padlan et al., (1995) FASEB J. 9:133-39; Tamura et al., (2000) J. Immunol. 164:1432-41; Zhang et al., (2005) Mol. Immunol. 42(12):1445-1451; Hwang et al., Methods. (2005) 36(1):35-42; Dall'Acqua et al., (2005) Methods 36(1):43-60; and Clark, (2000) Immunology Today 21(8):397-402.

An antigen binding molecule of the present invention may also be a fully human monoclonal antibody. Fully human monoclonal antibodies may be generated by any number of techniques with which those having ordinary skill in the art will be familiar. Such methods include, but are not limited to, Epstein Barr Virus (EBV) transformation of human peripheral blood cells (*e.g.,* containing B lymphocytes), *in vitro* immunization of human B-cells, fusion of spleen cells from immunized transgenic mice carrying inserted human immunoglobulin genes, isolation from human immunoglobulin V region phage libraries, or other procedures as known in the art and based on the disclosure herein.

Procedures have been developed for generating human monoclonal antibodies in non-human animals. For example, mice in which one or more endogenous immunoglobulin genes have been inactivated by various means have been prepared. Human immunoglobulin genes have been introduced into the mice to replace the inactivated mouse genes. In this technique, elements of the human heavy and light chain locus are introduced into strains of mice derived from embryonic stem cell lines that contain targeted disruptions of the endogenous heavy chain and light chain loci (see also Bruggemann et al., (1997) Curr. Opin. Biotechnol. 8:455-58).

Examples of techniques for production and use of transgenic animals for the production of human or partially human antibodies are described in U.S. Pat. Nos. 5,814,318, 5,569,825, and 5,545,806; Davis et al., Antibody Engineering: Methods and Protocols, (Lo, ed) Humana Press, NJ, 191-200 (2003); Kellermann et al., (2002) Curr Opin Biotechnol. 13:593-97; Russel et al., (2000) Infect Immun. 68:1820-26; Gallo et al., (2000) Eur J. Immun. 30:534-40; Davis et al., (1999) Cancer Metastasis Rev. 18:421-25; Green, (1999) J Immunol Methods 231:11-23; Jakobovits, (1998) Advanced Drug Delivery Reviews 31:33-42; Green et al., (1998) J Exp Med. 188:483-95; Jakobovits, (1998) Exp. Opin. Invest. Drugs. 7:607-14; Tsuda et al., (1997) Genomics, 42:413-21; Mendez et al., (1997) Nat. Genet. 15:146-56; Jakobovits, (1994) Curr Biol. 4:761-63; Arbones et al., (1994) Immunity 1:247-60; Green et al., (1994) Nat. Genet. 7:13-21; Jakobovits et al., (1993) Nature 362:255-58; Jakobovits et al., (1993) Proc Natl Acad Sci USA 90:2551-55; Chen et al., (1993) Intl Immunol 5:647-656; Choi et al., (1993) Nature Genetics 4:117-23; Fishwild et al., (1996) Nature Biotechnology 14:845-51; Lonberg et al., (1994) Nature 368: 856-59; Lonberg, (1994) Handbook of Experimental Pharmacology 113: 49-101; Neuberger, (1996) Nature Biotech 14:826; Taylor et al., (1992) Nucleic Acids Research 20:6287-95; Taylor et al., (1994) Intl Immunol 6:579-91; Tomizuka et al., (1997) Nature Genetics 16:133-43; Tomizuka et al., (2000) Proc Nat Acad Sci USA 97:722-27; Tuaillon et al., (1993) Proc Nat Acad Sci USA 90:3720-24; Tuaillon et al., (1994) J Immunol 152:2912-20.; Lonberg et al., (1994) Nature 368:856; Taylor et al., (1994) Intl Immunol 6:579; U.S. Pat. No. 5,877,397; Bruggemann et al., (1997) Curr. Opin. Biotechnol. 8:455-58; Jakobovits et al., (1995) Ann. N.Y. Acad. Sci. 764:525-35.

An additional method for obtaining antigen binding molecules of the invention is by the use of phage display, which is well-established for this purpose. *See, e.g.,* Winter et al., (1994) Ann. Rev. Immunol. 12:433-55; Burton et al., (1994) Adv. Immunol 57:191-280. Human or murine immunoglobulin variable region gene combinatorial libraries may be created in phage vectors that may be screened to select Ig fragments (Fab, Fv, sFv, or multimers thereof) that bind a BCMA binding molecule, as well as molecules comprising this sequence and cells presenting such molecules. *See,* e.g., U.S. Pat. No. 5,223,409; Huse et al., (1989) Science 246:1275-81; Sastry et al., (1989) Proc. Natl. Acad. Sci. USA 86:5728-32; Alting-Mees et al., (1990) Strategies in Molecular Biology 3:1-9; Kang et al., (1991) Proc. Natl. Acad. Sci. USA 88:4363-66; Hoogenboom et al., (1992) J. Mol. Biol. 227:381-388; Schlebusch et al., (1997) Hybridoma 16:47-52 and references cited therein. For example, a library containing a plurality of polynucleotide sequences encoding Ig variable region fragments may be inserted into the genome of a filamentous bacteriophage, such as M13 or lambda phage (λImmunoZap^{™}(H) and λImmunoZap^{™}(L) vectors (Stratagene, La Jolla, Calif) may also be used in this approach) or a variant thereof, in frame with the sequence encoding a phage coat protein.

Briefly, mRNA is isolated from a B-cell population, and used to create heavy and light chain immunoglobulin cDNA expression libraries in the λImmunoZap^{™}(H) and λImmunoZap^{™}(L) vectors. These vectors may be screened individually or co-expressed to form Fab fragments or antibodies. Positive plaques may subsequently be converted to a non-lytic plasmid that allows high level expression of monoclonal antibody fragments from *E. coli.*

In some embodiments, in a hybridoma the variable regions of a gene expressing a monoclonal antibody of interest are amplified using nucleotide primers. These primers may be synthesized by one of ordinary skill in the art, or may be purchased from commercial sources, which also sell primers for mouse and human variable regions including, among others, primers for V_{Ha}, V_{Hb}, V_{Hc}, V_{Hd}, C_{H1}, V_{L} and C_{L} regions). These primers may be used to amplify heavy or light chain variable regions, which may then be inserted into vectors such as λImmunoZap^{™}(H) and λImmunoZap^{™}(L) (Stratagene), respectively. These vectors may then be introduced into *E. coli,* yeast, or mammalian-based systems for expression. Large amounts of a single-chain protein containing a fusion of the V_{H} and V_{L} domains may be produced using these methods.

Once cells producing the antigen binding molecules provided herein have been obtained using any of the above-described immunization and other techniques, the specific antibody genes may be cloned by isolating and amplifying DNA or mRNA therefrom according to standard procedures as described herein. The antibodies produced therefrom may be sequenced and the CDRs identified and the DNA coding for the CDRs may be manipulated as described previously to generate other antibodies according to the invention.

It will be understood by one skilled in the art that some proteins, such as antibodies, may undergo a variety of posttranslational modifications. The type and extent of these modifications often depends on the host cell line used to express the protein as well as the culture conditions. Such modifications may include variations in glycosylation, methionine oxidation, diketopiperizine formation, aspartate isomerization and asparagine deamidation. A frequent modification is the loss of a carboxy-terminal basic residue (such as lysine or arginine) due to the action of carboxypeptidases (as described in Harris, (1995) J Chromatog 705: 129-34.

An alternative method for production of a murine monoclonal antibody is to inject the hybridoma cells into the peritoneal cavity of a syngeneic mouse, for example, a mouse that has been treated (*e.g*., pristane-primed) to promote formation of ascites fluid containing the monoclonal antibody. Monoclonal antibodies may be isolated and purified by a variety of well-established techniques. Such isolation techniques include affinity chromatography with Protein-A Sepharose, size-exclusion chromatography, and ion-exchange chromatography (*see, e.g.,* Baines and Thorpe, (1992) in Methods in Molecular Biology, 10:79-104 (The Humana Press). Monoclonal antibodies may be purified by affinity chromatography using an appropriate ligand selected based on particular properties of the antibody (*e.g*., heavy or light chain isotype, binding specificity, etc.). Examples of a suitable ligand, immobilized on a solid support, include Protein A, Protein G, an anti-constant region (light chain or heavy chain) antibody, and an anti-idiotype antibody.

Although the disclosed antigen binding molecules were produced in a rabbit system, human, partially human, or humanized antibodies may be suitable for many applications, particularly those involving administration of the antibody to a human subject, other types of antigen binding molecules will be suitable for certain applications. Such antibodies may be prepared as described herein and form an aspect of the instant disclosure.

In some embodiments, the antibody or antigen binding molecule that specifically binds to a BCMA binding molecule, molecules comprising this sequence and cells presenting this sequence, comprises any one, two, and/or three VH CDR sequences disclosed herein. In certain embodiments, the antibody or antigen binding molecule comprises a VH CDR1, a VH CDR2, and a VH CDR3 having the amino acid sequence of any VH CDR1, VH CDR2, and VH CDR3 disclosed herein, respectively. In some embodiments, the antibody or antigen binding molecule comprises any one, two, and/or three VL CDR sequences disclosed herein. In certain embodiments, the antibody or antigen binding molecule comprises a VL CDR1, a VL CDR2, and a VL CDR3 having the amino acid sequence of any VL CDR1, VL CDR2, and VL CDR3 disclosed herein, respectively.

In some embodiments, the antigen binding molecule or antibody that specifically binds to a BCMA binding molecule, molecules comprising this sequence and cells presenting this sequence, comprises a VH CDR1, a VH CDR2, and VH CDR3, wherein the VH CDR1, VH CDR2, and VH CDR3 comprise the amino acid sequence of the VH CDR1, VH CDR2, and VH CDR3 sequences presented in FIGURES 10 and 11.

The instant disclosure provides antigen binding molecules that specifically bind to a BCMA binding molecule and subsequences thereof, molecules comprising this sequence and cells presenting such molecules. Antigen binding molecules that cross compete with the disclosed antigen binding molecules disclosed herein for an aspect of the disclosure. In certain embodiments, the antigen binding molecule cross competes with a reference antibody comprising an amino acid sequence selected from SEQ ID NOs: 4-64. In certain embodiments, the antigen binding molecule cross competes with a reference antibody, wherein the reference antibody comprises a VH CDR1 comprising an amino acid sequence of SEQ ID NOs: 38-43, 65-67, 105, 106, 120, or 121. In certain embodiments, the antigen binding molecule cross competes with a reference antibody, wherein the reference antibody comprises a VH CDR2 comprising an amino acid sequence of SEQ ID NOs: 48-53 or 68-73, 107-109, or 122-124. In certain embodiments, the antigen binding molecule cross competes with a reference antibody, wherein the reference antibody comprises a VH CDR3 comprising an amino acid sequence of SEQ ID NOs: 59-64, 110-112, 125-127. In certain embodiments, the antigen binding molecule cross competes with a reference antibody, wherein the reference antibody comprises a VL CDR1 comprising an amino acid sequence of SEQ ID NOs: 32-37, 98, 99, 113, and 114. In certain embodiments, the antigen binding molecule cross competes with a reference antibody, wherein the reference antibody comprises a VL CDR2 comprising an amino acid sequence of SEQ ID NOs: 44-47, 100, 101, 115, and 116. In certain embodiments, the antigen binding molecule cross competes with a reference antibody, wherein the reference antibody comprises a VL CDR3 comprising an amino acid sequence of SEQ ID NOs: 54-58, 102-103, and 117-119.

In some embodiments, the polynucleotides of the present invention encodes an antibody or antigen binding molecule that specifically binds a BCMA binding molecule, as well as molecules comprising these sequences and cells presenting such molecules, wherein the antibody or antigen binding molecule binds the same or an overlapping epitope as a reference antibody disclosed herein (*e.g*., those comprising sequences presented in Figures 4A-11). In certain embodiments, the antibody or antigen binding molecule binds the same or an overlapping epitope as a reference antibody.

### Clone 9-1

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence GFSFSSGQ (SEQ ID NO: 38).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence YTSAGG (SEQ ID NO: 68).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence DPYLLSAGPDNNL (SEQ ID NO: 59).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence GFSFSSGQ (SEQ ID NO: 38); and/or (b) a VH CDR2 comprising, consisting of, or consisting essentially of the amino acid sequence YTSAGG (SEQ ID NO: 68); and/or (c) a VH CDR3 comprising, consisting of, or consisting essentially of the amino acid sequence DPYLLSAGPDNNL (SEQ ID NO: 59).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 11.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 11.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASQSIGSDFT (SEQ ID NO: 32).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence WASTLAS (SEQ ID NO: 44).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence AGYVSYSDDGNA (SEQ ID NO: 54).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASQSIGSDFT (SEQ ID NO: 32).; and/or (b) a VL CDR2 comprising, consisting of, or consisting essentially of the amino acid sequence WASTLAS (SEQ ID NO: 44); and/or (c) a VL CDR3 comprising, consisting of, or consisting essentially of the amino acid sequence AGYVSYSDDGNA (SEQ ID NO: 54).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 25.

In various embodiments, the light chain variable region is 80%, 85%, 90%, 91%, 92%, 93%, 94%, 95%, 96%, 97%, 98% or 99% identical to the light chain variable region sequence of SEQ ID NO: 25.

In some embodiments, an antigen binding molecule comprises (a) a VH comprising the amino acid sequence of SEQ ID NO: 11; and (b) a VL comprising the amino acid sequence of SEQ ID NO: 25. In some embodiments, an antigen binding molecule comprises: (a) a VH CDR1 region comprising the amino acid sequence of SEQ ID NO: 38; (b) a VH CDR2 region comprising the amino acid sequence of SEQ ID NO: 48 or 68; (c) a VH CDR3 region comprising the amino acid sequence of SEQ ID NO: 59; (d) a VL CDR1 region comprising the amino acid sequence of SEQ ID NO: 32; (e) a VL CDR2 region comprising the amino acid sequence of SEQ ID NO: 44; and (f) a VL CDR3 region comprising the amino acid sequence of SEQ ID NO: 54.

In some embodiments, the antibody or antigen binding molecule comprises: (a) a heavy chain variable region comprising the amino acid sequence of SEQ ID NO: 11; and (b) a light chain variable region comprising the amino acid sequence of SEQ ID NO: 25. Nucleotide sequences encoding the heavy chain variable region and the light chain variable region are provided in FIGURES 4A-Band 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 11; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 25.

### Clone 9-9

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence GFSFSSGQ (SEQ ID NO: 38).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence YTSAGG (SEQ ID NO: 68).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence DPYLLSAGPDNNL (SEQ ID NO: 59).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence GFSFSSGQ (SEQ ID NO: 38); and/or (b) a VH CDR2 comprising, consisting of, or consisting essentially of the amino acid sequence YTSAGG (SEQ ID NO: 68); and/or (c) a VH CDR3 comprising, consisting of, or consisting essentially of the amino acid sequence DPYLLSAGPDNNL (SEQ ID NO: 59).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 12.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 12.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASQSIGSDFT (SEQ ID NO: 32).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence WASTLAS (SEQ ID NO: 44).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence AGYVSYSDDGNA (SEQ ID NO: 54).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASQSIGSDFT (SEQ ID NO: 32).; and/or (b) a VL CDR2 comprising, consisting of, or consisting essentially of the amino acid sequence WASTLAS (SEQ ID NO: 44); and/or (c) a VL CDR3 comprising, consisting of, or consisting essentially of the amino acid sequence AGYVSYSDDGNA (SEQ ID NO: 54).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 26.

In various embodiments, the light chain variable region is 80%, 85%, 90%, 91%, 92%, 93%, 94%, 95%, 96%, 97%, 98% or 99% identical to the light chain variable region sequence of SEQ ID NO: 26.

In some embodiments, an antigen binding molecule comprises (a) a VH comprising the amino acid sequence of SEQ ID NO: 12; and (b) a VL comprising the amino acid sequence of SEQ ID NO: 26. In some embodiments, an antigen binding molecule comprises: (a) a VH CDR1 region comprising the amino acid sequence of SEQ ID NO: 38; (b) a VH CDR2 region comprising the amino acid sequence of SEQ ID NO: 48 or 68; (c) a VH CDR3 region comprising the amino acid sequence of SEQ ID NO: 59; (d) a VL CDR1 region comprising the amino acid sequence of SEQ ID NO: 32; (e) a VL CDR2 region comprising the amino acid sequence of SEQ ID NO: 44; and (f) a VL CDR3 region comprising the amino acid sequence of SEQ ID NO: 54.

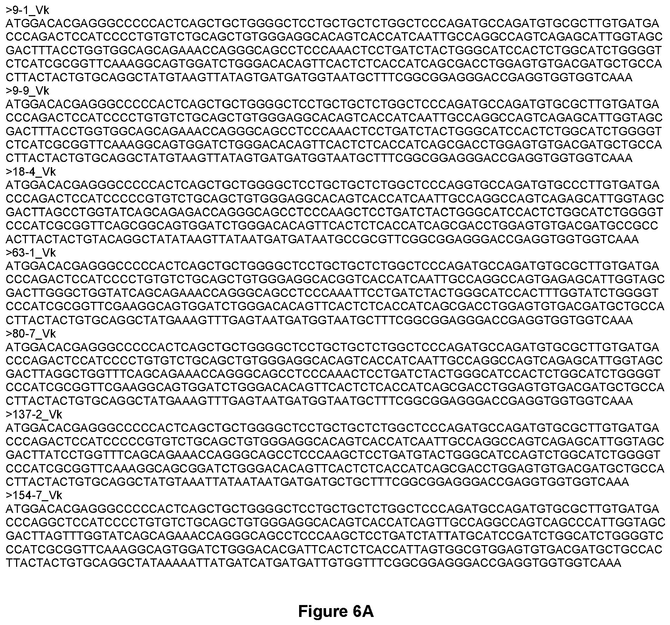

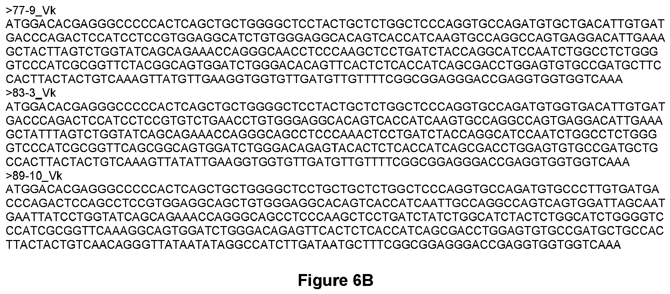

In some embodiments, the antibody or antigen binding molecule comprises: (a) a heavy chain variable region comprising the amino acid sequence of SEQ ID NO: 12; and (b) a light chain variable region comprising the amino acid sequence of SEQ ID NO: 26. Nucleotide sequences encoding the heavy chain variable region and the light chain variable region are provided in FIGURES 4A-B and 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 12; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 26.

### Clone 18-4

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence NYWLC (SEQ ID NO: 40).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence YTGNYG (SEQ ID NO: 69).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence DPYTLTNVDVL (SEQ ID NO: 60).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence NYWLC (SEQ ID NO: 40); and/or (b) a VH CDR2 comprising, consisting of, or consisting essentially of the amino acid sequence YTGNYG (SEQ ID NO: 69); and/or (c) a VH CDR3 comprising, consisting of, or consisting essentially of the amino acid sequence DPYTLTNVDVL (SEQ ID NO: 60).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 13.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 13.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASQSIGSDLA (SEQ ID NO: 33).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence WASTLAS (SEQ ID NO: 44).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence TGYISYNDDNAA (SEQ ID NO: 55).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASQSIGSDLA (SEQ ID NO: 33); and/or (b) a VL CDR2 comprising, consisting of, or consisting essentially of the amino acid sequence WASTLAS (SEQ ID NO: 44); and/or (c) a VL CDR3 comprising, consisting of, or consisting essentially of the amino acid sequence TGYISYNDDNAA (SEQ ID NO: 55).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 27.

In various embodiments, the light chain variable region is 80%, 85%, 90%, 91%, 92%, 93%, 94%, 95%, 96%, 97%, 98% or 99% identical to the light chain variable region sequence of SEQ ID NO: 27.

In some embodiments, an antigen binding molecule comprises (a) a VH comprising the amino acid sequence of SEQ ID NO: 13; and (b) a VL comprising the amino acid sequence of SEQ ID NO: 27. In some embodiments, an antigen binding molecule comprises: (a) a VH CDR1 region comprising the amino acid sequence of SEQ ID NO: 40; (b) a VH CDR2 region comprising the amino acid sequence of SEQ ID NO: 69; (c) a VH CDR3 region comprising the amino acid sequence of SEQ ID NO: 60; (d) a VL CDR1 region comprising the amino acid sequence of SEQ ID NO: 33; (e) a VL CDR2 region comprising the amino acid sequence of SEQ ID NO: 44; and (f) a VL CDR3 region comprising the amino acid sequence of SEQ ID NO: 55.

In some embodiments, the antibody or antigen binding molecule comprises: (a) a heavy chain variable region comprising the amino acid sequence of SEQ ID NO: 13; and (b) a light chain variable region comprising the amino acid sequence of SEQ ID NO: 27. Nucleotide sequences encoding the heavy chain variable region and the light chain variable region are provided in FIGURES 4A-B AND 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 13; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 27.

### Clone 63-1

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence GFSFSSGY (SEQ ID NO: 65).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence YTRSGG (SEQ ID NO: 70).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence DPYVLSAGPDNVL (SEQ ID NO: 61).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence GFSFSSGY (SEQ ID NO: 65); and/or (b) a VH CDR2 comprising, consisting of, or consisting essentially of the amino acid sequence YTRSGG (SEQ ID NO: 70); and/or (c) a VH CDR3 comprising, consisting of, or consisting essentially of the amino acid sequence DPYVLSAGPDNVL (SEQ ID NO: 61).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 14.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 14.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASESIGSDLG (SEQ ID NO: 34).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence WASTLVS (SEQ ID NO: 45).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence AGYESLSNDGNA (SEQ ID NO: 56).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASESIGSDLG (SEQ ID NO: 34); and/or (b) a VL CDR2 comprising, consisting of, or consisting essentially of the amino acid sequence WASTLVS (SEQ ID NO: 45); and/or (c) a VL CDR3 comprising, consisting of, or consisting essentially of the amino acid sequence AGYESLSNDGNA (SEQ ID NO: 56).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 27.

In various embodiments, the light chain variable region is 80%, 85%, 90%, 91%, 92%, 93%, 94%, 95%, 96%, 97%, 98% or 99% identical to the light chain variable region sequence of SEQ ID NO: 28.

In some embodiments, an antigen binding molecule comprises (a) a VH comprising the amino acid sequence of SEQ ID NO: 13; and (b) a VL comprising the amino acid sequence of SEQ ID NO: 28. In some embodiments, an antigen binding molecule comprises: (a) a VH CDR1 region comprising the amino acid sequence of SEQ ID NO: 65; (b) a VH CDR2 region comprising the amino acid sequence of SEQ ID NO: 70; (c) a VH CDR3 region comprising the amino acid sequence of SEQ ID NO: 61; (d) a VL CDR1 region comprising the amino acid sequence of SEQ ID NO: 34; (e) a VL CDR2 region comprising the amino acid sequence of SEQ ID NO: 45; and (f) a VL CDR3 region comprising the amino acid sequence of SEQ ID NO: 56.

In some embodiments, the antibody or antigen binding molecule comprises: (a) a heavy chain variable region comprising the amino acid sequence of SEQ ID NO: 14; and (b) a light chain variable region comprising the amino acid sequence of SEQ ID NO: 28. Nucleotide sequences encoding the heavy chain variable region and the light chain variable region are provided in FIGURES 4A-B AND 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 14; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 28.

### Clone 80-7

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence GFSFSSAY (SEQ ID NO: 66).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence YTGSGG (SEQ ID NO: 71).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence DPYILSAGPDNVL (SEQ ID NO: 62).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence GFSFSSAY (SEQ ID NO: 66); and/or (b) a VH CDR2 comprising, consisting of, or consisting essentially of the amino acid sequence YTGSGG (SEQ ID NO: 71); and/or (c) a VH CDR3 comprising, consisting of, or consisting essentially of the amino acid sequence DPYILSAGPDNVL (SEQ ID NO: 62).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 15.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 15.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASQSIGSDLG (SEQ ID NO: 35).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence WASTLAS (SEQ ID NO: 44).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence AGYESLSNDGNA (SEQ ID NO: 56).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASQSIGSDLG (SEQ ID NO: 35); and/or (b) a VL CDR2 comprising, consisting of, or consisting essentially of the amino acid sequence WASTLAS (SEQ ID NO: 44); and/or (c) a VL CDR3 comprising, consisting of, or consisting essentially of the amino acid sequence AGYESLSNDGNA (SEQ ID NO: 56).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 29.

In various embodiments, the light chain variable region is 80%, 85%, 90%, 91%, 92%, 93%, 94%, 95%, 96%, 97%, 98% or 99% identical to the light chain variable region sequence of SEQ ID NO: 29.

In some embodiments, an antigen binding molecule comprises (a) a VH comprising the amino acid sequence of SEQ ID NO: 15; and (b) a VL comprising the amino acid sequence of SEQ ID NO: 29. In some embodiments, an antigen binding molecule comprises: (a) a VH CDR1 region comprising the amino acid sequence of SEQ ID NO: 66; (b) a VH CDR2 region comprising the amino acid sequence of SEQ ID NO: 71; (c) a VH CDR3 region comprising the amino acid sequence of SEQ ID NO: 62; (d) a VL CDR1 region comprising the amino acid sequence of SEQ ID NO: 35; (e) a VL CDR2 region comprising the amino acid sequence of SEQ ID NO: 44; and (f) a VL CDR3 region comprising the amino acid sequence of SEQ ID NO: 56.

In some embodiments, the antibody or antigen binding molecule comprises: (a) a heavy chain variable region comprising the amino acid sequence of SEQ ID NO: 15; and (b) a light chain variable region comprising the amino acid sequence of SEQ ID NO: 29. Nucleotide sequences encoding the heavy chain variable region and the light chain variable region are provided in FIGURES 4A-B AND 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 15; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 29.

### Clone 137-2

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence GFSFSLSN (SEQ ID NO: 67).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence YVSVSG (SEQ ID NO: 72).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence DPFSLVSVDRSL (SEQ ID NO: 63).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence GFSFSLSN (SEQ ID NO: 67); and/or (b) a VH CDR2 comprising, consisting of, or consisting essentially of the amino acid sequence YVSVSG (SEQ ID NO: 72); and/or (c) a VH CDR3 comprising, consisting of, or consisting essentially of the amino acid sequence DPFSLVSVDRSL (SEQ ID NO: 63).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 16.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 16.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASQSIGSDLS (SEQ ID NO: 36).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence WASSLAS (SEQ ID NO: 46).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence AGYVNYNNDDAA (SEQ ID NO: 57).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASQSIGSDLS (SEQ ID NO: 36); and/or (b) a VL CDR2 comprising, consisting of, or consisting essentially of the amino acid sequence WASSLAS (SEQ ID NO: 46); and/or (c) a VL CDR3 comprising, consisting of, or consisting essentially of the amino acid sequence AGYVNYNNDDAA (SEQ ID NO: 57).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 30.

In various embodiments, the light chain variable region is 80%, 85%, 90%, 91%, 92%, 93%, 94%, 95%, 96%, 97%, 98% or 99% identical to the light chain variable region sequence of SEQ ID NO: 30.

In some embodiments, an antigen binding molecule comprises (a) a VH comprising the amino acid sequence of SEQ ID NO: 16; and (b) a VL comprising the amino acid sequence of SEQ ID NO: 30. In some embodiments, an antigen binding molecule comprises: (a) a VH CDR1 region comprising the amino acid sequence of SEQ ID NO: 67; (b) a VH CDR2 region comprising the amino acid sequence of SEQ ID NO: 72; (c) a VH CDR3 region comprising the amino acid sequence of SEQ ID NO: 63; (d) a VL CDR1 region comprising the amino acid sequence of SEQ ID NO: 36; (e) a VL CDR2 region comprising the amino acid sequence of SEQ ID NO: 46; and (f) a VL CDR3 region comprising the amino acid sequence of SEQ ID NO: 57.

In some embodiments, the antibody or antigen binding molecule comprises: (a) a heavy chain variable region comprising the amino acid sequence of SEQ ID NO: 16; and (b) a light chain variable region comprising the amino acid sequence of SEQ ID NO: 30. Nucleotide sequences encoding the heavy chain variable region and the light chain variable region are provided in FIGURES 4A-B AND 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 16; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 30.

### Clone 154-7

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence GFSFSSSY (SEQ ID NO: 85).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence YTANSG (SEQ ID NO: 73).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence DIYVLTTTDNL (SEQ ID NO: 64).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence GFSFSSSY (SEQ ID NO:85); and/or (b) a VH CDR2 comprising, consisting of, or consisting essentially of the amino acid sequence YTANSG (SEQ ID NO: 73); and/or (c) a VH CDR3 comprising, consisting of, or consisting essentially of the amino acid sequence DIYVLTTTDNL (SEQ ID NO: 64).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 17.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 17.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASQPIGSDLV (SEQ ID NO: 37).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence YASDLAS (SEQ ID NO: 47).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence AGYKNYDHDDCG (SEQ ID NO: 58).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASQPIGSDLV (SEQ ID NO: 37); and/or (b) a VL CDR2 comprising, consisting of, or consisting essentially of the amino acid sequence YASDLAS (SEQ ID NO: 47); and/or (c) a VL CDR3 comprising, consisting of, or consisting essentially of the amino acid sequence AGYKNYDHDDCG (SEQ ID NO: 58).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 31.

In various embodiments, the light chain variable region is 80%, 85%, 90%, 91%, 92%, 93%, 94%, 95%, 96%, 97%, 98% or 99% identical to the light chain variable region sequence of SEQ ID NO: 31.

In some embodiments, an antigen binding molecule comprises (a) a VH comprising the amino acid sequence of SEQ ID NO: 17; and (b) a VL comprising the amino acid sequence of SEQ ID NO: 31. In some embodiments, an antigen binding molecule comprises: (a) a VH CDR1 region comprising the amino acid sequence of SEQ ID NO: 85; (b) a VH CDR2 region comprising the amino acid sequence of SEQ ID NO: 73; (c) a VH CDR3 region comprising the amino acid sequence of SEQ ID NO: 64; (d) a VL CDR1 region comprising the amino acid sequence of SEQ ID NO: 37; (e) a VL CDR2 region comprising the amino acid sequence of SEQ ID NO: 47; and (f) a VL CDR3 region comprising the amino acid sequence of SEQ ID NO: 58.

In some embodiments, the antibody or antigen binding molecule comprises: (a) a heavy chain variable region comprising the amino acid sequence of SEQ ID NO: 17; and (b) a light chain variable region comprising the amino acid sequence of SEQ ID NO: 31. Nucleotide sequences encoding the heavy chain variable region and the light chain variable region are provided in FIGURES 4A-B AND 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 17; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 31.

### Clone 77-9

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence GFSFNLRH (SEQ ID NO: 120).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence HGGSSG (SEQ ID NO: 122).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence EDDIMMAGEFSL (SEQ ID NO: 125).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence GFSFNLRH (SEQ ID NO: 120); and/or (b) a VH CDR2 comprising, consisting of, or consisting essentially of the amino acid sequence HGGSSG (SEQ ID NO: 122); and/or (c) a VH CDR3 comprising, consisting of, or consisting essentially of the amino acid sequence EDDIMMAGEFSL (SEQ ID NO: 125).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 89.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 89.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASEDIESYLV (SEQ ID NO: 113).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence QASNLAS (SEQ ID NO: 115).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence QSYVEGGVDVV (SEQ ID NO: 117).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASEDIESYLV (SEQ ID NO: 113); and/or (b) a VL CDR2 comprising, consisting of, or consisting essentially of the amino acid sequence QASNLAS (SEQ ID NO: 115); and/or (c) a VL CDR3 comprising, consisting of, or consisting essentially of the amino acid sequence QSYVEGGVDVV (SEQ ID NO: 117).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 95.

In various embodiments, the light chain variable region is 80%, 85%, 90%, 91%, 92%, 93%, 94%, 95%, 96%, 97%, 98% or 99% identical to the light chain variable region sequence of SEQ ID NO: 95.

In some embodiments, an antigen binding molecule comprises (a) a VH comprising the amino acid sequence of SEQ ID NO: 89; and (b) a VL comprising the amino acid sequence of SEQ ID NO: 95. In some embodiments, an antigen binding molecule comprises: (a) a VH CDR1 region comprising the amino acid sequence of SEQ ID NO: 120; (b) a VH CDR2 region comprising the amino acid sequence of SEQ ID NO: 122; (c) a VH CDR3 region comprising the amino acid sequence of SEQ ID NO: 125; (d) a VL CDR1 region comprising the amino acid sequence of SEQ ID NO: 113; (e) a VL CDR2 region comprising the amino acid sequence of SEQ ID NO: 115; and (f) a VL CDR3 region comprising the amino acid sequence of SEQ ID NO: 117.

In some embodiments, the antibody or antigen binding molecule comprises: (a) a heavy chain variable region comprising the amino acid sequence of SEQ ID NO: 89; and (b) a light chain variable region comprising the amino acid sequence of SEQ ID NO: 95. Nucleotide sequences encoding the heavy chain variable region and the light chain variable region are provided in FIGURES 4A-B and 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 89; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 95.

### Clone 83-3

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence GFSFNLRH (SEQ ID NO: 120).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence HGGSSS (SEQ ID NO: 123).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence EDDIMMAGEFGL (SEQ ID NO: 126).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence GFSFNLRH (SEQ ID NO: 120); and/or (b) a VH CDR2 comprising, consisting of, or consisting essentially of the amino acid sequence HGGSSS (SEQ ID NO: 123); and/or (c) a VH CDR3 comprising, consisting of, or consisting essentially of the amino acid sequence EDDIMMAGEFGL (SEQ ID NO: 126).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 90.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 90.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASEDIESYLV (SEQ ID NO: 113).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence QASNLAS (SEQ ID NO: 115).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence QSYIEGGVDVV (SEQ ID NO: 118).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASEDIESYLV (SEQ ID NO: 113); and/or (b) a VL CDR2 comprising, consisting of, or consisting essentially of the amino acid sequence QASNLAS (SEQ ID NO: 115); and/or (c) a VL CDR3 comprising, consisting of, or consisting essentially of the amino acid sequence QSYIEGGVDVV (SEQ ID NO: 118).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 96.

In various embodiments, the light chain variable region is 80%, 85%, 90%, 91%, 92%, 93%, 94%, 95%, 96%, 97%, 98% or 99% identical to the light chain variable region sequence of SEQ ID NO: 96.

In some embodiments, an antigen binding molecule comprises (a) a VH comprising the amino acid sequence of SEQ ID NO: 90; and (b) a VL comprising the amino acid sequence of SEQ ID NO: 96. In some embodiments, an antigen binding molecule comprises: (a) a VH CDR1 region comprising the amino acid sequence of SEQ ID NO: 120; (b) a VH CDR2 region comprising the amino acid sequence of SEQ ID NO: 123; (c) a VH CDR3 region comprising the amino acid sequence of SEQ ID NO: 126; (d) a VL CDR1 region comprising the amino acid sequence of SEQ ID NO: 113; (e) a VL CDR2 region comprising the amino acid sequence of SEQ ID NO: 115; and (f) a VL CDR3 region comprising the amino acid sequence of SEQ ID NO: 118.

In some embodiments, the antibody or antigen binding molecule comprises: (a) a heavy chain variable region comprising the amino acid sequence of SEQ ID NO: 90; and (b) a light chain variable region comprising the amino acid sequence of SEQ ID NO: 96. Nucleotide sequences encoding the heavy chain variable region and the light chain variable region are provided in FIGURES 4A-B and 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 90; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 96.

### Clone 89-10

In some embodiments, an antigen binding molecule or antibody that specifically binds to a BCMA binding molecule, molecules comprising this sequence and cells presenting such molecules, comprises a VH CDR1 comprising, consisting of, or consisting essentially of the amino acid sequence GFSFSTGY (SEQ ID NO: 121).

In some embodiments, an antigen binding molecule or antibody that specifically binds to a BCMA binding molecule, molecules comprising this sequence and cells presenting such molecules, comprises a VH CDR2 comprising, consisting of, or consisting essentially of the amino acid sequence DTGSG (SEQ ID NO: 124).

In some embodiments, an antigen binding molecule or antibody that specifically binds to a BCMA binding molecule, molecules comprising this sequence and cells presenting such molecules, comprises a VH CDR3 comprising, consisting of, or consisting essentially of the amino acid sequence TALVMTDFYFNL (SEQ ID NO: 127).

In some embodiments, an antigen binding molecule or antibody that specifically binds to a BCMA binding molecule, molecules comprising this sequence and cells presenting such molecules, comprises a heavy chain VH comprising: (a) a VH CDR1 comprising, consisting of, or consisting essentially of the amino acid sequence GFSFSTGY (SEQ ID NO: 121); and/or (b) a VH CDR2 comprising, consisting of, or consisting essentially of the amino acid sequence DTGSG (SEQ ID NO: 124); and/or (c) a VH CDR3 comprising, consisting of, or consisting essentially of the amino acid sequence TALVMTDFYFNL (SEQ ID NO: 127).

In some embodiments, the antigen binding molecule or antibody that specifically binds to a BCMA binding molecule, molecules comprising this sequence and cells presenting this sequence, comprises a heavy chain variable region sequence comprising an amino acid sequence of SEQ ID NO: 91.

In various embodiments, the heavy chain variable region is 80%, 85%, 90%, 91%, 92%, 93%, 94%, 95%, 96%, 97%, 98% or 99% identical to the heavy chain variable region sequence of SEQ ID NO: 91.

In some embodiments, an antigen binding molecule or antibody that specifically binds to a BCMA binding molecule, molecules comprising this sequence and cells presenting such molecules, comprises a VL CDR1 comprising, consisting of, or consisting essentially of the amino acid sequence QASQWISNELS (SEQ ID NO: 114).

In some embodiments, an antigen binding molecule or antibody that specifically binds to a BCMA binding molecule, molecules comprising this sequence and cells presenting such molecules, comprises a VL CDR2 comprising, consisting of, or consisting essentially of the amino acid sequence LASTLAS (SEQ ID NO: 116).

In some embodiments, an antigen binding molecule or antibody that specifically binds to a BCMA binding molecule, molecules comprising this sequence and cells presenting such molecules, comprises a VL CDR3 comprising, consisting of, or consisting essentially of the amino acid sequence QQGYNIGHLDNA (SEQ ID NO: 119).

In some embodiments, an antigen binding molecule or antibody that specifically binds to a BCMA binding molecule, molecules comprising this sequence and cells presenting such molecules, comprises a light chain VL comprising: (a) a VL CDR1 comprising, consisting of, or consisting essentially of the amino acid sequence QASQWISNELS (SEQ ID NO: 114); and/or (b) a VL CDR2 comprising, consisting of, or consisting essentially of the amino acid sequence LASTLAS (SEQ ID NO: 116); and/or (c) a VL CDR3 comprising, consisting of, or consisting essentially of the amino acid sequence QQGYNIGHLDNA (SEQ ID NO: 119).

In some embodiments, the antigen binding molecule or antibody that specifically binds to a BCMA binding molecule, molecules comprising this sequence and cells presenting this sequence, comprises a light chain variable region sequence comprising an amino acid sequence of SEQ ID NO: 97.

In various embodiments, the light chain variable region is 80%, 85%, 90%, 91%, 92%, 93%, 94%, 95%, 96%, 97%, 98% or 99% identical to the light chain variable region sequence of SEQ ID NO: 97.

In some embodiments, an antigen binding molecule comprises (a) a VH comprising the amino acid sequence of SEQ ID NO: 91; and (b) a VL comprising the amino acid sequence of SEQ ID NO: 97. In some embodiments, an antigen binding molecule comprises: (a) a VH CDR1 region comprising the amino acid sequence of SEQ ID NO: 121; (b) a VH CDR2 region comprising the amino acid sequence of SEQ ID NO: 124; (c) a VH CDR3 region comprising the amino acid sequence of SEQ ID NO: 127; (d) a VL CDR1 region comprising the amino acid sequence of SEQ ID NO: 114; (e) a VL CDR2 region comprising the amino acid sequence of SEQ ID NO: 116; and (f) a VL CDR3 region comprising the amino acid sequence of SEQ ID NO: 119.

In some embodiments, the antibody or antigen binding molecule comprises: (a) a heavy chain variable region comprising the amino acid sequence of SEQ ID NO: 91; and (b) a light chain variable region comprising the amino acid sequence of SEQ ID NO: 97. Nucleotide sequences encoding the heavy chain variable region and the light chain variable region are provided in FIGURES 4A-B and 6A-B, respectively.

In some embodiments, the antibody or antigen binding molecule comprises: (a) a heavy chain variable region comprising an amino acid sequence that is 80%, 85%, 90%, 91%, 92%, 93%, 94%, 95%, 96%, 97%, 98% or 99% identical to the amino acid sequence of SEQ ID NO: 91; and (b) a light chain variable region comprising an amino acid sequence that is 80%, 85%, 90%, 91%, 92%, 93%, 94%, 95%, 96%, 97%, 98% or 99% identical to the amino acid sequence of SEQ ID NO: 97.

### III Polynucleotides Encoding Antibodies and other antigen binding molecules

The present invention is also directed to polynucleotides encoding antibodies and other antigen binding molecules that specifically bind to a BCMA binding molecule, molecules comprising this sequence and cells presenting this sequence.

In some embodiments, a polynucleotide of the present invention encodes an antigen binding molecule, wherein the antigen binding molecule comprises a heavy chain variable region amino acid sequence that is at least about 75%, at least about 85%, at least about 85%, at least about 90%, at least about 95%, at least about 96%, at least about 97%, at least about 98%, at least about 99%, or 100% identical to a heavy chain variable region amino acid sequence selected from the group consisting of SEQ ID NOs: 11-17, 89- 91.

In some embodiments, a polynucleotide of the present invention encodes antigen binding molecule, wherein the antigen binding molecule comprises a light chain variable amino acid sequence that is at least about 75%, at least about 85%, at least about 85%, at least about 90%, at least about 95%, at least about 96%, at least about 97%, at least about 98%, at least about 99%, or 100% identical to a light chain variable region amino acid sequence selected from the group consisting of SEQ ID NOs: 25-31, and 95-97.

In some embodiments, the polynucleotide comprises a heavy chain variable region coding sequence selected from the group consisting of SEQ ID NO: 4-10. In embodiments, the polynucleotide comprises a light chain coding sequence selected from the group consisting of SEQ ID NO: 18-24, and 92-94.

As will be appreciated by those of skill in the art, variations of the disclosed polynucleotide sequences are possible due to the degeneracy of the genetic code. Such variants of the disclose polynucleotide sequences thus form an aspect of the instant disclosure.

### IV. Vectors, Cells, and Pharmaceutical Compositions

In some aspects, provided herein are vectors comprising a polynucleotide disclosed herein. In some embodiments, the present invention is directed to a vector or a set of vectors comprising a polynucleotide(s) encoding an amino acid sequence of an antibody or antigen binding molecule that specifically binds to a BCMA binding molecule and fragments thereof, molecules comprising this sequence and cells presenting this sequence, as described herein.

Any vector known in the art may be suitable for expressing the antibodies and other antigen binding molecules of the present invention. In some embodiments, the vector is a viral vector. In some embodiments, the vector is a retroviral vector, a DNA vector, a murine leukemia virus vector, an SFG vector, a plasmid, a RNA vector, an adenoviral vector, a baculoviral vector, an Epstein Barr viral vector, a papovaviral vector, a vaccinia viral vector, a herpes simplex viral vector, an adenovirus associated vector (AAV), a lentiviral vector, or any combination thereof.

In other aspects, provided herein are cells comprising a polynucleotide or a vector of the present invention. In some embodiments, the present invention is directed to cells, *in vitro* cells, comprising a polynucleotide encoding an antigen binding molecule, as described herein. In some embodiments, the present invention is directed to cells, *e.g.*, *in vitro* cells, comprising a polynucleotide encoding an antibody or an antigen binding molecule thereof that specifically binds to a BCMA binding molecule, molecules comprising this sequence and cells presenting this sequence, as disclosed herein.

Any cell may be used as a host cell for the polynucleotides and vectors encoding all or a fragment of the antibodies and other antigen binding molecules of the present invention. In some embodiments, a host cell may be a prokaryotic cell, fungal cell, yeast cell, or higher eukaryotic cells such as a mammalian cell. Suitable prokaryotic cells include, without limitation, eubacteria, such as Gram-negative or Gram-positive organisms, for example, *Enterobactehaceae* such as *Escherichia, e.g., E. coli; Enterobacter; Erwinia; Klebsiella; Proteus*; *Salmonella*, e.g., *Salmonella typhimurium*; *Serratia*, e.g., *Serratia marcescans*, and *Shigella*; *Bacilli* such as *B. subtilis* and *B. licheniformis*; *Pseudomonas* such as *P. aeruginosa*; and *Streptomyces.* In some embodiments, a host cell is a human cell. In some embodiments, a host cell is a CHO cell and in other embodiments a host cell is a sP2/0 or other murine cell. A host cell of the present invention may be obtained through any source known in the art.

Other aspects of the present invention are directed to compositions comprising a polynucleotide described herein, a vector described herein, an antibody and/or an antigen binding molecule described herein, or an *in vitro* cell described herein. In some embodiments, the composition comprises a pharmaceutically acceptable carrier, diluent, solubilizer, emulsifier, preservative and/or adjuvant. In some embodiments, the composition comprises an excipient. In some embodiments, the composition comprises a polynucleotide encoding an antibody or antigen binding molecule that specifically binds to that specifically binds to a BCMA binding molecule and fragments thereof, molecules comprising this sequence and cells presenting this sequence. In another embodiment, the composition comprises an antigen binding molecule encoded by a polynucleotide of the present invention, wherein the antigen binding molecule specifically binds to a BCMA binding molecule, molecules comprising this sequence and cells presenting this sequence, as disclosed herein. In another embodiment, the composition comprises an *in vitro* cell comprising a polynucleotide encoding an antibody or an antigen binding molecule thereof encoded by a polynucleotide of the present invention.

In some embodiments, the composition comprises one antibody or antigen binding molecule that specifically binds to a BCMA binding molecule and fragments thereof, molecules comprising this sequence and cells presenting this sequence, as disclosed herein. In some embodiments, the composition comprises more than one antibody or antigen binding molecule that specifically binds to a BCMA binding molecule, molecules comprising this sequence and cells presenting this sequence, as disclosed herein, wherein the antibodies or antigen binding molecules bind more than one epitope. In some embodiments, the antibodies or antigen binding molecules will not compete with one another for binding to that epitope. In some embodiments, two or more of the antibodies or antigen binding molecules provided herein are combined together in a pharmaceutical composition. Preferably such a composition will be suitable for administration to a subject, including a human.

### V. Exemplary Methods

The following section describes various exemplary methods of using the disclosed antigen binding molecules herein. Any antigen binding molecule disclosed herein may be employed in the disclosed methods.

In various embodiments of the disclosed methods, the antigen binding molecule is selected from the group consisting of an antibody, an scFv, a Fab, a Fab', a Fv, a F(ab')₂, a dAb, a human antibody, a humanized antibody, a chimeric antibody, a monoclonal antibody, a polyclonal antibody, a recombinant antibody, an IgE antibody, an IgD antibody, an IgM antibody, an IgG1 antibody, an IgG1 antibody having at least one mutation in the hinge region, an IgG2 antibody an IgG2 antibody having at least one mutation in the hinge region, an IgG3 antibody, an IgG1 antibody having at least one mutation in the hinge region, an IgG4 antibody, an IgG4 antibody having at least one mutation in the hinge region, an antibody comprising at least one non-naturally occurring amino acid, and any combination thereof.

In some of the disclosed methods T cells may be employed. Such T cells may come from any source known in the art. For example, T cells may be differentiated *in vitro* from a hematopoietic stem cell population, or T cells may be obtained from a subject. T cells may be obtained from, e.g., peripheral blood mononuclear cells (PBMCs), bone marrow, lymph node tissue, cord blood, thymus tissue, tissue from a site of infection, ascites, pleural effusion, spleen tissue, and tumors. In addition, the T cells may be derived from one or more T cell lines available in the art. T cells may also be obtained from a unit of blood collected from a subject using any number of techniques known to the skilled artisan, such as FICOLL^{™} separation and/or apheresis. Additional methods of isolating T cells for a T cell therapy are disclosed in U.S. Patent Publication No. 2013/0287748, which is herein incorporated by references in its entirety.

In various embodiments of the disclosed methods, an antigen binding molecule specifically binds to a BCMA binding molecule, molecules comprising this sequence and cells presenting this sequence, as disclosed herein. In further embodiments of the disclosed methods, the antigen binding molecule comprises one or more of (a) a light chain CDR1, (b) a light chain CDR2, (c) a light chain CDR3, (d) a heavy chain CDR1, (e) a heavy chain CDR2, and (f) a heavy chain CDR3. In some embodiments of the disclosed methods, an antigen binding molecule comprises a heavy chain CDR3 comprising one of SEQ ID NOs: 59-64, 110-112, 125-127, or a light chain CDR3 comprising one of SEQ ID Nos: 54-58, 102-103, and 117-119, or both the heavy and light chain CDR3s. In some embodiments, the antigen binding molecule comprises a heavy chain CDR1 comprising an amino acid sequence comprising one of SEQ ID NOs: 38-43, or a heavy chain CDR2 comprising the amino acid sequence of one of SEQ ID NOs: 48-53, or a light chain CDR1 comprising the amino acid sequence of one of SEQ ID NOs: 32-37, 98, 99, 113, and 114 or a light chain CDR2 comprising the amino acid sequence of one of SEQ ID NOs: 44-47, 100, 101, 115, and 116. Referring to the Figures, in various embodiments of the disclosed methods, the antigen binding molecule comprises a heavy chain CDR1, a heavy chain CDR2, a heavy chain CDR3, a light chain CDR1, a light chain CDR2, and a light chain CDR3, each CDR comprising an amino acid sequence shown in FIGURES 4A-11.

In various embodiments of the disclosed methods, an antigen binding molecule comprises a heavy chain (HC), and the HC comprises a heavy chain variable region (VH) sequence comprising one of SEQ ID NOs: 11-17, 89- 91. Referring to the figures, in various embodiments of the disclosed methods the heavy chain comprises a heavy chain CDR1, a heavy chain CDR2, and a heavy chain CDR3, each CDR comprising an amino acid sequence shown in FIGURES 4A-11. Moreover, in embodiments of the disclosed methods, an antigen binding molecule may be employed which comprises a VH amino acid sequence that is at least about 70%, at least about 75%, at least about 80%, at least about 85%, at least about 90%, at least about 95%, at least about 96%, at least about 97%, at least about 98%, at least about 99%, or about 100% identical to a VH of an antigen binding molecule of claim disclosed herein *(e.g.,* an antigen binding molecules comprising a variable region (VH) sequence comprising one of SEQ ID NOs: 11-17, 89- 91).

In various embodiments of the disclosed methods, an antigen binding molecule comprises a light chain (LC), and the LC may comprise a heavy chain variable region (VL) sequence comprising one of SEQ ID NOs: 25-31, and 95-97. Referring to the figures, in various embodiments of the disclosed methods the light chain comprises a light chain CDR1, a light chain CDR2, and a light chain CDR3, each CDR comprising an amino acid sequence shown in FIGURES 4A-11. Moreover, in embodiments of the disclosed methods, an antigen binding molecule may be employed which comprises a VL amino acid sequence that is at least about 70%, at least about 75%, at least about 80%, at least about 85%, at least about 90%, at least about 95%, at least about 96%, at least about 97%, at least about 98%, at least about 99%, or about 100% identical to a VL of an antigen binding molecule of claim disclosed herein (e.g., an antigen binding molecules comprising a variable region (VL) sequence comprising SEQ ID NO: 25-31, and 95-97).

In view of the above description of antigen binding molecules that may be employed in the disclosed methods, representative methods will now be discussed in more detail.

### Va. Method of Administering a Dose of a Medicament to a Subject

In one aspect, a method of administering a dose of a medicament to a subject, the dose comprising a preselected number of cells presenting a therapeutic molecule comprising a BCMA binding molecule, is provided.

In specific embodiments, the dose comprises 0.5 ×10⁶ cells per kilogram of the subject, 1.0×10⁶ cells per kilogram of the subject, 2.0×10⁶ cells per kilogram of the subject, 3.0×10⁶ cells per kilogram of the subject, 4.0×10⁶ cells per kilogram of the subject, or 5.0×10⁶ cells per kilogram of the subject, although the method may be employed using any dose. 1.0×10⁶ cells per kilogram of the subject is a preferred dose.

Consistent with the definition provided herein, in various embodiments, a subject is a human or non-human subject. When the subject is a human, the subject may be, *e.g.*, any human who is being treated for an abnormal physiological condition, such as cancer or has been formally diagnosed with a disorder, those without formally recognized disorders, those receiving medical attention, those at risk of developing the disorders, those being studied for the presence or absence of a disorder, etc.

Initially, a sample of known volume comprising a population comprising a known number of cells, which cells are known or suspected to be presenting a molecule comprising a BCMA binding molecule, is provided. In the disclosed method, the number of cells may be determined using any known method. In preferred embodiments, the cells are counted using an automated apparatus, such as a cell sorter (*e.g*., a FACS), however traditional non-automated cell counting methods may also be employed.

The cells of the method may comprise any type of cell, with immune cells (*e.g.,* B lymphocytes, monocytes, dendritic cells, Langerhans cells, keratinocytes, endothelial cells, astrocytes, fibroblasts, and oligodendrocytes). T cells (including T cytotoxic, T helper and Treg cells) are especially preferred. In specific embodiments, the cells are T cells, which may be obtained as described herein and by methods known in the art. Any type of cell may be employed in the method, and the cell may be a human or non-human cell (including both prokaryotic and eukaryotic cells). Exemplary cells include, but are not limited to immune cells such as T cells, tumor infiltrating lymphocytes (TILs), NK cells, TCR-expressing cells, dendritic cells, and NK-T cells. The T cells may be autologous, allogeneic, or heterologous. In additional embodiments, the cells are T cells presenting a CAR. The T cells may be CD4+ T cells or CD8+ T cells. When a T cell is employed in the disclosed methods, the T cell may be an in vivo T cell or an in vitro T cell. Moreover, the cells may be disposed in, or isolated from, any environment capable of maintaining the cells in a viable form, such as blood, tissue or any other sample obtained from a subject, cell culture media, tissue grown *ex vivo,* etc. Gradient purification, cell culture selection and/or cell sorting may also be employed in obtaining T cells.

The therapeutic molecule expressed by the cell may comprise any molecule known or suspected to provide a therapeutic benefit to a subject to which is it administered. Thus, a therapeutic molecule may be a peptide or polypeptide of any structure or design. Preferably the therapeutic molecule component is expressed or disposed, at least in part, extracellularly, *i.e.*, to a degree that it may be recognized by an extracellular interaction partner such as the antigen binding molecules of the instant disclosure.

In specific embodiments, the therapeutic molecule is a CAR. When the therapeutic molecule is a CAR it may comprise a molecule, or fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CD1 ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof.

Continuing, an aliquot of the sample comprising a population of cells presenting a molecule comprising a BCMA binding molecule is provided. The aliquot may be obtained using any convenient means, such as by a cell sorter, by a simply pipetting of material out of the sample, etc.

Additionally, an antigen binding molecule that specifically binds a BCMA binding molecule further comprising a detectable label is provided. The antigen binding molecule is preferably an antigen binding molecule disclosed herein, *e.g*., in the figures, sequence listing or the instant disclosure. Any detectable label may be employed in the method, as described herein, and suitable labels may be selected using a desired set of criteria. Examples of types of detectable labels include fluorescent labels (*e.g*., an Atto dye, an Alexafluor dye, quantum dots, Hydroxycoumarin, Aminocoumarin, Methoxycoumarin, Cascade Blue, Pacific Blue, Pacific Orange, Lucifer yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhodamine B, Texas Red, Allophycocyanin (APC), APC-Cy7 conjugates, Indo-1, Fluo-3, Fluo-4, DCFH, DHR, SNARF, GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midoriishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP,Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoerythrin (BPE), mCherry, HcRed1, Katusha, P3, Peridinin Chlorophyll (PerCP), mKate (TagFP635), TurboFP635, mPlum, and mRaspberry). Suitable optical dyes, including fluoro-phores, are described in Johnson, Molecular Probes Handbook: A Guide to Fluorescent Probes and Labeling Techniques, 11th Edition, Life Technologies, (2010), hereby expressly incorporated by reference, radiolabels (*e.g.*, isotope markers such as ³H, ¹¹C, ¹⁴C, ¹⁵N, ¹⁸F, ³⁵S, ⁶⁴CU, ⁹⁰Y, ⁹⁹Tc, ¹¹¹In, ¹²⁴I, ¹²⁵I, ¹³¹I), photochromic compounds, a Halo-tag, Atto dyes, Tracy dyes, proteinaceous fluorescent labels (*e.g*., proteinaceous fluorescent labels also include, but are not limited to, green fluorescent protein, including a Renilla, Ptilosarcus, or Aequorea species of GFP (Chalfie et al., (1994) Science 263:802-805), EGFP (Clon-tech Labs., Inc., Genbank Accession Number U55762), blue fluorescent protein (BFP, Quantum Biotechnologies, Inc; Stauber, (1998) Biotechniques 24:462-471; Heim et al., (1996) Curr. Biol. 6: 178-182), enhanced yellow fluorescent protein (Clontech Labs., Inc.), luciferase (Ichiki et al., (1993) J. Immunol. 150:5408-5417), magnetic labels (*e.g*., DYNABEADS), etc. Strategies for the labeling of proteins are known in the art and may be employed in the disclosed method.

The label may be associated with the antigen binding molecule at any position in the molecule, although it is preferable to associate the label with the molecule at a position (or positions, if multiple labels are employed) at a point such that the binding properties of the molecule are not modified (unless such modified binding activity is desired). Any antigen binding molecule that specifically binds a BCMA binding molecule (or fragment thereof) may be employed. Multiple examples of suitable antigen binding molecules are provided herein, *e.g*., those having one or more of the CDRs shown in FIGURES 4A-11.

The antigen binding molecule may be disposed on any surface, or no surface at all. For example, the antigen binding molecule may be present in a buffer and the buffer-antigen binding molecule may be contacted with the sample. Alternatively, the antigen binding molecule may be associated with a surface. Suitable surfaces include agarose beads, magnetic beads such as DYNABEADS, or a plastic, glass or ceramic plate such as a welled plate, a bag such as a cell culture bag, etc. The surface may itself be disposed in another structure, such as a column.

Continuing, the aliquot of the sample is contacted with the antigen binding molecule under conditions that permit the formation of a binding complex comprising a cell present in the sample and the antigen binding molecule. Thus, the result of this step of the method is the formation of a binding complex in which the antigen binding molecule, with which a detectable label is associated, is bound to the cell expressing the therapeutic molecule, which comprises a BCMA binding molecule. Thus, the binding complex itself is detectable. Conditions that permit the formation of a binding complex will be dependent on a variety of factors, however generally aqueous buffers at physiological pH and ionic strength, such as in phosphate-buffered saline (PBS), will favor formation of binding complexes and are preferred in the disclosed method.

The fraction of cells present in a binding complex of in the aliquot is then determined. This calculation may be performed by comparing the number of cells bearing the detectable label to those that do not, and may be represented as percentage. The number of cells in binding complexes may be determined. The specific method employed to determine the number of cells present in a binding complex will be dependent on the nature of the label selected. For example, FACS may be employed when a fluorescent label is selected; when an isotope label is selected mass spectrometry, NMR or other technique may be employed; magnetic-based cell sorting may be employed when a magnetic label is chosen; microscopy may also be employed. The number of cells in the sample is known *ab initio* and thus the fraction of cells present in a binding complex may be easily determined.

Continuing, the concentration of cells in the initial sample expressing a molecule comprising a BCMA binding molecule is determined; the determination is based on the fraction of cells determined to be present in the binding complex, and thus expressing the therapeutic protein bearing a detectable label.

The fraction of cells presenting the therapeutic protein is known, and the volume of the aliquot is known; thus a simple comparison of the number of cells in the sample from which the aliquot was taken that are expressing the therapeutic molecule to the volume of the larger sample provides the fraction of the cells in the sample bearing the therapeutic molecule on a therapeutic molecule/volume basis *(i.e.,* the concentration of cells bearing the therapeutic molecule in the larger sample).

The volume of the sample that comprises the selected number of cells is then determined, by extrapolation based on the concentration of cells bearing therapeutic molecule present in the sample.

Finally, the volume of sample comprising the desired number of cells is administered to the subject. The administration may comprise an aspect of a therapeutic regimen based on the therapeutic molecule present in the sample and expressed by the cells in the sample.

Although the administration may be performed one time or more than one time, an advantage of the method is that by administering a dose comprising the preselected number of cells, which number of cells will be determined based on a known or expected efficacy, unnecessary administration of cells presenting the therapeutic molecule is avoided; *i.e*., the subject receives the correct number of cells to provide a desired therapeutic benefit and is not overdosed with cells.

### Vb. Method of Determining a Number of Cells Presenting a Molecule of Interest

There are situations in which it may be desirable to determine the number of cells present in a sample. For example, it may be desirable to determine the number of immune cells present a sample obtained from a subject. Or it may be desirable to determine the number of cells transfected and expressing a construct, which may be used as a measure of the level of efficiency of the transfection. The disclosed method may be employed in these and other applications in which it is desirable to determine the number of cells present in a sample.

Thus, a method of determining a number of cells presenting a molecule in a sample wherein the molecule comprising a BCMA binding molecule is provided.

In on embodiment, a sample comprising cells known or suspected to be expressing a molecule comprising the amino acid sequence of a BCMA binding molecule is provided.

The cell may be of any type, and may be human or non-human (*e.g*., mouse, rate, rabbit, hamster, etc). In preferred embodiment, the cell is an immune cell. An immune cell of the method may be any type of immune cell (*e.g*., B lymphocytes, monocytes, dendritic cells, Langerhans cells, keratinocytes, endothelial cells, astrocytes, fibroblasts, and oligodendrocytes). T cells (including T cytotoxic, T helper and Treg cells) are especially preferred. In specific embodiments, the cells are T cells, which may be obtained as described herein and by methods known in the art. Any type of immune cell may be employed in this embodiment of the disclosed method, and the cell may be a human or non-human cell (including both prokaryotic and eukaryotic cells). Exemplary cells include, but are not limited to immune cells such as T cells, tumor infiltrating lymphocytes (TILs), NK cells, TCR-expressing cells, dendritic cells, and NK-T cells. The T cells may be autologous, allogeneic, or heterologous. In additional embodiments, the cells are T cells presenting a CAR. The T cells may be CD4+ T cells or CD8+ T cells. When a T cell is employed in the disclosed methods, the T cell may be an *in vivo* T cell or an *in vitro* T cell. Moreover, the cells may be disposed in, or isolated from, any environment capable of maintaining the cells in a viable form, such as blood, tissue or any other sample obtained from a subject, cell culture media, tissue grown *ex vivo,* a suitable buffer, etc.

In specific embodiments, the molecule comprising a BCMA binding molecule is a CAR. When the molecule is a CAR it may comprise a molecule, or fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CD1 la, LFA-1, ITGAM, CD1 lb, ITGAX, CD1 lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof.

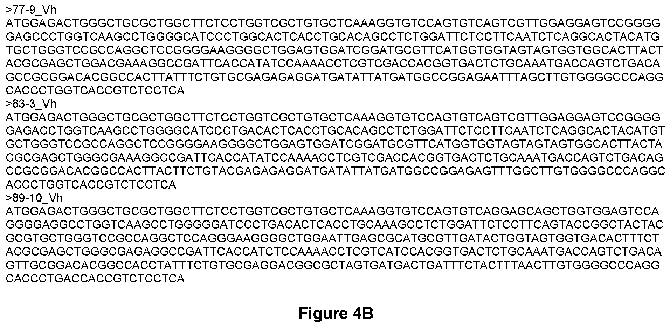

The sample is then contacted with an antigen binding molecule that specifically binds a BCMA binding molecule and comprises a detectable label, under conditions that permit the formation of a binding complex comprising a cell present in the sample and the antigen binding molecule. The antigen binding molecule is preferably an antigen binding molecule (or fragment thereof) disclosed herein, e.g., in the figures, sequence listing or the instant section of the disclosure. Any antigen binding molecule that specifically binds a BCMA binding molecule may be employed in the disclosed method. Multiple examples of suitable antigen binding molecules are provided herein, *e.g*., those having one or more of the CDRs shown in FIGURE 4-11.

Any detectable label may be employed in the method, as described herein, and suitable labels may be selected using a desired set of criteria. Examples of types of detectable labels include fluorescent labels (*e.g.*, fluorescein, rhodamine, tetramethylrhodamine, eosin, erythrosin, coumarin, methyl-coumarins, pyrene, Malachite green, stilbene, Lucifer Yellow, Cascade Blue, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 647, Alexa Fluor 660, Alexa Fluor 680), Cascade Blue, Cas-cade Yellow and R-phycoerythrin (PE) (Molecular Probes), FITC, Rhodamine, and Texas Red (Pierce), Cy5, Cy5.5, Cy7 (Amersham Life Science). Suitable optical dyes, including fluoro-phores, are described in Johnson, Molecular Probes Handbook:A Guide to Fluorescent Probes and Labeling Techniques, 11th Edition, Life Technologies, (2010), hereby expressly incorporated by reference, radiolabels (*e.g*., isotope markers such as ³H, ¹¹C, ¹⁴C, ¹⁵N, ¹⁸F, ³⁵S, ⁶⁴CU, ⁹⁰Y, ⁹⁹Tc, ¹¹¹In, ¹²⁴I, ¹²⁵I, ¹³¹I), photochromic compounds, a Halo-tag, Atto dyes, Tracy dyes, proteinaceous fluorescent labels (*e.g*., proteinaceous fluorescent labels also include, but are not limited to, green fluorescent protein, including a Renilla, Ptilosarcus, or Aequorea species of GFP (Chalfie et al., (1994) Science 263:802-805), EGFP (Clon-tech Labs., Inc., Genbank Accession Number U55762), blue fluorescent protein (BFP, Quantum Biotechnologies, Inc; Stauber, (1998) Biotechniques 24:462-471; Heim et al., (1996) Curr. Biol. 6: 178-182), enhanced yellow fluorescent protein (Clontech Labs., Inc.), luciferase (Ichiki et al., (1993) J. Immunol. 150:5408-5417), magnetic labels (e.g., DYNABEADS), etc. Strategies for the labeling of proteins are well known in the art and may be employed in the disclosed method. *See, e.g.,* Obermaier et al., (2015) Methods Mol Biol 1295:153-65; Strack (2016) Nature Methods 13:33; Site-Specific Protein Labeling: Methods and Protocols, (Gautier and Hinner, eds.) 2015, Springer.

The label may be associated with the antigen binding molecule at any position in the molecule, although it is preferable to associate the label with the molecule at a position (or positions, if multiple labels are employed) at a point such that the binding properties of the molecule are not modified (unless such modified binding activity is desired). Any antigen binding molecule that specifically binds a BCMA binding molecule (or fragment thereof) may be employed, such as those disclosed herein, *e.g*., those having one or more of the CDRs shown in FIGURES 4A-11.

The antigen binding molecule may be disposed on any surface, or no surface at all. For example, the antigen binding molecule may be present in a buffer and the buffer-antigen binding molecule may be contacted with the sample. Alternatively, the antigen binding molecule may be associated with a surface. Suitable surfaces include agarose beads, magnetic beads such as DYNABEADS, or a plastic, glass or ceramic plate such as a welled plate, a bag such as a cell culture bag, etc. The surface may itself be disposed in another structure, such as a column.

Conditions that permit the formation of a binding complex will be dependent on a variety of factors, however generally aqueous buffers at physiological pH and ionic strength, such as in phosphate-buffered saline (PBS), will favor formation of binding complexes and are preferred in the disclosed method.

Continuing, the number of cells present in a binding complex in the sample is determined. The specific method employed to determine the number of cells present in a binding complex will be dependent on the nature of the label selected. For example, FACS may be employed when a fluorescent label is selected; when an isotope label is selected mass spectrometry, NMR or other technique may be employed; magnetic-based cell sorting may be employed when a magnetic label is chosen; microscopy may also be employed. The output of these detection methods may be in the form of a number of cells or the output may be of a form that allows the calculation of the number of cells based on the output.

### Vc. Method of Isolating a Molecule

It is of tremendous value to have the ability to separate different populations of molecules, and particularly biologically-relevant molecules, from one another. Using the antigen binding molecules provided herein, such separation may be achieved and employed in a range of biotechnological, biopharmaceutical and therapeutic applications. Thus, in one aspect of the instant disclosure, a method of isolating a molecule comprising a BCMA binding molecule is provided.

In some embodiments, the method comprises providing a sample known or suspected to comprise a molecule comprising a BCMA binding molecule.

In specific embodiments, the molecule comprising a BCMA binding molecule is a CAR. When the molecule is a CAR it may comprise a molecule, or fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CD1 ld, ITGAE, CD103, ITGAL, CD1 la, LFA-1, ITGAM, CD1 lb, ITGAX, CD1 1c, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof.

An antigen binding molecule that specifically binds a BCMA binding molecule and optionally comprises a detectable label is provided. When it is decided to employ a detectable label, any detectable label may be employed in the method, as described herein, and suitable labels may be selected using a desired set of criteria. Examples of types of detectable labels include fluorescent labels (e.g., fluorescein, rhodamine, tetramethylrhodamine, eosin, erythrosin, coumarin, methyl-coumarins, pyrene, Malachite green, stilbene, Lucifer Yellow, Cascade Blue, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 647, Alexa Fluor 660, Alexa Fluor 680), Cascade Blue, Cas-cade Yellow and R-phycoerythrin (PE) (Molecular Probes), FITC, Rhodamine, and Texas Red (Pierce), Cy5, Cy5.5, Cy7 (Amersham Life Science)). Suitable optical dyes, including fluorophores, are described in Johnson, Molecular Probes Handbook:A Guide to Fluorescent Probes and Labeling Techniques, 11th Edition, Life Technologies, (2010), hereby expressly incorporated by reference, radiolabels (e.g., isotope markers such as ³H, ¹¹C, ¹⁴C, ¹⁵N, ¹⁸F, ³⁵S, ⁶⁴CU, ⁹⁰Y, ⁹⁹Tc, ¹¹¹In, ¹²⁴I, ¹²⁵I, ¹³³I). Photochromic compounds, a Halo-tag, Atto dyes, Tracy dyes, proteinaceous fluorescent labels (*e.g*., proteinaceous fluorescent labels also include, but are not limited to, green fluorescent protein, including a Renilla, Ptilosarcus, or Aequorea species of GFP (Chalfie et al., (1994) Science 263:802-805), EGFP (Clon-tech Labs., Inc., Genbank Accession Number U55762), blue fluorescent protein (BFP, Quantum Biotechnologies, Inc; Stauber, (1998) Biotechniques 24:462-471; Heim et al., (1996) Curr. Biol. 6: 178-182), enhanced yellow fluorescent protein (Clontech Labs., Inc.), luciferase (Ichiki et al., (1993) J. Immunol. 150:5408-5417), magnetic labels (e.g., DYNABEADS), etc may also be employed. Strategies for the labeling of proteins are well known in the art and may be employed in the disclosed method. *See, e.g.,* Obermaier et al., (2015) Methods Mol Biol 1295:153-65; Strack (2016) Nature Methods 13:33; Site-Specific Protein Labeling: Methods and Protocols, (Gautier and Hinner, eds.) 2015, Springer.

The label may be associated with the antigen binding molecule at any position in the molecule, although it is preferable to associate the label with the molecule at a position (or positions, if multiple labels are employed) at a point such that the binding properties of the molecule are not modified (unless such modified binding activity is desired). Any antigen binding molecule that specifically binds anti-BCMA scFv (or fragment thereof) may be employed, such as those disclosed herein, e.g., those having one or more of the CDRs shown in FIGURES 4A-11.

The antigen binding molecule may be disposed on any surface, or no surface at all. For example, the antigen binding molecule may be present in a buffer and the buffer-antigen binding molecule may be contacted with the sample. Alternatively, the antigen binding molecule may be associated with a surface. Suitable surfaces include agarose beads, magnetic beads such as DYNABEADS, or a plastic, glass or ceramic plate such as a welled plate, a bag such as a cell culture bag, etc. The surface may itself be disposed in another structure, such as a column.

Conditions that permit the formation of a binding complex will be dependent on a variety of factors, however generally aqueous buffers at physiological pH and ionic strength, such as in phosphate-buffered saline (PBS), will favor formation of binding complexes and are preferred in the disclosed method. Since the component parts of a binding complex may be disposed on surfaces as described herein, formed binding complexes may also be disposed on surfaces.

At this stage, no binding complexes may have formed, or a plurality of binding complexes comprising one or more antigen binding molecules bound to a molecule comprising a BCMA binding molecule (or one or more molecules comprising a BCMA binding molecule bound to an antigen binding molecule) may have formed. Unbound molecules comprising a BCMA binding molecule and/or unbound antigen binding molecules may also be present in the local environment of any formed binding complexes.

Any molecules not part of a binding complex are then separated from any formed binding complexes. The method of the removal will depend on the structure and/or local environment of the binding complexes. For example, if the antigen binding molecule is disposed on a bead, plate or bag the unbound components of the reaction mixture may be washed away using a solution that leaves formed binding complexes intact. If a binding complex is disposed on a bead, the bead itself may be situated in a column or other structure and the same approach may be used.

The solution used to induce the formation of binding complexes may be used, for example, as a wash solution to remove unbound components. Any suitable buffer or solution that does not disrupt formed binding complexes may also be used. Typically, buffers having high salt concentrations, non-physiological pH, containing chaotropes or denaturants, are preferably avoided when performing this step of the method.

A formed binding complex is then separated into (a) a molecule comprising a BCMA binding molecule, and (b) an antigen binding molecule. The separation may be achieved using standard methodologies known to those of skill in the art. For example, a solution of suitable pH and composition may be washed over the complexes. A solution that is commonly employed for this purpose is 0.1 M glycine HCl, pH 2.5-3.0, and this solution may be employed to achieve the separation. Other solutions that may be employed include 100 mM citric acid, pH 3.0, 50-100 mM triethylamine or triethanolamine, pH 11.5; 150 mM ammonium hydroxide, pH 10.5; 0.1 M glycine•NaOH, pH 10.0; 5 M lithium chloride, 3.5 M magnesium or potassium chloride, 3.0 M potassium chloride, 2.5 M sodium or potassium iodide, 0.2-3.0 M sodium thiocyanate, 0.1 M Tris-acetate with 2.0 M NaCl, pH 7.7; 2-6 M guanidine HCl, 2-8 M urea, 1.0 M ammonium thiocyanate, 1% sodium deoxycholate 1% SDS; and 10% dioxane 50% ethylene glycol, pH 8-11.5.

Following the separation, if the molecule comprising a BCMA binding molecule is of primary interest it may be collected; alternatively, if the antigen binding molecule is of primary interest it may be collected.

### Vd. Method of Determining the Presence or Absence of a Molecule

As disclosed herein, it may sometimes be desirable to isolate a molecule comprising a BCMA binding molecule, as provided herein. In other cases, simply knowing whether a molecule comprising a BCMA binding molecule provided herein is present or absent from a sample is enough information. For example, it may be beneficial to know that such a molecule is being expressed, regardless of the level of expression. In other cases, it may be desirable to know if a purification process or step designed to remove such a molecule has been effectively. Thus, the qualitative determination of the presence or absence of a molecule comprising a BCMA binding molecule of the instant disclosure may be useful in multiple applications. In view thereof, a method of determining the presence or absence of a molecule comprising a BCMA binding molecule in a sample is provided.

In some embodiments, the method comprises providing a sample known or suspected to comprise a molecule comprising a BCMA binding molecule.

In some embodiments, the molecule comprising a BCMA binding molecule is a CAR. When the molecule is a CAR it may comprise a molecule, or fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CD1 la, LFA-1, ITGAM, CD1 lb, ITGAX, CD1 lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof.

An antigen binding molecule comprising a detectable label that specifically binds a BCMA binding molecule is provided. Suitable labels may be selected using a desired set of criteria. Examples of types of detectable labels include fluorescent labels (e.g., fluorescein, rhodamine, tetramethylrhodamine, eosin, erythrosin, coumarin, methyl-coumarins, pyrene, Malachite green, stilbene, Lucifer Yellow, Cascade Blue, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 647, Alexa Fluor 660, Alexa Fluor 680), Cascade Blue, Cas-cade Yellow and R-phycoerythrin (PE) (Molecular Probes), FITC, Rhodamine, and Texas Red (Pierce), Cy5, Cy5.5, Cy7 (Amersham Life Science). Suitable optical dyes, including fluoro-phores, are described in Johnson, Molecular Probes Handbook:A Guide to Fluorescent Probes and Labeling Techniques, 11th Edition, Life Technologies, (2010), hereby expressly incorporated by reference, radiolabels (*e.g*., isotope markers such as ³H, ¹¹C, ¹⁴C, ¹⁵N, ¹⁸F, ³⁵S, ⁶⁴CU, ⁹⁰Y, ⁹⁹Tc, ¹¹¹In, ¹²⁴I, ¹²⁵I, ¹³¹I), photochromic compounds, a Halo-tag, Atto dyes, Tracy dyes, proteinaceous fluorescent labels (*e.g*., proteinaceous fluorescent labels also include, but are not limited to, green fluorescent protein, including a Renilla, Ptilosarcus, or Aequorea species of GFP (Chalfie et al., (1994) Science 263:802-805), EGFP (Clon-tech Labs., Inc., Genbank Accession Number U55762), blue fluorescent protein (BFP, Quantum Biotechnologies, Inc; Stauber, (1998) Biotechniques 24:462-471; Heim et al., (1996) Curr. Biol. 6: 178-182), enhanced yellow fluorescent protein (Clontech Labs., Inc.), luciferase (Ichiki et al., (1993) J. Immunol. 150:5408-5417), magnetic labels (e.g., DYNABEADS), etc. Strategies for the labeling of proteins are well known in the art and may be employed in the disclosed method. *See, e.g.,* Obermaier et al., (2015) Methods Mol Biol 1295: 153-65; Strack (2016) Nature Methods 13:33; Site-Specific Protein Labeling: Methods and Protocols, (Gautier and Hinner, eds.) 2015, Springer.

The label may be associated with the antigen binding molecule at any position in the molecule, although it is preferable to associate the label with the molecule at a position (or positions, if multiple labels are employed) at a point such that the binding properties of the molecule are not modified (unless such modified binding activity is desired). Any antigen binding molecule that specifically binds a BCMA binding molecule or fragment thereof) may be employed, such as those disclosed herein, e.g., those having one or more of the CDRs shown in FIGURES 4A-11.

Continuing, the sample is contacted with the antigen binding molecule under conditions that permit the formation of a binding complex comprising a molecule comprising a BCMA binding molecule(which may be presented on a cell) present in the sample and the antigen binding molecule. The antigen binding molecule may be disposed on any surface or no surface at all. For example, the antigen binding molecule may be present in a buffer and the buffer-antigen binding molecule may be contacted with the sample. Alternatively, the antigen binding molecule may be associated with a surface. Suitable surfaces include agarose beads, magnetic beads such as DYNABEADS, or a plastic, glass or ceramic plate such as a welled plate, a bag such as a cell culture bag, etc. The surface may itself be disposed in another structure, such as a column.

Conditions that permit the formation of a binding complex will be dependent on a variety of factors, however generally aqueous buffers at physiological pH and ionic strength, such as in phosphate-buffered saline (PBS), will favor formation of binding complexes and are preferred in the disclosed method. Since the component parts of a binding complex may be disposed on surfaces as described herein, formed binding complexes may also be disposed on surfaces.

At this stage, no binding complexes may have formed, or a plurality of binding complexes comprising one or more antigen binding molecules bound to a molecule comprising a BCMA binding molecule (or one or more molecules comprising a BCMA binding molecule bound to an antigen binding molecule) may have formed. Unbound molecules comprising a BCMA binding molecule and/or unbound antigen binding molecules may also be present in the local environment of any formed binding complexes.

Any molecules not part of a binding complex are then separated from any formed binding complexes. The method of the removal will depend on the structure and/or local environment of the binding complexes. For example, if the antigen binding molecule is disposed on a bead, plate or bag the unbound components of the reaction mixture may be washed away using a solution that leaves formed binding complexes intact. If a binding complex is disposed on a bead, the bead itself may be situated in a column or other structure and the same approach may be used.

The solution used to induce the formation of binding complexes may be used, for example, as a wash solution to remove unbound components. Any suitable buffer or solution that does not disrupt formed binding complexes may also be used. Typically, buffers having high salt concentrations, non-physiological pH, containing chaotropes or denaturants, should be avoided when performing this step of the method.

Lastly, the presence or absence of a binding complex--which will comprise a molecule comprising a BCMA binding molecule and an antigen binding molecule-is detected. The specific method employed to detect the presence or absence of a binding complex will be dependent on the nature of the label selected. For example, FACS may be employed when a fluorescent label is selected; when an isotope label is selected mass spectrometry, NMR or other technique may be employed; magnetic-based cell sorting may be employed when a magnetic label is chosen; microscopy may also be employed. The end result of the method is a qualitative assessment of the presence or absence of the antigen binding molecule comprising the detectable label, and thus, the presence or absence of its binding partner, the molecule comprising a BCMA binding molecule.

As is the case with all of the disclosed methods, the molecule comprising a BCMA binding molecule may be disposed in any environment. In preferred embodiments, the molecule comprising a BCMA binding molecule is expressed on the surface of a cell. In this embodiment, the cell may be of any type, and may be human or non-human (e.g., mouse, rate, rabbit, hamster, etc). In preferred embodiment, the cell is an immune cell. An immune cell of the method may be any type of immune cell (e.g., B lymphocytes, monocytes, dendritic cells, Langerhans cells, keratinocytes, endothelial cells, astrocytes, fibroblasts, and oligodendrocytes). T cells (including T cytotoxic, T helper and Treg cells) are especially preferred. In specific embodiments, the cells are T cells, which may be obtained as described herein and by methods known in the art. Any type of immune cell may be employed in this embodiment of the disclosed method, and the cell may be a human or non-human cell (including both prokaryotic and eukaryotic cells). Exemplary cells include, but are not limited to immune cells such as T cells, tumor infiltrating lymphocytes (TILs), NK cells, TCR-expressing cells, dendritic cells, and NK-T cells. The T cells may be autologous, allogeneic, or heterologous. In additional embodiments, the cells are T cells presenting a CAR. The T cells may be CD4+ T cells or CD8+ T cells. When a T cell is employed in the disclosed methods, the T cell may be an *in vivo* T cell or an *in vitro* T cell.

In additional embodiment, the cell may be disposed in, or isolated from, any environment capable of maintaining the cell in a viable form, such as blood, tissue or any other sample obtained from a subject, cell culture media, tissue grown *ex vivo,* a suitable buffer, etc.

### Ve. Method of Increasing the Concentration of a Molecule

Very often a molecule of interest is present in a sample in lower-than-desired levels. For example, when a cell is transfected with a foreign gene expression levels of the protein(s) encoded by the foreign gene are sometimes low. The same may be true for molecules secreted from a cell; such molecules are often present in low quantities (but may still be detected using the methods provided herein, if the molecule comprises a BCMA binding molecule. One solution to the problem of low expression levels is to increase the concentration of the molecule of interest, which may be free in solution, or expressed on the surface of a cell. The concentration of intracellularly-expressed molecules of interest may also be enhanced, however the cells must first be lysed to release the molecule. To address this problem, a method of increasing the concentration of cells presenting a molecule comprising a BCMA binding molecule is provided.

In some embodiments, the method comprises providing a sample comprising cells known or suspected to present a molecule comprising a BCMA binding molecule.

In specific embodiments, the molecule comprising the sequence a BCMA binding molecule is a CAR. When the molecule is a CAR it may comprise a molecule, or fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CD1 la, LFA-1, ITGAM, CD1 lb, ITGAX, CD1 lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof.

An antigen binding molecule that specifically binds a BCMA binding molecule, and optionally comprises a detectable label, is provided. When it is decided to employ a detectable label, any detectable label may be employed in the method, as described herein, and suitable labels may be selected using a desired set of criteria. Examples of types of detectable labels include fluorescent labels (*e.g*., fluorescein, rhodamine, tetramethylrhodamine, eosin, erythrosin, coumarin, methyl-coumarins, pyrene, Malachite green, stilbene, Lucifer Yellow, Cascade Blue, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 647, Alexa Fluor 660, Alexa Fluor 680), Cascade Blue, Cas-cade Yellow and R-phycoerythrin (PE) (Molecular Probes), FITC, Rhodamine, and Texas Red (Pierce), Cy5, Cy5.5, Cy7 (Amersham Life Science). Suitable optical dyes, including fluoro-phores, are described in Johnson, Molecular Probes Handbook:A Guide to Fluorescent Probes and Labeling Techniques, 11th Edition, Life Technologies, (2010), hereby expressly incorporated by reference, radiolabels (e.g., isotope markers such as ³H, ¹¹C, ¹⁴C, ¹⁵N, ¹⁸F, ³⁵S, ⁶⁴CU, ⁹⁰Y, ⁹⁹Tc, ¹¹¹In, ¹²⁴I, ¹²⁵I, ¹³¹I), photochromic compounds, a Halo-tag, Atto dyes, Tracy dyes, proteinaceous fluorescent labels (*e.g.*, proteinaceous fluorescent labels also include, but are not limited to, green fluorescent protein, including a Renilla, Ptilosarcus, or Aequorea species of GFP (Chalfie et al., (1994) Science 263:802-805), EGFP (Clon-tech Labs., Inc., Genbank Accession Number U55762), blue fluorescent protein (BFP, Quantum Biotechnologies, Inc; Stauber, (1998) Biotechniques 24:462-471; Heim et al., (1996) Curr. Biol. 6: 178-182), enhanced yellow fluorescent protein (Clontech Labs., Inc.), luciferase (Ichiki et al., (1993) J. Immunol. 150:5408-5417), magnetic labels (e.g., DYNABEADS), etc. Strategies for the labeling of proteins are well known in the art and may be employed in the disclosed method. *See, e.g.,* Obermaier et al., (2015) Methods Mol Biol 1295:153-65; Strack (2016) Nature Methods 13:33; Site-Specific Protein Labeling: Methods and Protocols, (Gautier and Hinner, eds.) 2015, Springer.

The label may be associated with the antigen binding molecule at any position in the molecule, although it is preferable to associate the label with the molecule at a position (or positions, if multiple labels are employed) at a point such that the binding properties of the molecule are not modified (unless such modified binding activity is desired). Any antigen binding molecule that specifically binds a BCMA binding molecule (or fragment thereof) may be employed, such as those disclosed herein, *e.g*., those having one or more of the CDRs shown in FIGURES 4A-11.

The antigen binding molecule may be disposed on any surface, or no surface at all. For example, the antigen binding molecule may be present in a buffer and the buffer-antigen binding molecule may be contacted with the sample. Alternatively, the antigen binding molecule may be associated with a surface. Suitable surfaces include agarose beads, magnetic beads such as DYNABEADS, or a plastic, glass or ceramic plate such as a welled plate, a bag such as a cell culture bag, etc. The surface may itself be disposed in another structure, such as a column.

A cell presenting a molecule comprising a BCMA binding molecule may be of any type, and may be human or non-human (*e.g*., mouse, rate, rabbit, hamster, etc). In preferred embodiment, the cell is an immune cell. An immune cell of the method may be any type of immune cell *(e.g.,* B lymphocytes, monocytes, dendritic cells, Langerhans cells, keratinocytes, endothelial cells, astrocytes, fibroblasts, and oligodendrocytes). T cells (including T cytotoxic, T helper and Treg cells) are especially preferred. In specific embodiments, the cells are T cells, which may be obtained as described herein and by methods known in the art. Any type of immune cell may be employed in this embodiment of the disclosed method, and the cell may be a human or non-human cell (including both prokaryotic and eukaryotic cells). Exemplary cells include, but are not limited to immune cells such as T cells, tumor infiltrating lymphocytes (TILs), NK cells, TCR-expressing cells, dendritic cells, and NK-T cells. The T cells may be autologous, allogeneic, or heterologous. In additional embodiments, the cells are T cells presenting a CAR. The T cells may be CD4+ T cells or CD8+ T cells. When a T cell is employed in the disclosed methods, the T cell may be an *in vivo* T cell or an *in vitro* T cell. Moreover, the cells may be disposed in, or isolated from, any environment capable of maintaining the cells in a viable form, such as blood, tissue or any other sample obtained from a subject, cell culture media, tissue grown *ex vivo,* a suitable buffer, etc.

The sample comprising cells is contacted with the antigen binding molecule, under conditions that permit the formation of a binding complex comprising a molecule comprising a BCMA binding molecule and the antigen binding molecule. Conditions that permit the formation of a binding complex will be dependent on a variety of factors, however generally aqueous buffers at physiological pH and ionic strength, such as in phosphate-buffered saline (PBS), will favor formation of binding complexes and are preferred in the disclosed method. Since the component parts of a binding complex may be disposed on surfaces as described herein, formed binding complexes may also be disposed on surfaces.

At this stage, no binding complexes may have formed, or a plurality of binding complexes comprising one or more antigen binding molecules bound to a molecule comprising a BCMA binding molecule (or one or more molecules comprising a BCMA binding molecule bound to an antigen binding molecule) may have formed. Unbound molecules comprising a BCMA binding molecule and/or unbound antigen binding molecules may also be present in the local environment of any formed binding complexes.

Any molecules or cells not part of a binding complex are then separated from any formed binding complexes. The method of the removal will depend on the structure and/or local environment of the binding complexes. For example, if the antigen binding molecule is disposed on a bead, plate or bag the unbound components of the reaction mixture may be washed away using a solution that leaves formed binding complexes intact. If a binding complex is disposed on a bead, the bead itself may be situated in a column or other structure and the same approach may be used.

The solution used to induce the formation of binding complexes may be used, for example, as a wash solution to remove unbound components. Any suitable buffer or solution that does not disrupt formed binding complexes may also be used. Typically, buffers having high salt concentrations, non-physiological pH, containing chaotropes or denaturants, should be avoided when performing this step of the method.

At this stage of the method, a population of cells presenting a molecule comprising the a BCMA binding molecule will be present. If a detectable label was employed, the concentration of the cells may be easily determined, consistent with the nature of the label. Cells not expressing the molecule comprising a BCMA binding molecule will be absent, and thus the population (or concentration) of cells presenting a molecule comprising a BCMA binding moleculewill be increased compared to the levels prior to performing the method.

If the concentration of the molecule comprising a BCMA binding molecule is not at a desired level, the above steps may be repeated a desired number of times. In the context of this step of the method, a desired number of times may also be zero, if the desired concentration of cells is already present.

### Vf. Method of Depleting a Population of Immune Cells

When a subject has an immune cell-mediated condition, it may be of significant importance that the condition be controlled in a timely fashion so as to prevent harm to the subject. For example, when a subject has an autoimmune reaction it may be desirable to suppress an immune cell-mediated response by depleting a population of immune cells, in an effort to prevent harm. In another example, a subject receiving immunotherapy may react too strongly to the therapy and be at risk of harm; depleting the population of immune cells administered to the subject may be an effective approach to mitigating the subject's reaction to the immunotherapy. In view of the need for a method of controlling a subject's immune cell-mediated response, a method of depleting a population of immune cells presenting a molecule comprising a BCMA binding molecule is provided. An antigen binding molecule that specifically recognizes an anti-BCMA scFv, e.g., those having one or more of the CDRs shown in Figures 4A-11, may be employed in the method.

In some embodiments, the method comprises providing a population of immune cells to be depleted, wherein the cells are known or suspected to be expressing a molecule comprising a BCMA binding molecule.

In specific embodiments, the molecule comprising a BCMA binding molecule is a CAR. When the molecule is a CAR it may comprise a molecule, or fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CD1 ld, ITGAE, CD103, ITGAL, CD1 la, LFA-1, ITGAM, CD1 lb, ITGAX, CD1 lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof.

An immune cell expressing a molecule comprising a BCMA binding molecule sequence may be of any type, and may be human or non-human (*e.g*., mouse, rate, rabbit, hamster, etc). An immune cell of the method may be any type of immune cell (*e.g*., B lymphocytes, monocytes, dendritic cells, Langerhans cells, keratinocytes, endothelial cells, astrocytes, fibroblasts, and oligodendrocytes). T cells (including T cytotoxic, T helper and Treg cells) are especially preferred. In specific embodiments, the cells are T cells, which may be obtained as described herein and by methods known in the art. Any type of immune cell may be employed in this embodiment of the disclosed method, and the cell may be a human or non-human cell (including both prokaryotic and eukaryotic cells). Exemplary cells include, but are not limited to immune cells such as T cells, tumor infiltrating lymphocytes (TILs), NK cells, TCR-expressing cells, dendritic cells, and NK-T cells. The T cells may be autologous, allogeneic, or heterologous. In additional embodiments, the cells are T cells presenting a CAR. The T cells may be CD4+ T cells or CD8+ T cells. When a T cell is employed in the disclosed methods, the T cell may be an in vivo T cell or an in vitro T cell. Moreover, the cells may be disposed in, or isolated from, any environment capable of maintaining the cells in a viable form, such as blood, tissue or any other sample obtained from a subject, cell culture media, tissue grown *ex vivo*, a suitable buffer, etc. As the disclosed method may be employed in therapeutic settings, in preferred embodiments the population of immune cells are disposed in a subject, and more preferably a human subject.

Continuing, the immune cells are contacted with an antigen binding molecule that specifically binds to (a) the molecule comprising a BCMA binding molecule, and (b) an activating molecule expressed on the surface of an immune cell not expressing the molecule comprising a BCMA binding molecule, under conditions that permit the formation of a ternary binding complex comprising the molecule comprising a BCMA binding molecule, the activating molecule and the antigen binding molecule. The antigen binding molecule may be disposed on any surface, or no surface at all. For example, the antigen binding molecule (which may also comprise the population of immune cells to be depleted and/or may be present in a buffer) and the buffer-antigen binding molecule may be contacted with the sample. Alternatively, the antigen binding molecule may be associated with a surface. Suitable surfaces include agarose beads, magnetic beads such as DYNABEADS, or a plastic, glass or ceramic plate such as a welled plate, a bag such as a cell culture bag, etc. The surface may itself be disposed in another structure, such as a column.

The immune cells are contacted with the antigen binding molecule, under conditions that permit the formation of a ternary binding complex comprising a molecule comprising a BCMA binding molecule sequence, the antigen binding molecule and an activating molecule expressed on the surface of an immune cell not expressing the molecule comprising a BCMA binding molecule. Conditions that permit the formation of a binding complex will be dependent on a variety of factors, however generally aqueous buffers at physiological pH and ionic strength, such as in phosphate-buffered saline (PBS), will favor formation of binding complexes and are preferred in the disclosed method. Since the component parts of a binding complex may be disposed on surfaces as described herein, formed binding complexes may also be disposed on surfaces.

In preferred embodiments, the contacting is performed by administering the antigen binding molecule directly to a subject. In this embodiment, the subject will already have a population of cells to be depleted, wherein the cells express a molecule comprising a BCMA binding molecule sequence. Thus, these cells, as well as cells presenting an activating molecule, will be present in the subject prior to the administration of the antigen binding molecule to the subject. The human blood, lymph and tissue environment will permit the formation of ternary binding complexes. The binding of the antigen binding molecule with the molecule comprising a BCMA binding molecule sequence serves to "tag" those cells presenting the molecule comprising a BCMA binding molecule (*i.e*., the cells to be depleted). This binding event may or may not lead to depletion on its own. When the antigen binding molecule binds the activating molecule to form the ternary binding complex, however, this binding event brings both cells (*i.e*., the cell expressing the molecule comprising a BCMA binding molecule, and the cell expressing the activating molecule) together into proximity. The physiological result of the binding event is the killing of the cell expressing the molecule comprising a BCMA binding molecule. Thus, with multiple binding events occurring throughout the subject the population of immune cells bearing the molecule comprising a BCMA binding molecule are depleted and the risk of harm to the subject decreases.

### SEQUENCES AND SEQ ID NOs

The instant disclosure comprises a number of nucleic acid and polypeptide sequences. For convenience, Table E below correlates each sequence with its appropriate description and SEQ ID NO.

**Table E. Sequence ID Numbers**

| SEQ ID NO: | Sequence | Description |
|---|---|---|
| 4 | ATGGAGACTGGGCTGCGCTGGCTTCTCCTGGTCGCTGTGCTCA AAGGTGTCCAGTGTCAGCAGCAGCTGGAGGAGTCCGGGGGAG GCCTGGTCAAGCCTGGAGGAACCCTGACACTCACCTGCACAGC CTCTGGATTCTCCTTCAGTAGCGGCCAATGGATGTGTTGGGTC CGCCAGGCTCCAGGGAAGGGGCTGGAATTGATCGCATGCATTT ATACTAGTGCTGGTGGTAGCACACATTACGCGAGCTGGGCGAC AGGCCGATTCACCATCTCCAAAACCTCGTCGACCACGGTGACT CTGCAAATGACCAGTCTGACAGCCGCGGACACGGCCACCTATT TCTGTGCGAGAGATCCCTATCTTCTTAGTGCTGGTCCTGATAAT AATTTGTGGGGCCCAGGCACCCTGGTCGCCGTCTCCTCA | >9-1_Vh DNA |
| 5 | ATGGAGACTGGGCTGCGCTGGCTTCTCCTGGTCGCTGTGCTCA AAGGTGTCCAGTGTCAGCAGCAGCTGGAGGAGTCCGGGGGAG GCCTGGTCAAGCCTGGAGGAACCCTGACACTCACCTGCACAGC CTCTGGATTCTCCTTCAGTAGCGGCCAAAGGATGTGTTGGGTC CGCCAGGCTCCAGGGAAGGGGCTGGAATTGATCGCATGCATTT ATACTAGTGCTGGTGGTAGCACACATTACGCGAGCTGGGCGAC AGGCCGATTCACCATCTCCAAAACCTCGTCGACCACGGTGACT CTGCAAATGACCAGTCTGACAGCCGCGGACACGGCCACCTATT TCTGTGCGAGAGATCCCTATCTTCTTAGTGCTGGTCCTGATAAT AATTTGTGGGGCCCAGGCACCCTGGTCGCCGTCTCCTCA | 9-9_Vh DNA |
| 6 | ATGGAGACTGGGCTGCGCTGGCTTCTCCTGGTCGCTGTGCTCA AAGGTGTCCAGTGTCAGGAGCGCCTGGTGGAGTCCGGGGGAG GCCTGGTCCAGCCTGAGGGATCCCTGACACTCACCTGCACAGC CTCTGGACTCACCCTCAGTAACTACTGGCTGTGCTGGGTCCGC CAGGCTCCAGGGAAGGGGCTGGAGTGGATCGCATGCATTTAT ACTGGTAATTATGGTAGCGCTTACTACGCGAACTGGGTGAAAG GCCGATTCACCATCTCCAAAGCCTCGTCGACCACGGTGACTCT GCAAATGACCAGTCTGACAGCCGCGGACACGGCCACCTATTTC TGTGCGAGAGATCCTTATACTCTAACCAATGTTGATGTCTTAT GGGGCCCAGGCACCCTGGTCACCGTCTCCTCA | 18-4_Vh DNA |
| 7 | ATGGAGACTGGGCTGCGCTGGCTTCTCCTGGTCGCTGTGCTCA AAGGTGTCCAGTGTCAGGAGCAGTTGGAGGAGTCCGGGGGAG ACCTGGTCAAGCCTGGGGCATCCCTGACACTCACCTGCACAGC CTCTGGATTCTCCTTCAGTAGCGGCTATTGGATATGTTGGGTCC | 63-1_Vh DNA |
| | GCCAGGCTCCAGGGAAGGGGCTGGAGTGGATCGCATGCATTT ATACTCGTAGTGGTGGTAGCACTCACTACGCGAGCTGGGCGAA AGGCCGATTCACCACCTCCAAAACCTCGTCGACCACGGTGACT CTGCAAATGACCAGTCTGACAGCCGCGGACATGGCCACCTATT TCTGTGCGAGAGATCCCTATGTTCTTAGTGCTGGTCCTGATAAT GTTTTGTGGGGCCCAGGCACCCTGGTCACCGTCTCCTCA | |
| 8 | ATGGAGACTGGGCTGCGCTGGCTTCTCCTGGTCGCTGTGCTCA AAGGTGTCCAGTGTCAGGAGCAGCTGGAGGAGTCCGGGGGAG ACCTGGTCAAGCCTGAGGGATCCCTGACACTCACCTGCACAGC CTCTGGATTCTCCTTCAGTAGCGCCTATTGGATATGTTGGGTCC GCCAGGTTCCAGGGAAGGGGCTGGAGTGGATCGCATGCATTT ATACTGGTAGTGGTGGTAGGTCTCACTACGCGAGCTGGGCGAA AGGCCGATTCACCATCTCCAAAACCTCGTCGACCACGGTGACT CTGCAAATGACCAGTCTGACAGCCGCGGACACGGCCACCTATT TCTGTGCGAGAGATCCCTATATTCTTAGTGCTGGTCCTGATAAT GTTTTGTGGGGCCCAGGCACCCTGGTCACCGTCTCCTCA | 80-7_Vh DNA |
| 9 | ATGGAGACTGGGCTGCGCTGGCTTCTCCTGGTCGCTGTGCTCA AAGGTGTCCAGTGTCAGGAGCAGCTGGTGGAGTCCGGGGGAG GCCTGGTCCAGCCTGAGGGATCCCTGACACTCACCTGCACAGC CTCTGGATTCTCCTTCAGTCTCAGCAATTGGATATGCTGGGTCC GCCAGGCTCCAGGGAAGGGGCTGGAGTGGGTCGCATGCATTT ATGTTAGTGTGAGCGGTAACACTCGCTACGCGAGCTGGGCGA AAGGCCGATTCACCATCTCCGAAACCTCGTCGACCACGGTGAC TCTGCAAATGACCGGTCTGACAGCCGCGGACACGGCCACCTAT TTCTGTGCGCGAGATCCATTTAGCCTTGTGAGTGTTGATCGTTC CTTATGGGGCCCAGGCACCCTGGTCACCGTCTCCTCA | 137-2_Vh DNA |
| 10 | ATGGAGACTGGGCTGCGCTGGCTTCTCCTGGTCGCTGTGCTCA AAGGTGTCCAGTGTCAGTCGTTGGAGGAGTCCGGGGGAGACC TGGTCAAGCCTGAGGGATCCCTGACACTCACCTGCACAGCCTC TGGATTCTCCTTCAGTAGCAGCTACTGGATATGCTGGGTCCGC CAGGCTCCAGGGAAGGGGCTGGAGTGGATCGCATGCATTTAT ACCGCTAACAGTGGTCGCACTGTCTACGCGAGCTGGGCGAAG GGCCGATTCACCATCTCCAAAACCTCGTCGACCACGGTGACTC TGCAGCTGCCCAGTCTGACAGCCGCGGACACGGCCACCTATTT CTGTGCGAGAGATATTTATGTGCTTACTACTACTGATAACTTGT GGGGCCCAGGCACCCTGGTCACCGTCTCCTCA | 154-7_Vh DNA |
| 11 | METGLRWLLLVAVLKGVQCQQLEESGGGLVKPGGTLTLTCTA SGFSFSSGQWMCWVRQAPGKGLELIACIYTSAGGSTHYASWATG | >9-1 Vh Protein |
| | RFTISKTSSTTVTLQMTSLTAADTATYFCARDPYLLSAGPDNNLW GPGTLVAVSS | |
| 12 | METGLRWLLLVAVLKGVQCQQQLEESGGGLVKPGGTLTLTCTA SGFSFSSGQRMCWVRQAPGKGLELIACIYTSAGGSTHYASWATG RFTISKTSSTTVTLQMTSLTAADTATYFCARDPYLLSAGPDNNLW GPGTLVAVSS | 9-9_Vh Protein |
| 13 | METGLRWLLLVAVLKGVQCQERLVESGGGLVQPEGSLTLTCTAS GLTLSNYWLCWVRQAPGKGLEWIACIYTGNYGSAYYANWVKG RFTISKASSTTVTLQMTSLTAADTATYFCARDPYTLTNVDVLWGP GTLVTVSS | 18-4_Vh Protein |
| 14 | METGLRWLLLVAVLKGVQCQEQLEESGGDLVKPGASLTLTCTAS GFSFSSGYWICWVRQAPGKGLEWIACIYTRSGGSTHYASWAKGR FTTSKTSSTTVTLQMTSLTAADMATYFCARDPYVLSAGPDNVLW GPGTLVTVSS | 63-1_Vh Protein |
| 15 | METGLRWLLLVAVLKGVQCQEQLEESGGDLVKPEGSLTLTCTAS GFSFSSAYWICWVRQVPGKGLEWIACIYTGSGGRSHYASWAKGR FTISKTSSTTVTLQMTSLTAADTATYFCARDPYILSAGPDNVLWG PGTLVTVSS | 80-7_Vh Protein |
| 16 | METGLRWLLLVAVLKGVQCQEQLVESGGGLVQPEGSLTLTCTAS GFSFSLSNWICWVRQAPGKGLEWVACIYVSVSGNTRYASWAKG RFTISETSSTTVTLQMTGLTAADTATYFCARDPFSLVSVDRSLWG PGTLVTVSS | 137-2_Vh Protein |
| 17 | METGLRWLLLVAVLKGVQCQSLEESGGDLVKPEGSLTLTCTASG FSFSSSYWICWVRQAPGKGLEWIACIYTANSGRTVYASWAKGRF TISKTSSTTVTLQLPSLTAADTATYFCARDIYVLTTTDNLWGPGTL VTVSS | 154-7_Vh Protein |
| 18 | ATGGACACGAGGGCCCCCACTCAGCTGCTGGGGCTCCTGCTGC TCTGGCTCCCAGATGCCAGATGTGCGCTTGTGATGACCCAGAC TCCATCCCCTGTGTCTGCAGCTGTGGGAGGCACAGTCACCATC AATTGCCAGGCCAGTCAGAGCATTGGTAGCGACTTTACCTGGT GGCAGCAGAAACCAGGGCAGCCTCCCAAACTCCTGATCTACT GGGCATCCACTCTGGCATCTGGGGTCTCATCGCGGTTCAAAGG CAGTGGATCTGGGACACAGTTCACTCTCACCATCAGCGACCTG GAGTGTGACGATGCTGCCACTTACTACTGTGCAGGCTATGTAA GTTATAGTGATGATGGTAATGCTTTCGGCGGAGGGACCGAGGT GGTGGTCAAA | >9-1_Vk |
| 19 | ATGGACACGAGGGCCCCCACTCAGCTGCTGGGGCTCCTGCTGC TCTGGCTCCCAGATGCCAGATGTGCGCTTGTGATGACCCAGAC TCCATCCCCTGTGTCTGCAGCTGTGGGAGGCACAGTCACCATC | >9-9_Vk |
| | AATTGCCAGGCCAGTCAGAGCATTGGTAGCGACTTTACCTGGT GGCAGCAGAAACCAGGGCAGCCTCCCAAACTCCTGATCTACT GGGCATCCACTCTGGCATCTGGGGTCTCATCGCGGTTCAAAGG CAGTGGATCTGGGACACAGTTCACTCTCACCATCAGCGACCTG GAGTGTGACGATGCTGCCACTTACTACTGTGCAGGCTATGTAA GTTATAGTGATGATGGTAATGCTTTCGGCGGAGGGACCGAGGT GGTGGTCAAA | |
| 20 | ATGGACACGAGGGCCCCCACTCAGCTGCTGGGGCTCCTGCTGC TCTGGCTCCCAGGTGCCAGATGTGCCCTTGTGATGACCCAGAC TCCATCCCCCGTGTCTGCAGCTGTGGGAGGCACAGTCACCATC AATTGCCAGGCCAGTCAGAGCATTGGTAGCGACTTAGCCTGGT ATCAGCAGAGACCAGGGCAGCCTCCCAAGCTCCTGATCTACTG GGCATCCACTCTGGCATCTGGGGTCCCATCGCGGTTCAGCGGC AGTGGATCTGGGACACAGTTCACTCTCACCATCAGCGACCTGG AGTGTGACGATGCCGCCACTTACTACTGTACAGGCTATATAAG TTATAATGATGATAATGCCGCGTTCGGCGGAGGGACCGAGGT GGTGGTCAAA | >18-4_Vk |
| 21 | ATGGACACGAGGGCCCCCACTCAGCTGCTGGGGCTCCTGCTGC TCTGGCTCCCAGATGCCAGATGTGCGCTTGTGATGACCCAGAC TCCATCCCCTGTGTCTGCAGCTGTGGGAGGCACGGTCACCATC AATTGCCAGGCCAGTGAGAGCATTGGTAGCGACTTGGGCTGGT ATCAGCAGAAACCAGGGCAGCCTCCCAAATTCCTGATCTACTG GGCATCCACTTTGGTATCTGGGGTCCCATCGCGGTTCGAAGGC AGTGGATCTGGGACACAGTTCACTCTCACCATCAGCGACCTGG AGTGTGACGATGCTGCCACTTACTACTGTGCAGGCTATGAAAG TTTGAGTAATGATGGTAATGCTTTCGGCGGAGGGACCGAGGTG GTGGTCAAA | >63-1_Vk |
| 22 | ATGGACACGAGGGCCCCCACTCAGCTGCTGGGGCTCCTGCTGC TCTGGCTCCCAGATGCCAGATGTGCGCTTGTGATGACCCAGAC TCCATCCCCTGTGTCTGCAGCTGTGGGAGGCACAGTCACCATC AATTGCCAGGCCAGTCAGAGCATTGGTAGCGACTTAGGCTGGT TTCAGCAGAAACCAGGGCAGCCTCCCAAACTCCTGATCTACTG GGCATCCACTCTGGCATCTGGGGTCCCATCGCGGTTCGAAGGC AGTGGATCTGGGACACAGTTCACTCTCACCATCAGCGACCTGG AGTGTGACGATGCTGCCACTTACTACTGTGCAGGCTATGAAAG TTTGAGTAATGATGGTAATGCTTTCGGCGGAGGGACCGAGGTG GTGGTCAAA | >80-7 Vk |
| 23 | ATGGACACGAGGGCCCCCACTCAGCTGCTGGGGCTCCTGCTGC TCTGGCTCCCAGATGCCAGATGTGCGCTTGTGATGACCCAGAC TCCATCCCCCGTGTCTGCAGCTGTGGGAGGCACAGTCACCATC AATTGCCAGGCCAGTCAGAGCATTGGTAGCGACTTATCCTGGT | >137-2_Vk |
| | TTCAGCAGAAACCAGGGCAGCCTCCCAAGCTCCTGATGTACTG GGCATCCAGTCTGGCATCTGGGGTCCCATCGCGGTTCAAAGGC AGCGGATCTGGGACACAGTTCACTCTCACCATCAGCGACCTGG AGTGTGACGATGCTGCCACTTACTACTGTGCAGGCTATGTAAA TTATAATAATGATGATGCTGCTTTCGGCGGAGGGACCGAGGTG GTGGTCAAA | |
| 24 | ATGGACACGAGGGCCCCCACTCAGCTGCTGGGGCTCCTGCTGC TCTGGCTCCCAGATGCCAGATGTGCGCTTGTGATGACCCAGGC TCCATCCCCTGTGTCTGCAGCTGTGGGAGGCACAGTCACCATC AGTTGCCAGGCCAGTCAGCCCATTGGTAGCGACTTAGTTTGGT ATCAGCAGAAACCAGGGCAGCCTCCCAAGCTCCTGATCTATTA TGCATCCGATCTGGCATCTGGGGTCCCATCGCGGTTCAAAGGC AGTGGATCTGGGACACGATTCACTCTCACCATTAGTGGCGTGG AGTGTGACGATGCTGCCACTTACTACTGTGCAGGCTATAAAAA TTATGATCATGATGATTGTGGTTTCGGCGGAGGGACCGAGGTG GTGGTCAAA | >154-7_Vk |
| 25 | MDTRAPTQLLGLLLLWLPDARCALVMTQTPSPVSAAVGGTVTIN CQASQSIGSDFTWWQQKPGQPPKLLIYWASTLASGVSSRFKGSGS GTQFTLTISDLECDDAATYYCAGYVSYSDDGNAFGGGTEVVVK | >9-1_Vk |
| 26 | MDTRAPTQLLGLLLLWLPDARCALVMTQTPSPVSAAVGGTVTIN CQASQSIGSDFTWWQQKPGQPPKLLIYWASTLASGVSSRFKGSGS GTQFTLTISDLECDDAATYYCAGYVSYSDDGNAFGGGTEVVVK | >9-9_Vk |
| 27 | MDTRAPTQLLGLLLLWLPGARCALVMTQTPSPVSAAVGGTVTIN CQASQSIGSDLAWYQQRPGQPPKLLIYWASTLASGVPSRFSGSGS GTQFTLTISDLECDDAATYYCTGYISYNDDNAAFGGGTEVVVK | >18-4_Vk |
| 28 | MDTRAPTQLLGLLLLWLPDARCALVMTQTPSPVSAAVGGTVTIN CQASESIGSDLGWYQQKPGQPPKFLIYWASTLVSGVPSRFEGSGS GTQFTLTISDLECDDAATYYCAGYESLSNDGNAFGGGTEVVVK | >63-1_Vk |
| 29 | MDTRAPTQLLGLLLLWLPDARCALVMTQTPSPVSAAVGGTVTIN CQASQSIGSDLGWFQQKPGQPPKLLIYWASTLASGVPSRFEGSGS GTQFTLTISDLECDDAATYYCAGYESLSNDGNAFGGGTEVVVK | >80-7_Vk |
| 30 | MDTRAPTQLLGLLLLWLPDARCALVMTQTPSPVSAAVGGTVTIN CQASQSIGSDLSWFQQKPGQPPKLLMYWASSLASGVPSRFKGSGS GTQFTLTISDLECDDAATYYCAGYVNYNNDDAAFGGGTEVVVK | >137-2_Vk |
| 31 | MDTRAPTQLLGLLLLWLPDARCALVMTQAPSPVSAAVGGTVTIS CQASQPIGSDLVWYQQKPGQPPKLLIYYASDLASGVPSRFKGSGS GTRFTLTISGVECDDAATYYCAGYKNYDHDDCGFGGGTEVVVK | >154-7_Vk |
| 32 | QASQSIGSDFT | 9-1_VL CDR1 Kabat |
| | QASQSIGSDFT | 9-9_VL CDR1 Kabat |
| 33 | QASQSIGSDLA | 18-4_VL CDR1 Kabat |
| 34 | QASESIGSDLG | 63-1_VL CDR1 Kabat |
| 35 | QASQSIGSDLG | 80-7_VL CDR1 Kabat |
| 36 | QASQSIGSDLS | 137-2_VL CDR1 Kabat |
| 37 | QASQPIGSDLV | 154-7_VL CDR1 Kabat |
| 38 | SGQWMC | 9-1_VH CDR1 Kabat |
| 39 | SGQRMC | 9-9_VH CDR1 Kabat |
| 40 | NYWLC | 18-4_VH CDR1 Kabat |
| 41 | SGYWIC | 63-1_VH CDR1 Kabat |
| 42 | SAYWIC | 80-7_VH CDR1 Kabat |
| 43 | LSNWIC | 137-2_VH CDR1 Kabat |
| 84 | SSYWIC | 154-7_VH CDR1 Kabat |
| 44 | wASTLAs | 9-1_VL CDR2 Kabat |
| | WASTLAS | 9-9_VL CDR2 Kabat |
| | WASTLAS | 18-4_VL CDR2 Kabat |
| 45 | WASTLVS | 63-1_VL CDR2 Kabat |
| 44 | wASTLAs | 80-7_VL CDR2 Kabat |
| 46 | WASSLAS | 137-2_VL CDR2 Kabat |
| 47 | YASDLAS | 154-7_VL CDR2 Kabat |
| 48 | CIYTSAGGSTHYASWAT | 9-1_VH CDR2 Kabat |
| | CIYTSAGGSTHYASWAT | 9-9_VH CDR2 Kabat |
| 49 | CIYTGNYGSAYYANWVK | 18-4_VH CDR2 Kabat |
| 50 | CIYTRSGGSTHYASWAK | 63-1_VH CDR2 Kabat |
| 51 | CIYTGSGGRSHYASWAK | 80-7_VH CDR2 Kabat |
| 52 | CIYVSVSGNTRYASWAK | 137-2_VH CDR2 Kabat |
| 53 | CIYTANSGRTVYASWAK | 154-7_VH CDR2 Kabat |
| 54 | AGYVSYSDDGNA | 9-1_VL CDR3 Kabat |
| | AGYVSYSDDGNA | 9-9_VL CDR3 Kabat |
| 55 | TGYISYNDDNAA | 18-4_VL CDR3 Kabat |
| 56 | AGYESLSNDGNA | 63-1_VL CDR3 Kabat |
| | AGYESLSNDGNA | 80-7_VL CDR3 Kabat |
| 57 | AGYVNYNNDDAA | 137-2_VL CDR3 Kabat |
| 58 | AGYKNYDHDDCG | 154-7_VL CDR3 Kabat |
| 59 | DPYLLSAGPDNNL | 9-1_VH CDR3 Kabat |
| | DPYLLSAGPDNNL | 9-9_VH CDR3 Kabat |
| 60 | DPYTLTNVDVL | 18-4_VH CDR3 Kabat |
| 61 | DPYVLSAGPDNVL | 63-1_VH CDR3 Kabat |
| 62 | DPYILSAGPDNVL | 80-7_VH CDR3 Kabat |
| 63 | DPFSLVSVDRSL | 137-2_VH CDR3 Kabat |
| 64 | DIYVLTTTDNL | 154-7_VH CDR3 Kabat |
| 32 | QASQSIGSDFT | 9-1_VL CDR1 Clothia |
| | QASQSIGSDFT | 9-9_VL CDR1 Clothia |
| 33 | QASQSIGSDLA | 18-4_VL CDR1 Clothia |
| 34 | QASESIGSDLG | 63-1_VL CDR1 Clothia |
| 35 | QASQSIGSDLG | 80-7_VL CDR1 Clothia |
| 36 | QASQSIGSDLS | 137-2_VL CDR1 Clothia |
| 37 | QASQPIGSDLV | 154-7_VL CDR1 Clothia |
| 38 | GFSFSSGQ | 9-1_VH CDR1 Clothia |
| | GFSFSSGQ | 9-9_VH CDR1 Clothia |
| 40 | NYWLC | 18-4_VH CDR1 Clothia |
| 65 | GFSFSSGY | 63-1_VH CDR1 Clothia |
| 66 | GFSFSSAY | 80-7_VH CDR1 Clothia |
| 67 | GFSFSLSN | 137-2_VH CDR1 Clothia |
| 85 | GFSFSSSY | 154-7_VH CDR1 Clothia |
| 44 | wASTLAs | 9-1_VL CDR2 Clothia |
| | WASTLAS | 9-9_VL CDR2 Clothia |
| | WASTLAS | 18-4_VL CDR2 Clothia |
| 45 | wASTLVs | 63-1_VL CDR2 Clothia |
| 44 | WASTLAS | 80-7_VL CDR2 Clothia |
| 46 | wASSLAs | 137-2_VL CDR2 Clothia |
| 47 | YASDLAS | 154-7_VL CDR2 Clothia |
| 68 | YTSAGG | 9-1_VH CDR2 Clothia |
| | YTSAGG | 9-9_VH CDR2 Clothia |
| 69 | YTGNYG | 18-4_VH CDR2 Clothia |
| 70 | YTRSGG | 63-1_VH CDR2 Clothia |
| 71 | YTGSGG | 80-7_VH CDR2 Clothia |
| 72 | YVSVSG | 137-2_VH CDR2 Clothia |
| 73 | YTANSG | 154-7_VH CDR2 Clothia |
| 54 | AGYVSYSDDGNA | 9-1_VL CDR3 Clothia |
| | AGYVSYSDDGNA | 9-9_VL CDR3 Clothia |
| 55 | TGYISYNDDNAA | 18-4_VL CDR3 Clothia |
| 56 | AGYESLSNDGNA | 63-1_VL CDR3 Clothia |
| | AGYESLSNDGNA | 80-7_VL CDR3 Clothia |
| 57 | AGYVNYNNDDAA | 137-2_VL CDR3 |
| | | Clothia |
| 58 | AGYKNYDHDDCG | 154-7_VL CDR3 Clothia |
| 59 | DPYLLSAGPDNNL | 9-1_VH CDR3 Clothia |
| | DPYLLSAGPDNNL | 9-9_VH CDR3 Clothia |
| 60 | DPYTLTNVDVL | 18-4_VH CDR3 Clothia |
| 61 | DPYVLSAGPDNVL | 63-1_VH CDR3 Clothia |
| 62 | DPYILSAGPDNVL | 80-7_VH CDR3 Clothia |
| 63 | DPFSLVSVDRSL | 137-2_VH CDR3 Clothia |
| 64 | DIYVLTTTDNL | 154-7_VH CDR3 Clothia |
| 86 | ATGGAGACTGGGCTGCGCTGGCTTCTCCTGGTCGCTGTGCTCA AAGGTGTCCAGTGTCAGTCGTTGGAGGAGTCCGGGGGAGCCC TGGTCAAGCCTGGGGCATCCCTGGCACTCACCTGCACAGCCTC TGGATTCTCCTTCAATCTCAGGCACTACATGTGCTGGGTCCGC CAGGCTCCGGGGAAGGGGCTGGAGTGGATCGGATGCGTTCAT GGTGGTAGTAGTGGTGGCACTTACTACGCGAGCTGGACGAAA GGCCGATTCACCATATCCAAAACCTCGTCGACCACGGTGACTC TGCAAATGACCAGTCTGACAGCCGCGGACACGGCCACTTATTT CTGTGCGAGAGAGGATGATATTATGATGGCCGGAGAATTTAG CTTGTGGGGCCCAGGCACCCTGGTCACCGTCTCCTCA | 77-9_Vh |
| 87 | ATGGAGACTGGGCTGCGCTGGCTTCTCCTGGTCGCTGTGCTCA AAGGTGTCCAGTGTCAGTCGTTGGAGGAGTCCGGGGGAGACC TGGTCAAGCCTGGGGCATCCCTGACACTCACCTGCACAGCCTC TGGATTCTCCTTCAATCTCAGGCACTACATGTGCTGGGTCCGC CAGGCTCCGGGGAAGGGGCTGGAGTGGATCGGATGCGTTCAT GGTGGTAGTAGTAGTGGCACTTACTACGCGAGCTGGGCGAAA GGCCGATTCACCATATCCAAAACCTCGTCGACCACGGTGACTC TGCAAATGACCAGTCTGACAGCCGCGGACACGGCCACTTACTT CTGTACGAGAGAGGATGATATTATGATGGCCGGAGAGTTTGG CTTGTGGGGCCCAGGCACCCTGGTCACCGTCTCCTCA | 83-3_Vh |
| 88 | ATGGAGACTGGGCTGCGCTGGCTTCTCCTGGTCGCTGTGCTCA AAGGTGTCCAGTGTCAGGAGCAGCTGGTGGAGTCCAGGGGAG GCCTGGTCAAGCCTGGGGGATCCCTGACACTCACCTGCAAAGC CTCTGGATTCTCCTTCAGTACCGGCTACTACGCGTGCTGGGTCC GCCAGGCTCCAGGGAAGGGGCTGGAATTGAGCGCATGCGTTG ATACTGGTAGTGGTGACACTTTCTACGCGAGCTGGGCGAGAGG CCGATTCACCATCTCCAAAACCTCGTCATCCACGGTGACTCTG CAAATGACCAGTCTGACAGTTGCGGACACGGCCACCTATTTCT GTGCGAGGACGGCGCTAGTGATGACTGATTTCTACTTTAACTT | 89-10_Vh |
| | GTGGGGCCCAGGCACCCTGACCACCGTCTCCTCA | |
| 89 | METGLRWLLLVAVLKGVQCQSLEESGGALVKPGASLALTCTASG FSFNLRHYMCWVRQAPGKGLEWIGCVHGGSSGGTYYASWTKGR FTISKTSSTTVTLQMTSLTAADTATYFCAREDDIMMAGEFSLWGP GTLVTVSS | 77-9_Vh |
| 90 | METGLRWLLLVAVLKGVQCQSLEESGGDLVKPGASLTLTCTASG FSFNLRHYMCWVRQAPGKGLEWIGCVHGGSSSGTYYASWAKGR FTISKTSSTTVTLQMTSLTAADTATYFCTREDDIMMAGEFGLWGP GTLVTVSS | 83-3_Vh |
| 91 | METGLRWLLLVAVLKGVQCQEQLVESRGGLVKPGGSLTLTCKA SGFSFSTGYYACWVRQAPGKGLELSACVDTGSGDTFYASWARG RFTISKTSSSTVTLQMTSLTVADTATYFCARTALVMTDFYFNLWG PGTLTTVSS | 89-10_Vh |
| 92 | ATGGACACGAGGGCCCCCACTCAGCTGCTGGGGCTCCTACTGC TCTGGCTCCCAGGTGCCAGATGTGCTGACATTGTGATGACCCA GACTCCATCCTCCGTGGAGGCATCTGTGGGAGGCACAGTCACC ATCAAGTGCCAGGCCAGTGAGGACATTGAAAGCTACTTAGTCT GGTATCAGCAGAAACCAGGGCAACCTCCCAAGCTCCTGATCTA CCAGGCATCCAATCTGGCCTCTGGGGTCCCATCGCGGTTCTAC GGCAGTGGATCTGGGACACAGTTCACTCTCACCATCAGCGACC TGGAGTGTGCCGATGCTTCCACTTACTACTGTCAAAGTTATGTT GAAGGTGGTGTTGATGTTGTTTTCGGCGGAGGGACCGAGGTGG TGGTCAAA | >77-9_Vk |
| 93 | ATGGACACGAGGGCCCCCACTCAGCTGCTGGGGCTCCTACTGC TCTGGCTCCCAGGTGCCAGATGTGGTGACATTGTGATGACCCA GACTCCATCCTCCGTGTCTGAACCTGTGGGAGGCACAGTCACC ATCAAGTGCCAGGCCAGTGAGGACATTGAAAGCTATTTAGTCT GGTATCAGCAGAAACCAGGGCAGCCTCCCAAACTCCTGATCTA CCAGGCATCCAATCTGGCCTCTGGGGTCCCATCGCGGTTCAGC GGCAGTGGATCTGGGACAGAGTACACTCTCACCATCAGCGAC CTGGAGTGTGCCGATGCTGCCACTTACTACTGTCAAAGTTATA TTGAAGGTGGTGTTGATGTTGTTTTCGGCGGAGGGACCGAGGT GGTGGTCAAA | >83-3_Vk |
| 94 | ATGGACACGAGGGCCCCCACTCAGCTGCTGGGGCTCCTGCTGC TCTGGCTCCCAGGTGCCAGATGTGCCCTTGTGATGACCCAGAC TCCAGCCTCCGTGGAGGCAGCTGTGGGAGGCACAGTCACCATC AATTGCCAGGCCAGTCAGTGGATTAGCAATGAATTATCCTGGT ATCAGCAGAAACCAGGGCAGCCTCCCAAGCTCCTGATCTATCT GGCATCTACTCTGGCATCTGGGGTCCCATCGCGGTTCAAAGGC AGTGGATCTGGGACAGAGTTCACTCTCACCATCAGCGACCTGG | >89-10_Vk |
| | AGTGTGCCGATGCTGCCACTTACTACTGTCAACAGGGTTATAA TATAGGCCATCTTGATAATGCTTTCGGCGGAGGGACCGAGGTG GTGGTCAAA | |
| 95 | MDTRAPTQLLGLLLLWLPGARCADIVMTQTPSSVEASVGGTVTI KCQASEDIESYLVWYQQKPGQPPKLLIYQASNLASGVPSRFYGSG SGTQFTLTISDLECADASTYYCQSYVEGGVDVVFGGGTEVVVK | >77-9_Vk |
| 96 | MDTRAPTQLLGLLLLWLPGARCGDIVMTQTPSSVSEPVGGTVTIK CQASEDIESYLVWYQQKPGQPPKLLIYQASNLASGVPSRFSGSGS GTEYTLTISDLECADAATYYCQSYIEGGVDVVFGGGTEVVVK | >83-3_Vk |
| 97 | MDTRAPTQLLGLLLLWLPGARCALVMTQTPASVEAAVGGTVTIN CQASQWISNELSWYQQKPGQPPKLLIYLASTLASGVPSRFKGSGS GTEFTLTISDLECADAATYYCQQGYNIGHLDNAFGGGTEVVVK | >89-10_Vk |
| 98 | QASEDIESYLV | 77-9_VL CDR1 (Kabat) |
| | | 83-3_VL CDR1 (Kabat) |
| 99 | QASQWISNELS | 89-10_VL CDR1 (Kabat) |
| 100 | QASNLAS | 77-9_VL CDR2 (Kabat) |
| | | 83-3_VL CDR2 (Kabat) |
| 101 | LASTLAS | 89-10_VL CDR2 (Kabat) |
| 102 | QSYVEGGVDW | 77-9_VL CDR3 (Kabat) |
| 103 | QSYIEGGVDVV | 83-3_VL CDR3 (Kabat) |
| 104 | QQGYNIGHLDNA | 89-10_VL CDR3 (Kabat) |
| 105 | LRHYMC | 77-9_VH CDR1 (Kabat) |
| | | 83-3_VH CDR1 (Kabat) |
| 106 | TGYYAC | 89-10_VH CDR1 (Kabat) |
| 107 | CVHGGSSGGTYYASWTK | 77-9_VH CDR2 (Kabat) |
| 108 | CVHGGSSSGTYYASWAK | 83-3_VH CDR2 (Kabat) |
| 109 | CVDTGSGDTFYASWAR | 89-10_VH CDR2 (Kabat) |
| 110 | EDDIMMAGEFSL | 77-9_VH CDR3 (Kabat) |
| 111 | EDDIMMAGEFGL | 83-3_VH CDR3 (Kabat) |
| 112 | TALVMTDFYFNL | 89-10_VH CDR3 (Kabat) |
| 113 | QASEDIESYLV | 77-9_VL CDR1 (Clothia) |
| | | 83-3_VL CDR1 (Clothia) |
| 114 | QASQWISNELS | 89-10_VL CDR1 (Clothia) |
| 115 | QASNLAS | 77-9_VL CDR2 (Clothia) |
| | | 83-3_VL CDR2 (Clothia) |
| 116 | LASTLAS | 89-10_VL CDR2 (Clothia) |
| 117 | QSYVEGGVDW | 77-9_VL CDR3 (Clothia) |
| 118 | QSYIEGGVDVV | 83-3_VL CDR3 (Clothia) |
| 119 | QQGYNIGHLDNA | 89-10_VL CDR3 (Clothia) |
| 120 | GFSFNLRH | 77-9_VH CDR1 (Clothia) |
| | | 83-3_VH CDR1 (Clothia) |
| 121 | GFSFSTGY | 89-10_VH CDR1 (Clothia) |
| 122 | HGGSSG | 77-9_VH CDR2 (Clothia) |
| 123 | HGGSSS | 83-3_VH CDR2 (Clothia) |
| 124 | DTGSG | 89-10_VH CDR2 (Clothia) |
| 125 | EDDIMMAGEFSL | 77-9_VH CDR3 (Clothia) |
| 126 | EDDIMMAGEFGL | 83-3_VH CDR3 (Clothia) |
| 127 | TALVMTDFYFNL | 89-10_VH CDR3 (Clothia) |
| 128 | GSTSGSGKPGSGEGSTK | Linker Sequence |

### INCORPORATION BY REFERENCE

All publications, patents, and patent applications mentioned in this specification are herein incorporated by reference to the same extent as if each individual publication, patent, or patent application was specifically and individually indicated to be incorporated by reference. However, the citation of a reference herein should not be construed as an acknowledgement that such reference is prior art to the present invention. To the extent that any of the definitions or terms provided in the references incorporated by reference differ from the terms and discussion provided herein, the present terms and definitions control.

The foregoing written specification is considered to be sufficient to enable one skilled in the art to practice the invention. The foregoing description and Examples that follow detail certain preferred embodiments of the invention and describe the best mode contemplated by the inventors. It will be appreciated, however, that no matter how detailed the foregoing may appear in text, the invention may be practiced in many ways and the invention should be construed in accordance with the appended claims and any equivalents thereof.

### EXAMPLES

The present invention is further illustrated by the following examples, which should not be construed as further limiting. The contents of all references cited throughout this application are expressly incorporated herein by reference.

### EXAMPLE 1

### Generation and Screening of Antigen Binding Molecules

Monoclonal antibodies were generated through immunization of rabbits using a BCMA binding molecule, conjugated to Fc as immunogen. Titer was determined via screening polyclonal sera by ELISA. A secondary screen was performed using CAR T cells assayed via flow cytometry. Once titer was achieved, the immunized rabbits were sacrificed and monoclonals were derived using standard hybridoma generation and subcloning techniques. The final screening of the hybridoma subclones was accomplished via additional rounds of flow cytometry of proliferating CAR T cells. The sequences of the final subclones selected were determined by standard Sanger sequencing of the hybridomas subclones.

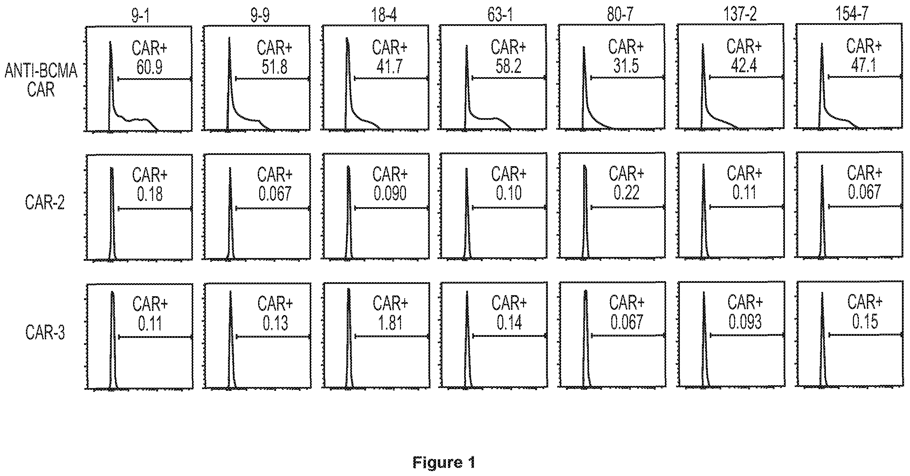

PBMCs were isolated from healthy donor leukopaks (Hemacare^{™}) using ficoll-paque density centrifugation per manufacturer's instructions. PBMCs were stimulated using OKT3 (50ng/ml, Miltenyi Biotec^{™}) in OpTmizer media + OpTmizer supplements + IL-2 (300IU/ml, Proleukin^{®}, Prometheus^{®} Therapeutics and Diagnostics). Two days after stimulation, CAR T cells presenting the anti-BCMA scFv were generated through viral transduction of these activated primary human T cells. Transduction was performed using lentivirus to express the CAR. Analogously, CAR-expressing Jurkat cells (E6-1, ATCC) cultured in RPMI-1640 + 10% FBS were generated by lentiviral transduction. Confirmation of CAR construct expression and viral transduction efficiency was determined using an antibody specific to the CAR linker sequence GSTSGSGKPGSGEGSTK (SEQ ID NO: 128). Monoclonal antibody supernatants were incubated with CAR+ cells and detected with a FITC-conjugated goat anti-rabbit F(ab')2 antibody. Results from flow cytometry experiments showing antibody binding to the anti-BCMA CAR are shown in Figure 1

### EXAMPLE 2

### Flow Cytometry Studies

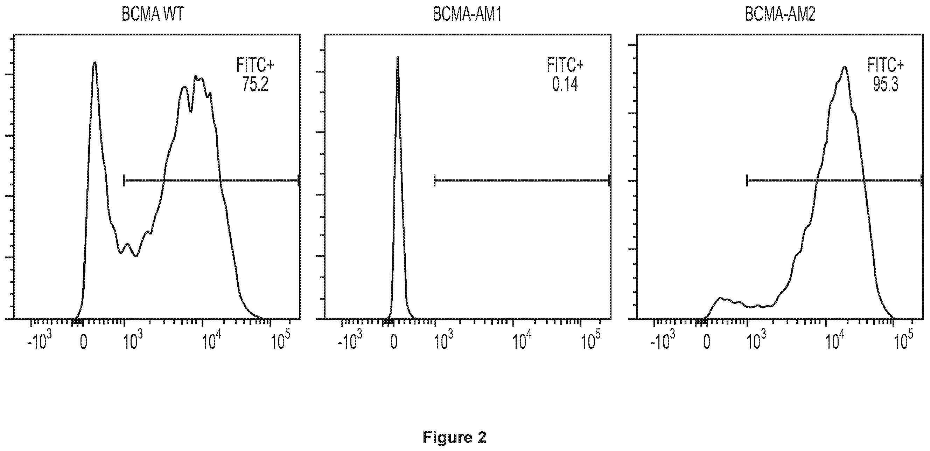

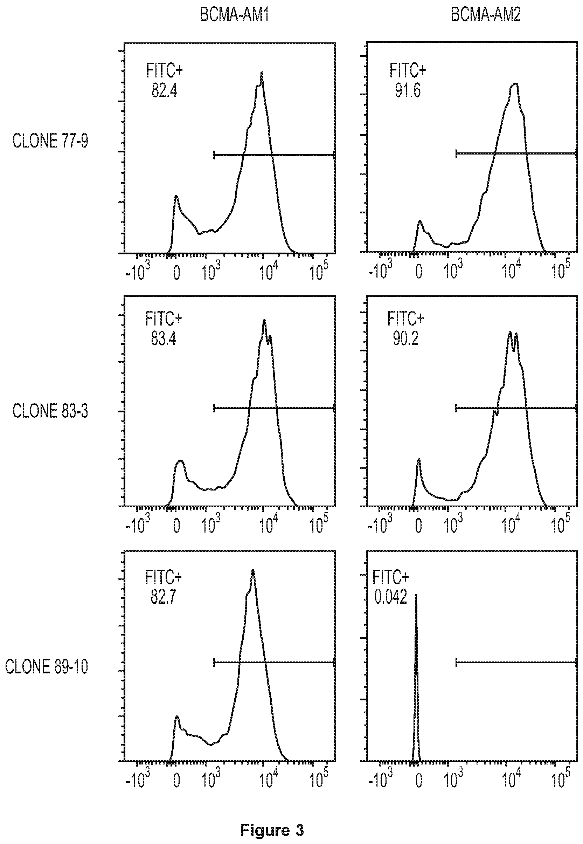

CAR-expressing Jurkat cells (E6-1, ATCC) cultured in RPMI-1640 + 10% FBS were generated by lentiviral transduction with anti-BCMA CAR and affinity-matured variants of the CAR scFv. Confirmation of CAR construct expression and viral transduction efficiency was determined using an antibody specific to the CAR linker sequence GSTSGSGKPGSGEGSTK (SEQ ID NO: 128). Monoclonal antibody supernatants were incubated with CAR+ cells and detected with a FITC-conjugated goat anti-rabbit F(ab')2 antibody. Results from flow cytometry experiments showing antibody binding to the anti-BCMA CAR are shown in Figure 2 Affinity-matured variants of the anti-BCMA CAR, BCMA-AM1 and BCMA-AM2 differ from the parent anti-BCMA antibody in their CDR H3, with BCMA-AM2 and the parent antibody sharing a common CDR H3, and BCMA-AM1 having a different CDR3. Based on this difference and the binding data in Figure 2, the epitope recognized by antibodies 9-1, 9-9, 18-4, 63-1, 80-7, 137-2, and 154-7 comprises CDR H3 of anti-BCMA CARs. Additional clones were identified that bind to common regions among the three constructs and those that bind to the anti-BCMA CAR and the affinity-matured clone BCMA-AM1, but not clone BCMA-AM2. Results are shown in Figure 3.

### EXAMPLE 3

### Use of an Antibody to an Anti-BCMA scFv for Purifying Macromolecules and Cells

The antigen binding molecules disclosed herein are anti-idiotypic antigen binding molecules, and recognize an epitope on a BCMA binding molecule. An antigen binding molecule (e.g., an antibody) disclosed herein may thus be used to purify a molecule, such as a BCMA binding molecule, macromolecule, polymer, cell, material, etc., displaying an epitope that is recognized by the antigen binding molecules disclosed herein.

In some embodiments, an antigen binding molecule disclosed herein (e.g., Clones 9-1, 9-9, 18-4, 63-1, 80-7, 137-2, and/or 154-7 and fragments thereof) may be attached to beads, attached to or associated with a resin, which may be disposed in a column or other structure. A sample comprising a molecule comprising all or a fragment of anti-BCMA scFv may then be contacted with the beads, resin, etc to which the antigen binding molecule was attached or with which an antigen binding molecule was associated. This allows the formation of an association or binding complex comprising the antigen binding molecule and the molecule comprising all or a fragment of a BCMA binding molecule. The beads or resin may then be washed with a suitable solution, such as a buffer solution (*e.g.*, PBS, HEPES, MOPS, Tris, Tricine, etc) having a pH selected to maintain the stability of the molecule comprising all or a fragment of a BCMA binding molecule. The washing may remove unwanted and unbound components of the sample. Following the washing step, the molecule comprising all or a fragment of a BCMA binding molecule may then be eluted from the antigen binding molecules using an elution buffer and conditions selected to disrupt any association or binding complexes formed. Examples of suitable elution buffers include 0.1M glycine, pH 2.5-3.0, and 0.1M citric acid, pH 3.0, 50-100mM triethylamine or triethanolamine, pH 11.5, 3.5-4.0M magnesium chloride, pH 7.0 in 10mM Tris, 2-6M guanidine, and 2-8M urea. During the elution step, eluted molecules, cells and moieties of interest comprising all or a fragment of a BCMA binding molecule is collected, and purity may be subsequently checked by running a sample on an SDS polyacrylamide gel.

In another embodiment, an antigen binding molecule may be disposed in solution with any molecular entity displaying the epitope, and purified from a mixed population of molecules, cells, etc. and eluted from the beads, resin, or free antibody by washing with 300-500 mM sodium chloride or lowering the pH and neutralizing with 1 M Tris, for proteins, or phosphate buffer. Subsequently, dialysis may be used to return materials to desired buffer conditions.

In some embodiments, cells displaying a molecule comprising all or a fragment of a BCMA binding molecule may be incubated with magnetic beads (*e.g*., DYNABEADS) with which an antigen binding molecule disclosed herein has been associated. Preferably the incubation is performed under conditions that both allow for the formation of binding complexes/associations, such as under physiological conditions, in the presence of a media selected for this purpose (e.g., RPMI-1640).

Cells bound by the beads (which will be presenting molecules comprising a BCMA binding molecule) are then separated from cells not displaying a molecule comprising a BCMA binding molecule or fragment thereof. In some embodiments, the beads may be washed with media, such as RPMI-1640 supplemented with 10% FBS, in the presence of a magnet.

Selected cells, *i.e.,* those presenting molecules that comprise a BCMA binding molecule may then be separated from the beads: First, selected cells are grown out in media. After growing out cells for 48 hours, the magnetic beads may be separated from cells in solution and discarded, leaving a pure population of cells expressing desired molecule.

In an alternative embodiment, the beads are not magnetic, and in this embodiment the above steps may also be followed and adapted to maintain cell integrity, but also to allow separation of bead-bound cells from non-bead bound cells.

In another alternative embodiment, an antigen binding molecule disclosed herein (*e.g*., 9-1, 9-9, 18-4, 63-1, 80-7, 137-2, and/or 154-7 and fragments thereof) may be His-tagged (*i.e*., labeled with a short polyhistidine sequence), thereby facilitating the separation of cells using a resin comprising a transition metal ion such as Ni²⁺, Co²⁺, Cu²⁺ or Zn²⁺, which are immobilized on the resin. The antigen binding molecules may then be incubated with cells known or suspected to be expressing a BCMA binding molecule under conditions suitable for the formation of complexes comprising the cells and the antigen binding molecules. Following the incubation, the cells are contacted with the resin, which may be disposed in a solid structure such as a welled plate, column or other structure. The antigen binding molecule-cell complexes may then be separated from one another by washing with imidazole, which will be of a higher concentration than any imidazole included in any solutions used in the formation of the binding complexes. Eluted cells may then be spun down, washed in RPMI or other suitable media, and then resuspended in media.

### EXAMPLE 4

### Activating CAR-positive T cells Using Antibodies to an Anti-BCMA CAR

Also provided is a method of activating CAR-positive T cells presenting a molecule comprising a specific idiotope recognized by a specific antigen binding molecule *(e.g.,* an antigen binding molecule that comprises a BCMA binding molecule, such as those disclosed herein: Clone 9-1, 9-9, 18-4, 63-1, 80-7, 137-2, and/or 154-7, and fragments thereof). This method may be adapted for any antibody recognizing a protein of interest on a T-cell containing an activation domain, such as a chimeric antigen receptor (CAR) comprising a BCMA binding molecule. Activation may be achieved using plate-bound, bead-bound, polymer-bound, or other form of the antibody that specifically recognizes an extracellular component of the CAR or similar molecule.

In some embodiments, the method may be performed as follows: first, a 12-well tissue culture treated plate is coated with 1.5 µg/mL of a BCMA binding molecule antigen binding molecule disclosed herein, which has been diluted in HBSS or other phosphate buffer, and placed in an incubator at 37 C for 2 hours. Next, the plate is washed three times with HBSS or other phosphate buffer having a suitable pH, ionic strength, etc. Continuing, CAR-positive T-cells in OpTmizer media (with supplements) or RPMI-1640 media with 10% FBS are added to the tissue culture treated plate. The cells are then grown at 37 C with 5% CO₂.

After 2 days, the cells are examined to determine any increase in the percent CAR-positive cells. This determination may be made by identifying any increase in the expression of any cell-surface and/or internal markers, including, but not limited to 4-1BB, CD69, CD25, PD-1, and Ki-67.

### EXAMPLE 5

### Generation of Humanized Sequences from Rabbit Antibodies

The Molecular Operating Environment (MOE) software developed by Chemical Computing Group (CCG) may be used to generate alignments between the rabbit antibody Clones 9-1, 9-9, 18-4, 63-1, 80-7, 137-2, and/or 154-7 and pairs of variable light and heavy chains, VL and VH, respectively from two databases:
(1) The Abysis human database: a database of about 2000 known human VL/VH sequence pairs from IMGT-LigM DB; and
(2) A human germline database: a database of germline sequences.

Humanized models show the best sequence alignments (highest identity to both the VL and VH domains) with fewest gaps. The top 100 antibody pairs from each human database may be exported and clustered using kClust (Hauser, Mayer, & Soding, (2013) BMC Bioinformatics, 248). Tables for VL and VH sequences for each of the antibodies, may be constructed, with sequences from each of the two databases clustered at 90% and 95%.

In particular, the present invention pertains to the following:
1. An isolated antigen binding molecule that specifically binds a molecule comprising a BCMA binding molecule.
2. The antigen binding molecule of item 1, wherein the antigen binding molecule is an antibody comprising a heavy chain variable region (VH) sequence that is at least about 70%, at least about 75%, at least about 80%, at least about 85%, at least about 90%, at least about 95%, at least about 96%, at least about 97%, at least about 98%, at least about 99%, or about 100% identical to a sequence selected from the group consisting of SEQ ID NOs: 11-17, and 89- 91.
3. The antigen binding molecule of item 1 or 2, wherein the antigen binding molecule comprises
   a heavy chain CDR1 selected from the group consisting of SEQ ID NOs: 38-43, 65-67,105, 106, 120, and 121;
   a heavy chain CDR2 selected from the group consisting of SEQ ID NOs: 48-53 68-73, 107-109, and 122-124; and/or
   a heavy chain CDR3 selected from the group consisting of SEQ ID NOs: 59-64,110-112, 125-127.
4. The antigen binding molecule of any one of items 1-3, wherein the antigen binding molecule is an antibody comprising a light chain variable region (VL) sequence that is at least about 70%, at least about 75%, at least about 80%, at least about 85%, at least about 90%, at least about 95%, at least about 96%, at least about 97%, at least about 98%, at least about 99%, or about 100% identical to a sequence selected from the group consisting of SEQ ID NOs: 25-31 and 95-97.
5. The antigen binding molecule of item 13, wherein the antigen binding molecule comprises
   a light chain CDR1 selected from the group consisting of SEQ ID NOs: 32-37, 98, 99, 113, and 114;
   a light chain CDR2 selected from the group consisting of SEQ ID NOs: 44-47, 100, 101, 115, and 116; and/or
   a light chain CDR3 selected from the group consisting of SEQ ID NOs: 54-58 102-103, and 117-119.
6. The antigen binding molecule of any of items 1-5, wherein the antigen binding molecule further comprises a detectable label selected from the group consisting of a fluorescent label, a photochromic compound, a proteinaceous fluorescent label, a magnetic label, a radiolabel, and a hapten; optionally
   wherein the fluorescent label is selected from the group consisting of an Atto dye, an Alexafluor dye, quantum dots, Hydroxycoumarin, Aminocouramin, Methoxycourmarin, Cascade Blue, Pacific Blue, Pacific Orange, Lucifer Yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhocamine B, Texas Red, Allophycocyanin (APC), APC-Cy7 conjugates, Indo-1, Fluo-3, Fluo-4, DCFH, DHR, SNARF, GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midorishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP,Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoeryhring (BPE), mCherry, HcRed1, Katusha, P3, Peridinin Chlorophyll (PerCP), mKate (TagFP635), TurboFP635, mPlum, and mRaspberry.
7. A polynucleotide encoding the heavy chain of an antigen binding molecule of any of items 1-5 and/or the light chain of an antigen binding molecule of any of items 1-5, optionally in a vector.
8. A cell comprising one or both of the polynucleotides of item 7, wherein the cell is selected from the group consisting of a CHO cell, a Sp2/0 cell, a rabbit cell and an *E. coli* cell.
9. A method of making an antigen binding molecule comprising incubating the cell of item 8 under suitable conditions.
10. A method of administering a dose of a medicament to a subject, the dose comprising a preselected number of cells presenting a therapeutic molecule comprising a BCMA binding molecule, the method comprising:
   (a) providing a sample of known volume comprising a population comprising a known number of cells, which cells are known or suspected to be presenting a molecule comprising a BCMA binding molecule;
   (b) providing an aliquot of the sample comprising a population of cells presenting a therapeutic molecule comprising a BCMA binding molecule;
   (c) providing an antigen binding molecule that specifically binds the a BCMA binding molecule, the antigen binding molecule further comprising a detectable label;
   (d) contacting the aliquot of (b) with the antigen binding molecule of (c) under conditions that permit the formation of a binding complex comprising a cell present in the sample and the antigen binding molecule;
   (e) determining the fraction of cells present in a binding complex of (d) in the aliquot;
   (f) determining the concentration of cells presenting a molecule comprising a BCMA binding molecule in the sample, based on the fraction of cells determined in (e);
   (g) determining the volume of the sample that comprises the selected number of cells; and
   (h) administering the volume of the sample determined in (g) to the subject.
11. A method of determining a number of cells presenting a molecule comprising a BCMA binding molecule in a sample, the method comprising:
   (a) providing a sample comprising cells known or suspected to be presenting a molecule comprising a BCMA binding molecule;
   (b) contacting the sample of (a) with an antigen binding molecule that specifically binds the molecule comprising a BCMA binding molecule, the antigen binding molecule further comprising a detectable label, under conditions that permit the formation of a binding complex comprising a cell present in the sample and the antigen binding molecule; and
   (c) determining the number of cells present in a binding complex of (b) in the sample.
12. A method of increasing the concentration of cells presenting a molecule comprising a BCMA binding molecule, the method comprising:
   (a) providing a sample comprising a cell known or suspected to present a molecule comprising a BCMA binding molecule;
   (b) providing an antigen binding molecule that specifically binds a molecule comprising a BCMA binding molecule, optionally comprising a detectable label;
   (c) contacting the sample with the antigen binding molecule under conditions that permit the formation of a binding complex comprising the molecule comprising a BCMA binding molecule and the antigen binding molecule;
   (d) removing any components not part of a binding complex; and
   (e) repeating steps (a)-(d) a desired number of times.
13. A method of depleting a population of immune cells presenting a molecule comprising a BCMA binding molecule, the method comprising:
   (a) providing a population of immune cells to be depleted, wherein the immune cells are known or suspected to be presenting a molecule comprising a BCMA binding molecule; and
   (b) contacting the immune cells with an antigen binding molecule that specifically binds to (a) the molecule comprising a BCMA binding molecule, and (b) an activating molecule expressed on the surface of the an immune cell not presenting the molecule comprising a BCMA binding molecule, under conditions that permit the formation of a ternary binding complex comprising the molecule comprising a BCMA binding molecule, the activating molecule and the antigen binding molecule.
14. The method of any one of items 10-13, wherein the cell is an immune cell selected from the group consisting of CD8+ T cells, CD4+ T cells, tumor infiltrating lymphocytes (TILs), NK cells, TCR-expressing cells, dendritic cells, and NK-T cells.
15. The method of any one of items 10-14, wherein the immune cell is a T cell, optionally, wherein the T cell is disposed *in vitro, in vivo,* or
   wherein the T cell is in blood, extracted tissue, tissue grown *ex vivo,* or cell culture media; or
   wherein the T cell is an autologous T cell or an allogenic T cell.
16. A method of isolating a molecule comprising a BCMA binding molecule, comprising:
   (a) providing a sample known or suspected to comprise a molecule comprising a BCMA binding molecule;
   (b) providing an antigen binding molecule that specifically binds a molecule comprising a BCMA binding molecule, optionally comprising a detectable label;
   (c) contacting the sample with the antigen binding molecule, under conditions that permit the formation of a binding complex comprising the molecule comprising a BCMA binding molecule and the antigen binding molecule;
   (d) separating any molecules not part of a binding complex from formed binding complexes; and
   (e) separating a formed binding complex into: (a) a molecule comprising Sa BCMA binding molecule, and (b) an antigen binding molecule.
17. A method of determining the presence or absence of a molecule comprising a BCMA binding molecule in a sample, the method comprising:
   (a) providing a sample known or suspected to comprise a molecule comprising a BCMA binding molecule;
   (b) providing an antigen binding molecule comprising a detectable label that specifically binds a molecule comprising a BCMA binding molecule;
   (c) contacting the sample with the antigen binding molecule under conditions that permit the formation of a binding complex;
   (d) separating any molecules not part of a binding complex from formed binding complexes; and
   (e) detecting the presence or absence of a binding complex.
18. The method of item 16 or 17, wherein the antigen binding molecule is disposed on a surface selected from the group consisting of an agarose bead, a magnetic bead, a plastic welled plate, a glass welled plate, a ceramic welled plate and a cell culture bag.
19. The method of any one of items 10-18, wherein the molecule comprising a BCMA binding molecule is a CAR; optionally, wherein the CAR further comprises a molecule, or a fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGBl, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof.
20. The method of any one of items 10-19, wherein the antigen binding molecule comprises an antigen binding molecule of items 1-6, and humanized forms thereof.

## Claims

1. An isolated antigen binding molecule that specifically binds a molecule comprising a BCMA binding molecule, said antigen binding molecule comprising a heavy chain variable region (VH) and a light chain variable region (VL) of an antibody, wherein
(i) the VH comprises the amino acid sequence of VH CDR1, VH CDR2 and VH CDR3 depicted in SEQ ID NOS: 67, 72 and 63, respectively and the VL comprises the amino acid sequence of VL CDR1, VL CDR2 and CDR3 depicted in SEQ ID NOS: 36, 46 and 57, respectively (clone 137-2);
(ii) the VH comprises the amino acid sequence of VH CDR1, VH CDR2 and VH CDR3 depicted in SEQ ID NOS: 38, 68 and 59, respectively and the VL comprises the amino acid sequence of VL CDR1, VL CDR2 and CDR3 depicted in SEQ ID NOS: 32, 44 and 54, respectively (clone 9-1);
(iii) the VH comprises the amino acid sequence of VH CDR1, VH CDR2 and VH CDR3 depicted in SEQ ID NOS: 121, 124 and 127, respectively and the VL comprises the amino acid sequence of VL CDR1, VL CDR2 and CDR3 depicted in SEQ ID NOS: 114, 116 and 119, respectively (clone 89-10);
(iv) the VH comprises the amino acid sequence of VH CDR1, VH CDR2 and VH CDR3 depicted in SEQ ID NOS: 65, 70 and 61, respectively and the VL comprises the amino acid sequence of VL CDR1, VL CDR2 and CDR3 depicted in SEQ ID NOS: 34, 45 and 56, respectively (clone 63-1); or
(v) the VH comprises the amino acid sequence of VH CDR1, VH CDR2 and VH CDR3 depicted in SEQ ID NOS: 85, 73 and 64, respectively and the VL comprises the amino acid sequence of VL CDR1, VL CDR2 and CDR3 depicted in SEQ ID NOS: 37, 47 and 58, respectively (clone 154-7).

2. The antigen binding molecule of claim 1, wherein the antigen binding molecule is an antibody comprising (i) a heavy chain variable region (VH) and (ii) a light chain variable region, wherein:
(i) the heavy chain variable region (VH) amino acid sequence is at least about 70%, at least about 75%, at least about 80%, at least about 85%, at least about 90%, at least about 95%, at least about 96%, at least about 97%, at least about 98%, at least about 99%, or about 100% identical to the amino acid sequence depicted in SEQ ID NO: 16, 11, 91, 14 or 17 and
(ii) the light chain variable region (VL) amino acid sequence is at least about 70%, at least about 75%, at least about 80%, at least about 85%, at least about 90%, at least about 95%, at least about 96%, at least about 97%, at least about 98%, at least about 99%, or about 100% identical to the amino acid sequence depicted in SEQ ID NO: 30, 25, 97, 27 or 31, respectively.

3. The antigen binding molecule of claim 1 or 2, wherein the antigen binding molecule further comprises a detectable label selected from the group consisting of a fluorescent label, a photochromic compound, a proteinaceous fluorescent label, a magnetic label, a radiolabel, and a hapten;
optionally wherein the fluorescent label is selected from the group consisting of an Atto dye, an Alexafluor dye, quantum dots, Hydroxycoumarin, Aminocouramin, Methoxycourmarin, Cascade Blue, Pacific Blue, Pacific Orange, Lucifer Yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhocamine B, Texas Red, Allophycocyanin (APC), APC-Cy7 conjugates, Indo-1, Fluo-3, Fluo-4, DCFH, DHR, SNARF, GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midorishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP,Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoeryhring (BPE), mCherry, HcRed1, Katusha, P3, Peridinin Chlorophyll (PerCP), mKate (TagFP635), TurboFP635, mPlum, and mRaspberry.

4. A polynucleotide encoding the heavy chain variable region of an antigen binding molecule of claim 1 or 2 and/or the light chain variable region of an antigen binding molecule of claim 1 or 2, optionally in a vector.

5. A cell comprising one or both of the polynucleotides of claim 4, wherein the cell is selected from the group consisting of a CHO cell, a Sp2/0 cell, a rabbit cell and an *E*. *coli* cell.

6. A method of making an antigen binding molecule comprising incubating the cell of claim 5 under suitable conditions.

7. A dose of a medicament comprising a preselected number of cells presenting a therapeutic molecule comprising a BCMA binding molecule for use in a method of treating an abnormal physiological condition in a subject, the method comprising:
(a) providing a sample of known volume comprising a population comprising a known number of cells, which cells are known or suspected to be presenting a molecule comprising a BCMA binding molecule;
(b) providing an aliquot of the sample comprising a population of cells presenting a therapeutic molecule comprising a BCMA binding molecule;
(c) providing the antigen binding molecule that specifically binds the a BCMA binding molecule of claim 1 or 2, the antigen binding molecule further comprising a detectable label;
(d) contacting the aliquot of (b) with the antigen binding molecule of (c) under conditions that permit the formation of a binding complex comprising a cell present in the sample and the antigen binding molecule;
(e) determining the fraction of cells present in a binding complex of (d) in the aliquot;
(f) determining the concentration of cells presenting a molecule comprising a BCMA binding molecule in the sample, based on the fraction of cells determined in (e);
(g) determining the volume of the sample that comprises the selected number of cells; and
(h) administering the volume of the sample determined in (g) to the subject.

8. A method of determining a number of cells presenting a molecule comprising a BCMA binding molecule in a sample, the method comprising:
(a) providing a sample comprising cells known or suspected to be presenting a molecule comprising a BCMA binding molecule;
(b) contacting the sample of (a) with the antigen binding molecule that specifically binds the molecule comprising a BCMA binding molecule of claim 1 or 2, the antigen binding molecule further comprising a detectable label, under conditions that permit the formation of a binding complex comprising a cell present in the sample and the antigen binding molecule; and
(c) determining the number of cells present in a binding complex of (b) in the sample.

9. A method of increasing the concentration of cells presenting a molecule comprising a BCMA binding molecule, the method comprising:
(a) providing a sample comprising a cell known or suspected to present a molecule comprising a BCMA binding molecule;
(b) providing the antigen binding molecule that specifically binds a molecule comprising a BCMA binding molecule of claim 1 or 2, optionally comprising a detectable label;
(c) contacting the sample with the antigen binding molecule under conditions that permit the formation of a binding complex comprising the molecule comprising a BCMA binding molecule and the antigen binding molecule;
(d) removing any components not part of a binding complex; and
(e) repeating steps (a)-(d) a desired number of times.

10. A method of depleting a population of immune cells presenting a molecule comprising a BCMA binding molecule, the method comprising:
(a) providing a population of immune cells to be depleted, wherein the immune cells are known or suspected to be presenting a molecule comprising a BCMA binding molecule; and
(b) contacting the immune cells with (a) the antigen binding molecule of claim 1 or 2 that specifically binds to the molecule comprising a BCMA binding molecule, and (b) an activating molecule expressed on the surface of the an immune cell not presenting the molecule comprising a BCMA binding molecule, under conditions that permit the formation of a ternary binding complex comprising the molecule comprising a BCMA binding molecule, the activating molecule and the antigen binding molecule.

11. The dose of the medicament for use of claim 7, or the method of any one of claims 8 to 10, wherein the cell presenting the molecule comprising a BCMA binding molecule is an immune cell selected from the group consisting of CD8+ T cells, CD4+ T cells, tumor infiltrating lymphocytes (TILs), NK cells, TCR-expressing cells, dendritic cells, and NK-T cells.

12. The dose of the medicament for use of claim 11, or the method of claim 11, wherein the immune cell is a T cell, optionally, wherein the T cell is disposed *in vitro, in vivo,* or wherein the T cell is in blood, extracted tissue, tissue grown *ex vivo,* or cell culture media; or wherein the T cell is an autologous T cell or an allogenic T cell.

13. A method of isolating a molecule comprising a BCMA binding molecule, comprising:
(a) providing a sample known or suspected to comprise a molecule comprising a BCMA binding molecule;
(b) providing the antigen binding molecule that specifically binds a molecule comprising a BCMA binding molecule of claim 1 or 2, optionally comprising a detectable label;
(c) contacting the sample with the antigen binding molecule, under conditions that permit the formation of a binding complex comprising the molecule comprising a BCMA binding molecule and the antigen binding molecule;
(d) separating any molecules not part of a binding complex from formed binding complexes; and
(e) separating a formed binding complex into: (a) a molecule comprising Sa BCMA binding molecule, and (b) an antigen binding molecule;
optionally wherein the antigen binding molecule is disposed on a surface selected from the group consisting of an agarose bead, a magnetic bead, a plastic welled plate, a glass welled plate, a ceramic welled plate and a cell culture bag.

14. A method of determining the presence or absence of a molecule comprising a BCMA binding molecule in a sample, the method comprising:
(a) providing a sample known or suspected to comprise a molecule comprising a BCMA binding molecule;
(b) providing the antigen binding molecule of claim 1 or 2 that specifically binds a molecule comprising a BCMA binding molecule comprising a detectable label;
(c) contacting the sample with the antigen binding molecule under conditions that permit the formation of a binding complex;
(d) separating any molecules not part of a binding complex from formed binding complexes; and
(e) detecting the presence or absence of a binding complex;
optionally wherein the antigen binding molecule is disposed on a surface selected from the group consisting of an agarose bead, a magnetic bead, a plastic welled plate, a glass welled plate, a ceramic welled plate and a cell culture bag.

15. The dose of the medicament for use of any one of claims 7, 11 or 12, or the method of any one of claims 8 to 14, wherein the molecule comprising a BCMA binding molecule is a CAR; optionally, wherein the CAR further comprises a molecule, or a fragment thereof, selected from the group consisting of CD28, OX-40, 4-1BB/CD137, CD2, CD7, CD27, CD30, CD40, Programmed Death-1 (PD-1), inducible T cell co-stimulator (ICOS), lymphocyte function-associated antigen-1 (LFA-1, CDl-la/CD18), CD3 gamma, CD3 delta, CD3 epsilon, CD3 zeta, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CDl ld, ITGAE, CD103, ITGAL, CDl la, LFA-1, ITGAM, CDl lb, ITGAX, CDl lc, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, and combinations thereof; and/or wherein the antigen binding molecule comprises an antigen binding molecule of claim 1 or 2, and humanized forms thereof.
